# EUROPEAN PATENT APPLICATION

(11) **EP 4 410 793 A1**
(43) Date of publication of application: **07.08.2024**
(21) Application number: 22874875.2
(22) Date of filing: 27.09.2022
(51) Int. Cl.: C07D 471/08, C07D 491/056, A61K 31/4741, A61P 3/00, A61P 3/10

(54) **SOLID COMPOSITION OF GLP-1 RECEPTOR AGONIST**

(30) Priority: 28.09.2021 CN 202111140592; 28.09.2021 US 202163249110 P
(71) Applicant: Hangzhou Zhongmeihuadong Pharmaceutical Co., Ltd., GongShu District HangZhou, Zhejiang 310011 (CN); vTv Therapeutics LLC, High Point, NC 27265 (US)
(72) Inventor: CHEN, Meimei, Hangzhou, Zhejiang 310011 (CN); FANG, Jie, Hangzhou, Zhejiang 310011 (CN); WU, Hui, Hangzhou, Zhejiang 310011 (CN); TAN, Fang, Hangzhou, Zhejiang 310011 (CN); LI, Haiyan, Hangzhou, Zhejiang 310011 (CN); XIAO, Li, Hangzhou, Zhejiang 310011 (CN); BIAN, Cong, Hangzhou, Zhejiang 310011 (CN); CHEN, Lina, Hangzhou, Zhejiang 310011 (CN); ALMARIEGO, Danilo, High Point, North Carolina 27265 (US); ANANTHA, Andy, High Point, North Carolina 27265 (US)
(74) Representative: Mewburn Ellis LLP
(86) International application number: PCT/CN2022/121554
(87) International publication number: WO 2023/051490

(57) **Abstract**

Provided in the present invention is a pharmaceutical composition of a small molecular GLP-1R receptor agonist suitable for oral administration. More specifically, the present invention relates to a pharmaceutical composition containing (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinoline-8-ylformylamino)-3-(4-(2,3-dimethylpyridin-4-yl)phenyl)propanoic acid ("OAD2") and a pharmaceutically acceptable salt thereof, and a preparation method therefor. The composition may contain a low level of one or more oxidative degradation substances or may contain a low level of reactive oxygen species. The pharmaceutical composition provided in the present invention has low content of oxidative degradation impurity B and a low total impurity content, so that the composition can be stably stored for 12 months or more under normal temperature and normal humidity. The present invention further relates to a method for treating type II diabetes and indications related to improper blood glucose control using such pharmaceutical compositions.

## Description

### Technical Field

The present invention relates to pharmaceutical compositions of a small molecule GLP-1R receptor agonist suitable for oral administration, and more particularly to pharmaceutical compositions comprising (S)-2-(3S,8S)-3-(4-(3,4-dichlorobenzyloxy)phenyl-7-((S)-1-phenylpropyl)- 2,3,6,7,8,9-hexahydro-[1,4]-dioxino[2,3-g]isoquinolin-8-formylamino)-3-(4-(2,3-dimethylpyridin-4-yl)phenyl)propanoic acid ("OAD2") or a pharmaceutically acceptable salt thereof and preparation methods thereof. The present invention is also directed to a method of using such pharmaceutical compositions to treat type II diabetes and indications associated with poor glycemic control.

### Background technology

Type II diabetes can be characterized by one or more of the following metabolic disorders and their disease progression: peripheral tissue insulin resistance, hyperglycemia, islet b-cell compensation, hyperinsulinemia, dyslipidemia, increased hepatic gluconeogenesis, and eventual losses of b-cell population and function. Pathophysiological consequences of abnormal glucose and lipid metabolism are toxicities to organs such as the kidney, eye, peripheral neurons, vasculature and heart. Therefore, there is a need for agents that can delay the progression of diseases associated with type II diabetes, thereby achieving improved glycemic control and improved b-cell populations and function.

Glucagon-like peptide-1 (GLP-1) is a kind of brain-gut peptide secreted by ileal endocrine cells and is mainly used as a target of actions of type II diabetes drugs at present. An important function of GLP-1 is to activate its receptor on islet b-cells, and the positive metabolic benefits of which may include, but are not limited to, inhibition of excessive glucagon production, delayed gastric emptying time, and improvement of b-cell population and function. The positive effect of GLP-1 on b-cell population and function can be provided: GLP-1-like therapies can delay early disease progression. In addition, GLP-1 agonists may also be used in combination therapy, such as in combination with insulin in patients with type II diabetes.

(S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-formylamino)-3-(4-(2,3-dimethylpyridin-4-yl)phenyl)propanoic acid dihydrochloride (hereinafter referred to as "OAD2 dihydrochloride") has a formula of C₅₀H₄₉Cl₄N₃O₆, a molecular weight of 929.76, having the following chemical formula:

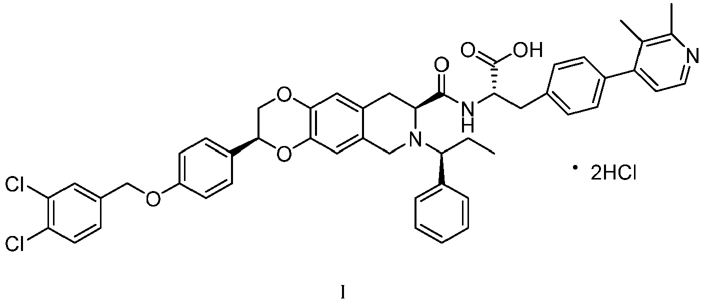

OAD2 dihydrochloride is a non-peptide, oral glucagon-like peptide-1 receptor (GLP-1r) agonist.

WO 2010/114824 discloses the structure of the free base of OAD2, but does not systematically study the composition of OAD2 or a pharmaceutically acceptable salt thereof.

As with any small molecule drug, there is a need to identify formulations that reduce or eliminate impurity growth under various storage conditions so that the formulations can be stored for longer time or cost less.

### Summary of the present invention

(S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propionic acid ("OAD2") is a small molecule, non-peptide glucagon-like peptide 1 (GLP-1) receptor agonist that is under development to treat diabetes and other related indications. OAD2 has a MW of 856 and is disclosed in International publication WO 2010/114824. The OAD2 dihydrochloride has the following chemical structure:

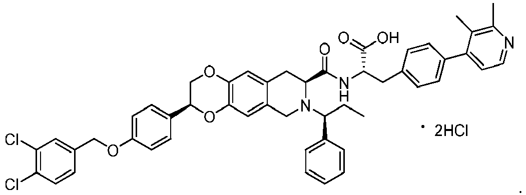

The present invention provides pharmaceutical compositions comprising OAD2 or a pharmaceutically acceptable salt thereof, methods for their preparation, and use thereof in treating conditions where modulation of the human GLP-1 receptor is beneficial, such as diabetes.

The present invention also provides pharmaceutical compositions with low levels of impurity B through research on compatibility of raw auxiliary materials and screening of auxiliary materials.

The present invention provides pharmaceutical compositions with low levels of reactive oxygen species (ROS) through reducing oxides, peroxides, superoxides and other oxides or active oxygen structural components in raw auxiliary materials.

The present invention also provides pharmaceutical compositions with low levels of total impurities.

### Detailed description

Research shows that related substances, especially oxidative degradation impurity B can rapidly grow in solid form and/or solid preparation of OAD2 dihydrochloride under normal temperature condition. Because the oxidative degradation impurity B may increase the risk of druggability of the formulation, and at the same time, the storage conditions of the pharmaceutical composition or the formulation become harsh due to too high growth rate of the oxidative degradation impurities, it is necessary to control the limits of the oxidative degradation impurity B and the related substances in total by optimizing the auxiliary materials, so as to reduce the risk of drug administration after the pharmaceutical composition is formed into a formulation.

During the course of research on the cause of impurity growth, researchers have found that OAD2 dihydrochloride reacts readily with some solubilizing agents under acidic conditions, resulting in the growth of related substances. In this regard, researchers have screened compatibility of the raw auxiliary materials based on the original formulation, reevaluated the auxiliary materials, particularly solubilizing agents and disintegrating agents, and controlled the limits of related substances by selecting solubilizing agents and disintegrating agents that do not readily react with OAD2 dihydrochloride.

The present invention provides pharmaceutical compositions of OAD2 dihydrochloride with good dissolution, good formulation and process reproducibility.

The present invention provides pharmaceutical compositions of OAD2 dihydrochloride that are low in related substances, including oxidative degradation impurity B.

The pharmaceutical compositions provided by the present invention have the advantages of stable production process and strong reproducibility, and the prepared OAD2 dihydrochloride pharmaceutical compositions have good dissolution, low contents of oxidative degradation impurity B and low contents of related substances.

The pharmaceutical compositions provided by the present invention allow effective control of the growth of related substances, so that the compositions can be stored at normal temperature, and meanwhile, the probability of adverse reaction of the formulation composition is reduced, and the safety of the drug is ensured.

### Pharmaceutical compositions of OAD2 and a pharmaceutically acceptable salt thereof

The present invention provides a pharmaceutical composition comprising OAD2 or a pharmaceutically acceptable salt thereof and one or more pharmaceutically acceptable auxiliary materials.

Compositions of the present invention comprise OAD2, or a pharmaceutically acceptable salt thereof, in combination with one or more pharmaceutically acceptable excipients or carrier materials suitable for oral administration. The compositions of the present invention may comprise a required amount of OAD2 or a pharmaceutically acceptable salt thereof mixed with one or more of a disintegrant, a binder, a filler, and a surfactant. The compositions may also comprise one or more of a lubricant, a glidant, an acidifer, and an absorption enhancer. The compositions may also optionally comprise one or more antioxidants.

In an embodiment, such compositions are tableted or encapsulated to facilitate administration in the form of immediate release capsules or tablets.

The present invention provides a pharmaceutical composition comprising OAD2, or a pharmaceutically acceptable salt thereof, and at least one solubilizer.

In some embodiments, the mass percent content of the active ingredient is 1-60%.

In some embodiments, the mass percent content of the active ingredient is 5%-40%.

In some embodiments, the mass percent content of the active ingredient is 10%-20%.

As a specific embodiment, the mass percentage content of the active ingredient is 1 to 5%, 2 to 6%, 3 to 7%, 4 to 8%, 5 to 9%, 6 to 10%, 11 to 15%, 12 to 16%, 13 to 17%, 14 to 18%, 15 to 19%, 16 to 20%, 21 to 25%, 22 to 26%, 23 to 27%, 24 to 28%, 25 to 29 %, 26 to 30%, 31 to 35%, 32 to 36%, 33 to 37%, 34 to 38%, 35 to 39%, 36 to 40%, 41 to 45%, 42 to 46%, 43 to 47 %, 44 to 48%, 45 to 49%, 46 to 50%, 51 to 55%, 52 to 56%, 53 to 57%, 54 to 58%, 55 to 59%, 56 to 60%.

As a specific embodiment, the mass percentage content of the active ingredient is 4±2%, 6±2%, 8±2%, 10±2%, 12±2%, 14±2%, 16±2%, 18±2%, 20±2%, 22±2%, 24±2%, 26±2%, 28±2%, 30±2%, 32±2%, 34±2%, 36±2%, 40±2%, 42±2%, 44±2%, 46±2%, 48±2%, 50±2%.

As a specific embodiment, the mass of the active ingredient in the pharmaceutical composition is 1 to 600 mg, 5 to 300 mg, 10 to 150 mg, 20 to 75 mg, or 15 to 25 mg, 30 to 40 mg, 45 to 55 mg, 60 to 70mg, 75 to 85mg, 90 to 100mg, 105 to 115mg, 120 to 130mg, 135 to 145mg, 150 to 160mg, 165 to 175mg, 180 to 190mg, 200 to 210mg, 220 to 230mg, 240 to 250mg, 260 to 270mg, 280 to 290mg, 300 to 310mg, 320 to 330mg, 340 to 350mg, 360 to 370mg, 380 to 390mg, 400 to 410mg, 420 to 430mg, 440 to 450mg.

As a specific embodiment, the mass of the active ingredient in the pharmaceutical composition is 10±2.5mg, 15±2.5mg, 20±2.5mg, 25±2.5mg, 30±2.5mg, 35±2.5mg, 40±2.5mg, 45±2.5mg, 50±2.5mg, 55±2.5mg, 60±2.5mg, 65±2.5mg, 70±2.5mg, 75±2.5mg, 80±2.5mg, 85±2.5mg, 90±2.5mg, 95±2.5mg, 100±2.5mg, 105±2.5mg, 110±2.5mg, 115±2.5mg, 120±2.5mg, 125±2.5mg, 130±2.5mg, 135±2.5mg, 140±2.5mg, 145±2.5mg, 150±2.5mg, 155±2.5mg, 160±2.5mg, 165±2.5mg, 170±2.5mg, 175±2.5mg, 180±2.5mg, 185±2.5mg, 190±2.5mg, 195±2.5mg, 200±2.5mg, 205±2.5mg, or 210±5mg, 220±5mg, 230±5mg, 240±5mg, 250±5mg, 260±5mg, 270±5mg, 280±5mg, 290±5mg, 300±5mg, 310±5mg, 320±5mg, 330±5mg, 340±5mg, 350±5mg, 360±5mg, 370±5mg, 380±5mg, 390±5mg, 400± 5mg, 410±5mg, 420±5mg, 430±5mg, 440±5mg, 450±5mg.

### 1. Solubilizer

In the pharmaceutical compositions provided by the present invention, the mass percentage content of the solubilizer is selected from 0.1% to 10%.

In some embodiments, the mass percentage content of the solubilizer is 0.2% to 5%.

As a specific embodiment, the mass percentage content of the solubilizer is 0.1 to 0.6%, 0.2 to 0.7%, 0.3 to 0.8%, 0.4 to 0.9%, 0.5 to 1.0%, 1.1 to 1.6%, 1.2 to 1.7 %, 1.3 to 1.8%, 1.4 to 1.9%, 1.5 to 2.0%, 2.1 to 2.6%, 2.2 to 2.7%, 2.3 to 2.8%, 2.4 to 2.9%, 2.5 to 3.0%, 3.1 to 3.6%, 3.2 to 3.7%, 3.3 to 3.8%, 3.4 to 3.9%, 4.5 to 5.0%, 5.1 to 5.6%, 5.2 to 5.7%, 5.3 to 5.8%, 5.4 to 5.9%, 5.5 to 6.0%, 6.1 to 6.6%, 6.2 to 6.7%, 6.3 to 6.8%, 6.4 to 6.9%, 6.5 to 7.0%, 7.1 to 7.6%, 7.2 to 7.7%, 7.3 to 7.8%, 7.4 to 7.9%, 7.5 to 8.0%, 8.1 to 8.6%, 8.2 to 8.7%, 8.3 to 8.8%, 8.4 to 8.9%, 8.5 to 9.0%, 9.1 to 9.6%, 9.2 to 9.7%, 9.3 to 9.8%, 9.4 to 9.9%, 9.5 to 10.0%.

As a specific embodiment, the mass of the solubilizing agent in the pharmaceutical composition is 0.5 to 50 mg, 1 to 30 mg, 2 to 20 mg, or 2 to 5 mg, 6 to 9 mg, 10 to 14 mg, 15 to 19 mg, 20 to 24mg, 25 to 29mg, 30 to 34mg, 35 to 39mg, 40 to 44mg, 45 to 49mg.

As a specific embodiment, the mass of the solubilizer in the pharmaceutical composition is 3±1 mg, 6±1 mg, 9±1 mg, 12±1 mg, 15±1 mg, 18±1 mg, 21±1 mg, 24±1mg, 27±1mg, 30±1mg, 33±1mg, 36±1mg, 39±1mg, 42±1mg, 45±1mg, 48±1mg.

In some embodiments, the solubilizer is selected from one or two or more of HS15, RH40, hydroxypropyl βcyclodextrin, SoluPlus, castor oil polyoxyl 35, polyethylene glycol cetostearyl ether 12.

As a specific embodiment, the solubilizer is HS15.

As a specific embodiment, the solubilizer is RH40.

As a specific embodiment, the solubilizer is hydroxypropyl βcyclodextrin.

As a specific embodiment, the solubilizer is SoluPlus.

### 2. OAD2 and a pharmaceutically acceptable salt Thereof

In some embodiments, in the pharmaceutical compositions provided by the present invention, OAD2 or a pharmaceutically acceptable salt thereof is the only active ingredient.

In some embodiments, the pharmaceutically acceptable salt is selected from one of hydrochloride, dihydrochloride, p-toluenesulfonate, sulfate, hydrobromide, tartrate, citrate, glycolate, methanesulfonate.

In a specific embodiment, the pharmaceutically acceptable salt is dihydrochloride salt.

In some embodiments, the active ingredient is one of OAD2 hydrochloride, OAD2 dihydrochloride, OAD2 p-toluenesulfonate, OAD2 sulfate, OAD2 hydrobromide, OAD2 tartrate, OAD2 citrate, OAD2 glycolate, and OAD2 methanesulfonate.

As a specific embodiment, the active ingredient is OAD2 dihydrochloride.

As a specific embodiment, the active ingredient is present in the pharmaceutical composition in the form of free OAD2.

As a specific embodiment, the active ingredient is present in the pharmaceutical composition in the form of OAD2 dihydrochloride.

The pharmaceutically acceptable salt of OAD2 as described in the present invention is formed from OAD2 and a pharmaceutically acceptable acid.

In some embodiments, the pharmaceutically acceptable acid is selected from the group consisting of 1-hydroxy-2-naphthoic acid, 4-aminosalicylic acid, adipic acid, L-aspartic acid, benzenesulfonic acid, benzoic acid, trans-cinnamic acid, citric acid, fumaric acid, galactonic acid, gentisic acid, gluconic acid, glutamic acid, glutaric acid, glycolic acid, caproic acid, hippuric acid, hydrobromic acid, hydrochloric acid, L-lactic acid, maleic acid, L-malic acid, malonic acid, R-mandelic acid, methanesulfonic acid, naphthalenesulfonic acid, nicotinic acid, oxalic acid, palmitic acid, phosphoric acid, propionic acid, saccharin, salicylic acid, stearic acid, succinic acid, sulfuric acid, L-tartaric acid, p-toluenesulfonic acid, vanillic acid, and vanillin.

In some embodiments, the pharmaceutically acceptable acid is selected from the group consisting of hydrobromic acid, hydrochloric acid, p-toluenesulfonic acid, tartaric acid, citric acid, glycolic acid, methanesulfonic acid, and sulfonic acid.

The total amount of the active ingredient (OAD2 or a pharmaceutically acceptable salt thereof) in the pharmaceutical compositions or dosage forms of the present invention is not limited.

In an embodiment, the content of the active ingredient is within the range of from 0.01 to 80 wt%, or 0.1 to 50 wt%, or 10 to 40 wt%, based on the total weight of the dosage form. In an embodiment, the content of the active ingredient is within the range of from 1 to 4 wt%, or 2 to 5 wt%, or 3 to 6 wt%, or 4 to 7 wt%, or 5 to 8 wt%, or 6 to 9 wt%, or 7 to 10 wt%, or 8 to 11 wt%, or 9 to 12 wt%, or 10 to 13 wt%, or 11 to 14 wt%, or 12 to 15 wt%, or 13 to 16 wt%, or 14 to 17 wt%, or 15 to 18 wt%, or 16 to 19 wt%, or 17 to 20 wt%, or 18 to 21 wt%, or 19 to 22 wt%, or 20 to 23 wt%, or 21 to 24 wt%, or 22 to 25 wt%, or 23 to 26 wt%, or 24 to 27 wt%, or 25 to 28 wt%, or 26 to 29 wt%, or 27 to 30 wt%, or 28 to 31 wt%, or 29 to 32 wt%, or 30 to 33 wt%, or 31 to 24 wt%, or 32 to 35 wt%, or 33 to 36 wt%, or 34 to 37 wt%, or 35 to 38 wt%, or 36 to 39 wt%, or 37 to 40 wt%. In an embodiment, the content of active ingredient is within the range of from 28 to 32 wt% or from 10 to 14 wt%.

In another embodiment, the content of the active ingredient is 4 ± 2.5 wt%, 6 ± 2.5 wt%, 8 ± 2.5 wt%, 10 ± 2.5 wt%, or 12 ± 2.5 wt%, or 14 ± 2.5 wt%, or 16 ± 2.5 wt%, or 18 ± 2.5 wt%, or 20 ± 2.5 wt%, or 22 ± 2.5 wt%, or 24 ± 2.5 wt%, or 26 ± 2.5 wt%, or 28 ± 2.5 wt%, or 30 ± 2.5 wt%, or 32 ± 2.5 wt%, or 34 ± 2.5 wt%, or 36 ± 2.5 wt%, or 38 ± 2.5 wt%, or 40 ± 2.5 wt%, or 42 ± 2.5 wt%, or 44 ± 2.5 wt%, or 46 ± 2.5 wt%, or 48 ± 2.5 wt%, or 50 ± 2.5 wt%, based on the total weight of the dosage form. In an embodiment, the content of the active ingredient is 30 ± 2.5 wt% or 12 ± 2.5 wt%.

In another embodiment, the amount of the active ingredient that is contained in the dosage form is within the range of from 1 to 500 mg, or from 10 to 250 mg, or from 25 to 200 mg, or from 20 to 60 mg, or from 40 to 80 mg, or from 60 to 100 mg, or from 80 to 140 mg, or from 100 to 160 mg, or from 120 to 180 mg, or from 140 to 200 mg, or from 160 to 220 mg, or from 180 to 240 mg, or from 200 to 260 mg.

In another embodiment, the amount of the active ingredient that is contained in the dosage form is 25 mg ± 5 mg, or 30 mg ± 5 mg, or 40 mg ± 5 mg, or 50 mg ± 5 mg, or 60 mg ± 5 mg, or 75 mg ± 5 mg, or 80 mg ± 5 mg, or 90 mg ± 5 mg, or 100 mg ± 5 mg, or 110 mg ± 5 mg, or 120 mg ± 5 mg, or 125 mg ± 5 mg, or 130 mg ± 5 mg, or 140 mg ± 5 mg, or 150 mg ± 5 mg, or 160 mg ± 5 mg, or 170 mg ± 5 mg, or 175 mg ± 5 mg, or 180 mg ± 5 mg, or 190 mg ± 5 mg, or 200 mg ± 5 mg, or 225 mg ± 5 mg, or 250 mg ± 5 mg. In another embodiment, the amount of active ingredient that is contained in the dosage form is 25 mg ± 5 mg, or 50 mg ± 5 mg, or 75 mg ± 5 mg, or 100 mg ± 5 mg, or 125 mg ± 5 mg. In another embodiment, the amount of active ingredient that is contained in the dosage form is 25 mg ± 2.5 mg, or 50 mg ± 2.5 mg, or 75 mg ± 2.5 mg, or 100 mg ± 2.5 mg, or 125 mg ± 2.5 mg.

In another embodiment, the active ingredient in the composition or dosage form is OAD2 dihyrdrochloride salt, and the dosage form is adapted for oral administration once daily or twice daily and the amount of OAD2 dihyrdrochloride contained in the dosage form is 25 mg ± 2.5 mg, 50 mg ± 2.5 mg, 75 mg ± 2.5 mg, or 100 mg ± 2.5 mg.

In an embodiment, after storage for 4 weeks at 40 °C and 75% rel. humidity, the content of the active ingredient (preferably OAD2 dihyrdrochloride) in the composition or dosage form amounts to at least 90%, or at least 98.0%, or at least 98.5%, or at least 99.0%, or at least 99.2%, or at least 99.4%, or at least 99.6%, or at least 99.8% of its original content before storage.

In another embodiment, after storage for 6 months at 40°C ± 2°C and 75% ± 5% relative humidity, the content of the active ingredient (preferably OAD2 dihyrdrochloride) in the composition or dosage form amounts to at least 90%, or at least 98.0%, or at least 98.5%, or at least 99.0%, or at least 99.2%, or at least 99.4%, or at least 99.6%, or at least 99.8% of its original content before storage.

In another embodiment, after storage for 6 months at 30 °C ± 2 °C and 65% ± 5% relative humidity, the content of the active ingredient (preferably OAD2 dihyrdrochloride) in the composition or dosage form amounts to at least 90%, or at least 98.0%, or at least 98.5%, or at least 99.0%, or at least 99.2%, or at least 99.4%, or at least 99.6%, or at least 99.8% of its original content before storage.

In another embodiment, after storage for 12 months at 30°C ± 2°C and 65% ± 5% relative humidity or at 25°C ± 2°C and 60% ± 5% relative humidity, the content of the active ingredient (preferably OAD2 dihyrdrochloride) in the composition or dosage form amounts to at least 90%, or at least 98.0%, or at least 98.5%, or at least 99.0%, or at least 99.2%, or at least 99.4%, or at least 99.6%, or at least 99.8% of its original content before storage.

Suitable methods for measuring the content of the active ingredient in the dosage form are well known to the skilled artisan, some of which are described in the Examples section below.

### 3. Filler (also called "diluent")

The pharmaceutical compositions provided by the present invention may also comprise one or more pharmaceutically acceptable auxiliary materials in addition to OAD2 or a pharmaceutically acceptable salt thereof; the auxiliary materials may be disintegrants, fillers, binders, lubricants, wetting agents, glidants, acidifiers, surfactants, absorption enhancers.

The pharmaceutical compositions provided by the present invention may also comprise disintegrants, fillers, binders, lubricants, wetting agents, glidants, acidifiers, surfactants, absorption enhancers, in addition to OAD2 or a pharmaceutically acceptable salt thereof.

In some embodiments, the compositions of the present invention optionally, but preferably, comprise one or more pharmaceutically acceptable fillers as carrier materials. In some embodiments, the pharmaceutical compositions provided by the present invention comprises fillers in addition to OAD2 or a pharmaceutically acceptable salt thereof.

In some embodiments, the filler is selected from at least one of microcrystalline cellulose, lactose, sucrose, mannitol, corn starch, pregelatinized starch, dextrin, sorbitol, inorganic calcium salt, cellulose acetate, glucose, ethyl cellulose and glyceryl palmitostearate.

The mass percentage content of the filler in the pharmaceutical compositions provided by the present invention is 10 to 75%.

In some embodiments, the filler is preferably selected from one or two or more of microcrystalline cellulose, lactose, mannitol, and pregelatinized starch.

In some embodiments, the filler is selected from the combination of microcrystalline cellulose and lactose.

In some embodiments, the mass percentage content of the filler is 20 to 60%.

As a specific embodiment, the mass percentage content of the filler in the pharmaceutical compositions of the present invention is 15 to 19%, 16 to 20%, 21 to 25%, 22 to 26%, 23 to 27%, 24 to 28%, 25 to 29%, 26 to 30%, 31 to 35%, 32 to 36%, 33 to 37%, 34 to 38%, 35 to 39%, 36 to 40%, 41 to 45%, 42 to 46%, 43 to 47%, 44 to 48%, 45 to 49%, 46 to 50%, 51 to 55%, 52 to 56%, 53 to 57%, 54 to 58%, 55 to 59%, 56 to 60%.

As a specific embodiment, the mass of the filler in the pharmaceutical compositions of the present invention is 50±2.5 mg, 55±2.5 mg, 60±2.5 mg, 65±2.5 mg, 70±2.5 mg, 75±2.5 mg, 80±2.5mg, 85±2.5mg, 90±2.5mg, 95±2.5mg, 100±2.5mg, 105±2.5mg, 110±2.5mg, 115±2.5mg, 120±2.5mg, 125±2.5 mg, 130±2.5mg, 135±2.5mg, 140±2.5mg, 145±2.5mg, 150±2.5mg, 155±2.5mg, 160±2.5mg, 165±2.5mg, 170±2.5mg, 175±2.5 mg, 180±2.5mg, 185±2.5mg, 190±2.5mg, 195±2.5mg, 200±2.5mg, 205±2.5mg, or 210±5mg, 220±5mg, 230±5mg, 240±5mg, 250±5mg, 260±5mg, 270±5mg, 280±5mg, 290±5mg, 300±5mg, 310±5mg, 320±5mg, 330±5mg, 340±5mg, 350±5mg, 360±5mg, ±5mg, 370±5mg, 380±5mg, 390±5mg, 400±5mg, 410±5mg, 420±5mg, 430±5mg, 440±5mg, 450±5mg, 460±5mg, 470±5mg, 480±5mg, 490 ±5mg, 500±5mg.

### 4. Disintegrant

The pharmaceutical compositions provided by the present invention can optionally comprise a disintegrant, and in some embodiments preferably comprise one or more pharmaceutically acceptable disintegrants as carrier material, particularly for tablet formulations.

In some embodiments, the disintegrant is selected from at least one of microcrystalline cellulose, crospovidone, sodium carboxymethyl starch, croscarmellose sodium, low-substituted hydroxypropyl cellulose, dry starch and carboxymethylcellulose calcium.

The mass percentage content of the disintegrant in the pharmaceutical compositions provided by the present invention is 5 to 40%.

In some embodiments, the disintegrant is preferably selected from one or two or more of microcrystalline cellulose, crospovidone, low-substituted hydroxypropyl cellulose, croscarmellose sodium.

In some embodiments, the disintegrant is the combination of microcrystalline cellulose and any one selected from the group consisting of crospovidone, low-substituted hydroxypropyl cellulose, croscarmellose sodium.

In some embodiments, the mass percentage content of the disintegrant is 10 to 30%.

As a specific embodiment, the mass percentage content of the disintegrant in the pharmaceutical compositions of the present invention is 5 to 9%, 6 to 10%, 11 to 15%, 12 to 16%, 13 to 17%, 14 to 18%, 15 to 19%, 16 to 20%, 21 to 25%, 22 to 26%, 23 to 27%, 24 to 28%, 25 to 29%, 26 to 30%, 31 to 35%, 32 to 36%, 33 to 37%, 34 to 38%, 35 to 39%, 36 to 40%.

As a specific embodiment, the mass of the disintegrant in the pharmaceutical compositions of the present invention is 30±2.5 mg, 35±2.5 mg, 40±2.5 mg, 45±2.5 mg, 50±2.5 mg, 55± 2.5mg, 60±2.5mg, 65±2.5mg, 70±2.5mg, 75±2.5mg, 80±2.5mg, 85±2.5mg, 90±2.5mg, 95±2.5mg, 100±2.5mg, 105± 2.5mg, 110±2.5mg, 115±2.5mg, 120±2.5mg, 125±2.5mg, 130±2.5mg, 135±2.5mg, 140±2.5mg, 145±2.5mg, 150±2.5mg, 155± 2.5mg, 160±2.5mg, 165±2.5mg, 170±2.5mg, 175±2.5mg, 180±2.5mg, 185±2.5mg, 190±2.5mg, 195±2.5mg, 200±2.5mg, 205± 2.5mg, or 210±5mg, 220±5mg, 230±5mg, 240±5mg, 250±5mg, 260±5mg, 270±5mg, 280±5mg, 290±5mg, 300±5mg.

In some embodiments, the disintegrant is low-substituted hydroxypropyl cellulose, wherein the hydroxypropoxyl content of the low-substituted hydroxypropyl cellulose is 5 to 12 %.

### 5. Binder

The pharmaceutical compositions provided by the present invention can optionally contain a binder, and in some embodiments preferably comprise one or more pharmaceutically acceptable binders as carrier material, particularly for tablet formulations. Such binders ideally impart sufficient cohesion to the tableted powder to permit normal processing operations such as sizing, lubrication, compression, and packaging, but still allow disintegration of the tablets and absorption of the composition upon ingestion.

In an embodiment, the binder is selected from pregelatinized starch, starch, lactose, methyl cellulose, ethyl cellulose, povidone, copovidone, hydroxypropyl cellulose, hydroxypropyl methylcellulose, sodium carboxymethyl cellulose, polyethylene glycol, polyvinylpyrrolidone, gelatin, sucrose, sodium alginate, carobbean gum, chitosan, dextrin and glyceryl behenate.

In the pharmaceutical compositions provided by the present invention, the mass percentage content of the binder is selected from 5 to 40%.

In some embodiments, the binder is preferably selected from one or two or more of pregelatinized starch, lactose, hypromellose.

In some embodiments, the mass percentage content of the binder is 10 to 30%.

As a specific embodiment, the mass percentage of the binder is 11 to 15%, 12 to 16%, 13 to 17%, 14 to 18%, 15 to 19%, 16 to 20%, 21 to 25 %, 22 to 26%, 23 to 27%, 24 to 28%, 25 to 29%, 26 to 30%.

As a specific embodiment, the mass of the binder in the pharmaceutical compositions of the present invention is 30±2.5mg, 35±2.5mg, 40±2.5mg, 45±2.5mg, 50±2.5mg, 55± 2.5mg, 60±2.5mg, 65±2.5mg, 70±2.5mg, 75±2.5mg, 80±2.5mg, 85±2.5mg, 90±2.5mg, 95±2.5mg, 100±2.5mg, 105± 2.5mg, 110±2.5mg, 115±2.5mg, 120±2.5mg, 125±2.5mg, 130±2.5mg, 135±2.5mg, 140±2.5mg, 145±2.5mg, 150±2.5mg, 155± 2.5mg, 160±2.5mg, 165±2.5mg, 170±2.5mg, 175±2.5mg, 180±2.5mg, 185±2.5mg, 190±2.5mg, 195±2.5mg, 200±2.5mg, 205± 2.5mg, or 210±5mg, 220±5mg, 230±5mg, 240±5mg, 250±5mg, 260±5mg, 270±5mg, 280±5mg, 290±5mg, 300±5mg.

### 6. Lubricant

In some embodiments, the compositions of the present invention optionally comprise one or more pharmaceutically acceptable lubricants as carrier material.

In an embodiment, the lubricant is selected from stearic acid, magnesium stearate, sodium stearyl fumarate, glyceryl behenate, micropowder silica gel, talcum powder, polyethylene glycol, hydrogenated vegetable oil, sodium lauryl sulfate and glyceryl monostearate.

In the pharmaceutical compositions provided by the present invention, the mass percentage content of the lubricant is selected from 0.1 to 10%.

In some embodiments, the lubricant is preferably selected from one or two or more of magnesium stearate, micropowder silica gel and talcum powder.

In some embodiments, the mass percentage content of the lubricant is 0.2% to 5%.

As a specific embodiment, the mass percentage content of the lubricant is 0.1 to 0.6%, 0.2 to 0.7%, 0.3 to 0.8%, 0.4 to 0.9%, 0.5 to 1.0%, 1.1 to 1.6%, 1.2 to 1.7%, 1.3 to 1.8%, 1.4 to 1.9%, 1.5 to 2.0%, 2.1 to 2.6%, 2.2 to 2.7%, 2.3 to 2.8%, 2.4 to 2.9%, 2.5 to 3.0%, 3.1 to 3.6%, 3.2 to 3.7%, 3.3 to 3.8%, 3.4 to 3.9%, 4.5 to 5.0%, 5.1 to 5.6%, 5.2 to 5.7%, 5.3 to 5.8%, 5.4 to 5.9%, 5.5 to 6.0%, 6.1 to 6.6%, 6.2 to 6.7%, 6.3 to 6.8%, 6.4 to 6.9%, 6.5 to 7.0%, 7.1 to 7.6%, 7.2 to 7.7%, 7.3 to 7.8%, 7.4 to 7.9%, 7.5 to 8.0%, 8.1 to 8.6%, 8.2 to 8.7%, 8.3 to 8.8%, 8.4 to 8.9%, 8.5 to 9.0%, 9.1 to 9.6%, 9.2 to 9.7%, 9.3 to 9.8%, 9.4 to 9.9%, 9.5 to 10.0%.

As a specific embodiment, the mass of the lubricant in the pharmaceutical compositions of the present invention is 0.1 to 40 mg, 0.2 to 30 mg, 0.3 to 20 mg, 0.4 to 15 mg, 0.5 to 10 mg, or 1 to 6 mg, 2 to 7mg, 3 to 8mg, 4 to 9mg, 5 to 10mg, 11 to 16mg, 12 to 17mg, 13 to 18mg, 14 to 19mg, 15 to 20mg, 21 to 26mg, 22 to 27mg, 23 to 28mg, 24 to 29mg, 25 to 30mg.

As a specific embodiment, the mass of the lubricant in the pharmaceutical compositions of the present invention is 1±0.5 mg, 2±0.5 mg, 3±0.5 mg, 4±0.5 mg, 5±0.5 mg, 6±0.5 mg, 7±0.5mg, 8±0.5mg, 9±0.5mg, 10±0.5mg, 11±0.5mg, 12±0.5mg, 13±0.5mg, 14±0.5mg, 15±0.5mg, 16±0.5 mg, 17±0.5mg, 18±0.5mg, 19±0.5mg, 20±0.5mg.

### 7. Wetting Agent

The pharmaceutical compositions provided by the present invention can also optionally comprise a wetting agent.

In some embodiments, the wetting agent is selected from ethanol, glycerol and tween.

In the pharmaceutical compositions provided by the present invention, the mass percentage content of the wetting agent is selected from 0.1 to 10%.

In some embodiments, the wetting agent is preferably tween 80.

In some embodiments, the mass percentage content of the wetting agent is 1% to 10%.

As a specific embodiment, the mass percentage content of the wetting agent is 0.1 to 0.6%, 0.2 to 0.7%, 0.3 to 0.8%, 0.4 to 0.9%, 0.5 to 1.0%, 1.1 to 1.6%, 1.2 to 1.7%, 1.3 to 1.8%, 1.4 to 1.9%, 1.5 to 2.0%, 2.1 to 2.6%, 2.2 to 2.7%, 2.3 to 2.8%, 2.4 to 2.9%, 2.5 to 3.0%, 3.1 to 3.6%, 3.2 to 3.7%, 3.3 to 3.8%, 3.4 to 3.9%, 4.5 to 5.0%, 5.1 to 5.6%, 5.2 to 5.7%, 5.3 to 5.8%, 5.4 to 5.9%, 5.5 to 6.0%.

As a specific embodiment, the mass of the wetting agent in the pharmaceutical compositions of the present invention is 0.5 to 50 mg, 1 to 30 mg, 2 to 20 mg, or 2 to 5 mg, 6 to 9 mg, 10 to 14 mg, 15 to 19mg, 20 to 24mg, 25 to 29mg, 30 to 34mg, 35 to 39mg, 40 to 44mg, 45 to 49mg.

As a specific embodiment, the mass of the wetting agent in the pharmaceutical compositions of the present invention is 3±1 mg, 6±1 mg, 9±1 mg, 12±1 mg, 15±1 mg, 18±1 mg, 21±1 mg , 24±1mg, 27±1mg, 30±1mg, 33±1mg, 36±1mg, 39±1mg, 42±1mg, 45±1mg, 48±1mg.

### 8. Glidant

In some embodiments, the compositions of the present invention optionally comprise one or more pharmaceutically acceptable glidants as carrier material.

In some embodiments, the glidant is selected from micropowder silica gel, talcum powder and colloidal silicon dioxide.

In the pharmaceutical compositions provided by the present invention, the mass percentage content of the glidant is selected from 0.1 to 10%.

In some embodiments, the glidant is preferably micropowder silica gel or colloidal silicon dioxide.

In some embodiments, the mass percentage content of the glidant is 0.2% to 5%.

As a specific embodiment, the mass percentage content of the glidant is 0.1 to 0.6%, 0.2 to 0.7%, 0.3 to 0.8%, 0.4 to 0.9%, 0.5 to 1.0%, 1.1 to 1.6%, 1.2 to 1.7%, 1.3 to 1.8%, 1.4 to 1.9%, 1.5 to 2.0%, 2.1 to 2.6%, 2.2 to 2.7%, 2.3 to 2.8%, 2.4 to 2.9%, 2.5 to 3.0%, 3.1 to 3.6%, 3.2 to 3.7%, 3.3 to 3.8%, 3.4 to 3.9%, 4.5 to 5.0%, 5.1 to 5.6%, 5.2 to 5.7%, 5.3 to 5.8%, 5.4 to 5.9%, 5.5 to 6.0%, 6.1 to 6.6%, 6.2 to 6.7%, 6.3 to 6.8%, 6.4 to 6.9%, 6.5 to 7.0%, 7.1 to 7.6%, 7.2 to 7.7%, 7.3 to 7.8%, 7.4 to 7.9%, 7.5 to 8.0%, 8.1 to 8.6%, 8.2 to 8.7%, 8.3 to 8.8%, 8.4 to 8.9%, 8.5 to 9.0%, 9.1 to 9.6%, 9.2 to 9.7%, 9.3 to 9.8%, 9.4 to 9.9%, 9.5 to 10.0%.

As a specific embodiment, the mass of the glidant in the pharmaceutical compositions of the present invention is 0.1 to 40 mg, 0.2 to 30 mg, 0.3 to 20 mg, 0.4 to 15 mg, 0.5 to 10 mg, or 1 to 6 mg, 2 to 7mg, 3 to 8mg, 4 to 9mg, 5 to 10mg, 11 to 16mg, 12 to 17mg, 13 to 18mg, 14 to 19mg, 15 to 20mg, 21 to 26mg, 22 to 27mg, 23 to 28mg, 24 to 29mg, 25 to 30mg.

As a specific embodiment, the mass of the glidant in the pharmaceutical compositions of the present invention is 1±0.5mg, 2±0.5mg, 3±0.5mg, 4±0.5mg, 5±0.5mg, 6±0.5mg, 7±0.5mg, 8±0.5mg, 9±0.5mg, 10±0.5mg, 11±0.5mg, 12±0.5mg, 13±0.5mg, 14±0.5mg, 15±0.5mg, 16± 0.5mg, 17±0.5mg, 18±0.5mg, 19±0.5mg, 20±0.5mg.

### 9. Acidifier

In some embodiments, the pharmaceutical compositions of the present invention optionally comprise one or more acidifiers as carrier material.

In some embodiments, the acidifier is selected from tartaric acid, citric acid, propionic acid, phosphoric acid, malic acid, lactic acid and hydrochloric acid.

In the pharmaceutical compositions provided by the present invention, the mass percentage content of the acidifier in selected from 1% to 40%.

In some embodiments, the acidifier is preferably selected from citric acid or anhydrous citric acid.

In some embodiments, the mass percentage content of the acidifier is 5% to 20%.

### 10. Absorption Enhancer

The pharmaceutical compositions of the present invention optionally comprise one or more absorption enhancers.

In some embodiments, the absorption enhancer is selected from oleic acid, laurocapram, menthol, vitamin E-TPGS, propylene glycol, β-cyclodextrin, hydroxypropyl-β-cyclodextrin, sodium lauryl sulfate, N-methyl-2-pyrrolidone, dimethyl sulfoxide, ethanol, ethylene glycol, ethoxydiglycol, bisabolol, liposome, azone, piperine, sodium caprylate, sodium caprate, sodium caproate, decanoyl carnitine, sucrose ester and chitosan.

In the pharmaceutical compositions provided by the present invention, the mass percentage content of the absorption enhancer in selected from 0.1% to 10%.

In some embodiments, the absorption enhancer is preferably selected from one or two or more of vitamin E, oleic acid, propylene glycol, hydroxypropyl-β-cyclodextrin.

In some embodiments, the mass percentage content of the absorption enhancer is 0.5% to 5%.

### 11. Surfactant

The pharmaceutical compositions of the present invention optionally, but preferably comprise one or more pharmaceutically acceptable surfactants as carrier material. Such surfactants are preferably selected to keep OAD2 or a pharmaceutically acceptable salt thereof tightly bound to water.

In some embodiments, the surfactant is selected from lecithin, fatty acid glyceride, sucrose fatty acid ester, span, tween, myrij, brij, poloxamer, sodium dodecyl sulfate, sodium hexadecyl sulfate, sodium octadecyl sulfate, sodium dioctyl sulfosuccinate, sodium dihexyl sulfosuccinate, sodium dodecylbenzenesulfonate, benzyl benzoate and docusate sodium.

In some embodiments, the mass percentage content of the surfactant is selected from 0.2% to 20%.

### 12. Antioxidant

The compositions of the present invention optionally comprise one or more antioxidants. The term "antioxidant" refers to a component in a composition that may prevent and/or inhibit the formation of unacceptable amounts of oxidative degradants in the compositions after a certain period of shelf life. In some embodiments, the antioxidant may react with oxygen that might otherwise compromise the compositions by producing impurities in the compositions. Oxygen may originate from the compositions' environment or the compositions *per se.* For example, oxygen may originate from residual oxygen present in the headspace of vials containing a composition. In some embodiments, the oxidative degradants comprise impurity B, and the antioxidant may limit the formation of oxidative degradants (such as impurity B) in the compositions to less than about 5%, or less than about 4%, or less than about 3%, or less than about 2.5%, or less than about 2%, or less than about 1.5%, or less than about 1 %, or less than about 0.9%, or less than about 0.8%, or less than about 0.7%, or less than about 0.6%, or less than about 0.5%, or less than about 0.4%, or less than about 0.3%, or less than about 0.2%, or less than about 0.1%, or less than about 0.05%, or less than about 0.04%, or less than about 0.03%, or less than about 0.02%, or less than about 0.01% after a certain period of shelf life.

In some embodiments, the antioxidant may be present at the lowest concentration that will inhibit and/or prevent the composition from undergoing unacceptable physical changes. In some embodiments, the antioxidant may be present at the lowest concentration that will inhibit and/or prevent unacceptable oxidation of components of the composition.

In some embodiments, the antioxidant is selected from sulfite, ascorbic acid, thiourea, cysteine, ascorbyl palmitate, α-tocopherol, dibutyl cresol, dibutyl thiodiacetate, tartaric acid, citric acid, disodium edetate, tocopheryl polyethylene glycol succinate, vitamin E and derivatives thereof, etc.

In some embodiments, the mass percentage content of the antioxidant is selected from 0.5% to 10%.

As for the pharmaceutical compositions of the present invention, mass percentage content refers to the percentage relative to the total weight of the drug, wherein the total weight of the drug does not include the weight of the coating. As for a specific dosage form, it is for example the weight relative to the tablet core or the granule.

In the pharmaceutical compositions of OAD2 or a pharmaceutically acceptable salt thereof provided by the present invention, the mass percentage of each component is:

| | |
|---|---|
| Active ingredient | 1 to 60% |
| Solubilizer | 0.1 to 10% |
| Filler | 10 to 75% |
| Disintegrant | 5 to 40% |
| Optionally, binder | 5 to 40% |
| Optionally, lubricant | 0.2 to 5% |
| Optionally, wetting agent | 0.1 to 10% |
| Optionally, glidant | 0.1 to 10% |
| Optionally, acidifier | 1 to 40% |
| Optionally, absorption enhancer | 0.1 to 10% |
| Optionally, surfactant | 0.1 to 5% |
| Optionally, antioxidant | 0.2 to 20% |

In the pharmaceutical compositions of OAD2 or a pharmaceutically acceptable salt thereof provided by the present invention, the mass percentage of each component is:

| | |
|---|---|
| Active ingredient | 5 to 40% |
| Solubilizer | 0.2 to 5% |
| Filler | 20 to 60% |
| Disintegrant | 10 to 30% |
| Optionally, binder | 10 to 30% |
| Optionally, lubricant | 0.2 to 5% |
| Optionally, wetting agent | 0.2 to 5% |
| Optionally, glidant | 0.2 to 5% |
| Optionally, acidifier | 5 to 30% |
| Optionally, absorption enhancer | 0.2 to 5% |
| Optionally, surfactant | 0.1 to 2.5% |
| Optionally, antioxidant | 0.5 to 10% |

In the pharmaceutical compositions of OAD2 or a pharmaceutically acceptable salt thereof provided by the present invention, the mass percentage of each component is:

| Ingredients | Mass percentage |
|---|---|
| Active ingredient | 12% |
| Solubilizer | 1% |
| Filler | 30% |
| Disintegrant | 18% |
| Binder | 21% |
| Lubricant | 0.5% |
| Wetting agent | 3.0% |
| Glidant | 0.5% |
| Acidifier | 12.5% |
| Absorption enhancer | 1.5% |

Suitable fillers (alone or in combination) also include: lactose USP; lactose USP, anhydrous; lactose USP, spray dried; starch USP; pregelatinized starches (e.g., National 1511 and Starch 1500); direct compressible starches; mannitol USP; sorbitol; dextrose monohydrate; microcrystalline cellulose NF; calcium hydrogen phosphate dihydrate NF; sucrose-based filler; powdered sugar (confectioner's sugar); monobasic calcium sulfate monohydrate; calcium sulfate dihydrate NF; calcium lactate trihydrate granular NF; dextrates, inositol; hydrolyzed grain solids such as Maltrons and Mor-Rex; amylase; Rexcel; powdered cellulose (e.g., Elcema); calcium carbonate; glycine; bentonite; polyvinylpyrrolidone, et al.. Such fillers, if present, constitute in total from about 5% to about 99%, or from about 10% to about 85%, or from about 20% to about 60%, or from about 10% to about 40%, or from about 15wt% to about 30wt%, or from about 10wt% to about 20wt%, or from about 15wt% to about 25wt%, or from about 20wt% to about 30wt%, or from about 25wt% to about 35wt%, or from about 30wt% to about 40wt% of the total weight of the compositions. The selected one or more fillers preferably exhibit suitable flowability and, where a tablet is required, also exhibit compressibility.

In further embodiments, lactose, pregelatinized starch and microcrystalline cellulose (alone or in combination) are preferred fillers. In another embodiment, the filler comprises microcrystalline cellulose and/or pregelatinized starch in an amount ranging from 20 wt% to 60 wt% of the total weight of the compositions. In another embodiment, the filler comprises microcrystalline cellulose in an amount ranging from 20 wt% to 40 wt% of the total weight of the compositions. In another embodiment, the filler constitutes in total about 5 wt%, 10 wt%, 15 wt%, 20 wt%, 25 wt%, or 30 wt% ± 2.5 wt% of the total weight of the compositions.

Suitable disintegrants (alone or in combination) also include: starches; sodium starch glycolate; clays (such as Veegum HV); celluloses (such as purified celluloses, methylcellulose, sodium carboxymethylcellulose and carboxymethylcellulose); alginate; pregelatinized corn starch (e.g. National 1551 and National 1550); crospovidone USP NF; and gums (e.g. agar, guar, locust bean gum, carrageenan, pectin and tragacanth). Disintegrants may be added at any suitable step during the preparation of the compositions, in particular as an extragranular excipient prior to granulation or prior to compression. Such disintegrants, if present, constitute in total from about 0.2 wt% to about 30 wt%, or from about 0.2 wt% to about 10 wt%, or from about 0.2 wt% to about 5 wt%, of the total weight of the compositions. In another embodiment, the disintegrant constitutes in total from about 5 wt%, 10 wt%, 15 wt%, 20 wt%, 25 wt%, or 30 wt% ± 2.5 wt% of the total weight of the compositions.

Crospovidone (cross-linked polyvinylpyrrolidone) is the preferred disintegrant for tablet or capsule disintegration and, if present, may constitute from about 0.1 wt% to about 20 wt%, or about 0.2 wt% to about 10 wt%, or about 0.2 wt% to about 6 wt%, or about 0.2 wt% to about 5 wt%, or about 1 wt% to about 5 wt% of the total weight of the compositions. In another embodiment, crospovidone constitutes in total about 2 wt%, 5 wt%, 10 wt%, 15 wt%, 20 wt%, 25 wt%, or 30 wt% ± 1.5 wt% of the total weight of the compositions. The crospovidone used in the pharmaceutical compositions (European Pharmacopoeia, United States Pharmacopoeia-National Formulary, Japanese Pharmacopoeia, Ph.Eur., USP-NF, JP) can be of European Pharmacopoeia Crospovidone Monograph Class A or Class B quality. In another embodiment, the amount of peroxides in crospovidone as measured by the European Pharmacopoeia Crospovidone Monograph Class A method does not exceed 50 ppm, or 45 ppm, or 40 ppm, or 35 ppm, or 30 ppm, or 25 ppm, or 20ppm, or 15ppm, or 10ppm. In another embodiment, the amount of peroxides in the crospovidone as measured by the European Pharmacopoeia Crospovidone Monograph Type B method does not exceed 125 ppm, or 100 ppm, or 75 ppm, or 50 ppm, or 25 ppm. In another embodiment, the quality of crospovidone is equivalent to the quality of grade "Ultra" or "Ultra-10" available from Ashland under the tradename Polyplasdone^{™} Ultra. In another embodiment, the typical average particle size of crospovidone is 110-140 microns or 25-40 microns.

Binders may also include (alone or in combination): gum arabic; tragacanth; sucrose; gelatin; glucose; starch; cellulosic materials such as, but not limited to, methylcellulose and sodium carboxymethylcellulose (e.g., Tylose); alginic acid and alginate; magnesium aluminum silicate; polyethylene glycol; guar gum; polysaccharide acid; bentonite; polyvinylpyrrolidone; copovidone (copolymer of vinylpyrrolidone and vinyl acetate); polymethacrylates; hydroxypropylmethylcellulose (HPMC); hydroxypropylcellulose (Klucel); ethylcellulose (Ethocel); pregelatinized starches (such as National 1511 and Starch 1500). Such binders, if present, constitute in total from about 0.1 wt% to about 20 wt%, or from about 0.75 wt% to about 15 wt%, or from about 1 wt% to about 10 wt%, or from about 1 wt% to about 5wt% of the total weight of the compositions.

Crospovidone is the preferred binder for tablet or capsule disintegration and, if present, constitutes from about 0.1 wt% to about 10 wt%, or from about 0.2 wt% to about 5 wt%, or from about 0.3 wt% to about 4 wt%, or from about 0.4 wt% to about 3 wt%, or from about 0.5 wt% to about 1 wt% of the total weight of the compositions. In another embodiment, the amount of crospovidone present in the compositions is about 0.5 wt%, 1.0 wt%, 1.5 wt%, 2.0 wt%, 2.5 wt%, 3.0 wt%, 3.5 wt%, 4.0 wt%, 4.5 wt%, or 5.0 wt% ± 0.5 wt% of the total weight of the compositions.

Suitable lubricants (alone or in combination) also include: glyceryl behapate (Compritol 888); stearates (magnesium, calcium and sodium); stearic acid; hydrogenated vegetable oils (e.g., Sterotex); talcum powder; wax; Stearowet; boric acid; sodium benzoate; sodium acetate; sodium fumarate; sodium chloride; DL-leucine; polyethylene glycols (e.g., Carbowax 4000 and Carbowax 6000); sodium oleate; sodium lauryl sulfate; and magnesium lauryl sulfate. Such lubricants, if present, constitute in total from about 0.1 wt% to about 10 wt%, from about 0.2 wt% to about 8 wt%, or from about 0.25 wt% to about 5 wt% of the total weight of the compositions.

In some embodiments, magnesium stearate is a preferred lubricant and is present in an amount of 0.25 wt% to about 1.5 wt% of the total weight of the compositions.

Suitable glidants (alone or in combination) also include magnesium trisilicate, powdered cellulose, starch, talcum powder, tricalcium phosphate, stearates and colloidal silicon dioxide, and the preferred glidant is colloidal silicon dioxide. Such glidants, if present, constitute in total from about 0.1 wt% to about 10 wt%, or from about 0.2 wt% to about 8 wt%, or preferably from about 0.25 wt% to about 5 wt% of the total weight of the compositions.

In one embodiment, the glidant comprises colloidal silicon dioxide and is present in an amount of 0.25 wt% to about 1.5 wt% of the total weight of the compositions.

Suitable acidifiers (alone or in combination) also include acetic acid, amino acids, citric acid, nitric acid, fumaric acid and other α-hydroxy acids, hydrochloric acid, ascorbic acid and nitric acid and other acids known to those of ordinary skill in the art. In a preferred embodiment, the acidifier is citric acid.

Such acidifiers, if present, constitute in total from about 0.1 wt% to about 50 wt%, from about 1 wt% to about 50 wt%, from about 1 wt% to about 10 wt%, from about 5 wt% to about 15 wt%, from about 10 wt% to about 20 wt%, from about 15 wt% to about 25 wt%, from about 20 wt% to about 30 wt%, from about 25 wt% to about 35 wt% of the total weight of the compositions.

In one embodiment, the acidifier includes citric acid. In another embodiment, the acidifier is citric acid and is present in an amount ranging from 5 wt% to 15 wt% or from 25 wt% to 35 wt% of the total weight of the compositions.

Absorption enhancers can also be surface active agents, which act both as dissolution enhancers and uptake enhancers. Solubility enhancers can improve the ability of active ingredients in dissolving in the aqueous environment to which they are initially released, or in the lipophilic environment of the mucus layer lining the intestinal wall, or both. Transit (uptake) enhancers (which are usually the same as the surfactants used as dissolution enhancers) are those that facilitate the easy passage of the active ingredient through intestinal wall.

One or more absorption enhancers may only perform one function (e.g., dissolution), or one or more absorption enhancers may only perform another function (e.g., uptake). It is also possible to have a mixture of several compounds, some of which provide improved solubility, some of which provide improved uptake and/or some of which perform both functions.

Surfactants can be used as dissolution enhancers and uptake enhancers. Non-limiting examples of absorption enhancers include: salicylates such as sodium salicylate, 3-methoxysalicylate, 5-methoxysalicylate, and homovanilate; cholic acids such as taurochol acid, tauorodeoxycholic acid, and deoxycholic acid; nonionic surfactants such as polyoxyethylene ethers, p-t-octylphenoxy polyoxyethylene ether, nonylphenoxypolyoxyethylene ether, polyoxyethylene sorbitan esters (such as Tween-20, Tween-80, etc.), d-α tocopheryl polyethylene glycol 1000 succinate (vitamin E TPGS); anionic surfactants, such as dioctyl sodium sulfosuccinate; lysophospholipids such as lysophosphatidylcholine and lysophosphatidylethanolamine; acylcarnitines, acylcholines, and acylamino acids such as lauroylcarnitine, lauroylcholine and cetyllysine; medium-chain glycerides, which are mixtures containing monoglycerides, diglycerides, and triglycerides of medium-chain length fatty acids (caprylic acid, capric acid, lauric acid, etc.), fatty acid derivatives of polyethylene glycol (such as caprylocaproyl macrogolglycerides (Labrasol), caprylic triglyceride (Labrafac), etc.) and alkyl sugars (such as lauroyl maltoside, lauroyl sucrose, myristoyl sucrose and palmitoyl sucrose).

Such absorption enhancers, if present, constitute in total from about 0.1 wt% to about 5 wt%, or from about 0.25 wt% to about 5 wt%, or from about 0.5 wt% to about 4 wt% of the total weight of the compositions.

In a preferred embodiment, the absorption enhancer comprises Vitamin E TPGS in an amount from 0.5 wt% to 5 wt% or from 0.5 wt% to 2.5 wt% of the total weight of the compositions.

Suitable surfactants (alone or in combination) also include oleic acid, glyceryl monostearate, sorbitan monooleate, sorbitan monolaurate, triethanolamine oleate, polyoxyethylene sorbitan monooleate (Tween 80), d-α tocopheryl macrogol 1000 succinate (Vitamin E TPGS), polyoxyethylene sorbitan monolaurate, sodium oleate, sodium laurylsulfate, poloxamer and poloxamer 188. Such surfactants, if present, constitute in total from about 0.25 wt% to about 15 wt%, or from about 0.4 wt% to about 10 wt%, or from about 3 wt% to about 9 wt% of the total weight of the compositions.

In one embodiment, the surfactant comprises one or two surfactants selected from polyoxyethylene sorbitan monooleate and poloxamer 188, wherein the surfactant is present in an amount from 0.5 wt% to 5 wt% of the total weight of the compositions. In another embodiment, the surfactant is present in the compositions in an amount of about 0.5 wt%, 1.0 wt%, 1.5 wt%, 2.0 wt%, 2.5 wt%, 3.0 wt%, 3.5 wt%, 4.0wt%, 4.5wt% or 5.0wt%±0.5wt% of the total weight of the compositions.

Suitable antioxidants (alone or in combination) also include amino acid sulfites (such as L-lysine sulfite), ascorbic acid, ascorbyl palmitate, benzotriazoles, butylhydroxyanisole (BHA), butylated hydroxytoluene (BHT), citric acid, cysteine, cysteine hydrochloride, calcium disodium EDTA, disodium EDTA, dithiothreitol, dl-α-tocopherol, erythorbic acid, ethoxyquinoline, EDTA salt, fumaric acid, glutathione, guaiac, homocysteine, isopropyl citrate, l-ascorbic acid stearate, thioglycerin, nordihydroguaiaretic acid (NDGA), palmitic acid, ascorbic acid, sodium ascorbate, sodium bisulfite, tetrasodium EDTA, sodium erythorbate, sodium bisulfite, sodium metabisulfite, sodium pyrosulfite, 1,3-butanediol, sodium sulfite, sodium thioglycolate, sodium thiosulfate, tert-butylhydroquinone, thioglycerol, thiourea, TPGS (tocopheryl polyethylene glycol succinate), vitamin E or its derivatives, α-thioglycerol and/or its salts.

In some embodiments, the antioxidant may be present at a concentration ranging from about 0.1 to 9.0 wt%, or at a concentration ranging from about 0.5 and 5.0 wt%, or at a concentration ranging from about 0.3 and 1.5 wt%, or at a concentration ranging from about 0.4 and 1.6 wt%, or at a concentration ranging from 0.5 and 1.7 wt%, or at a concentration ranging from 0.6 and 1.8 wt%, or at a concentration ranging from 0.7 and 1.9 wt%, or at a concentration ranging from 0.8 and 2.0 wt%, or at a concentration ranging from 1.0 to 2.5 wt%, or at a concentration ranging from 1.5 to 3.0 wt%, or at a concentration ranging from 2.0 and 3.5 wt%, or at a concentration ranging from 2.5 and 4.0 wt%, or at a concentration ranging from 3.0 and 5.0 wt%.

### Preparation method of pharmaceutical compositions of OAD2

In some embodiments, the pharmaceutical compositions of OAD2 or a pharmaceutically acceptable salt thereof provided by the present invention are oral pharmaceutical compositions.

Liquid formulations (e.g., suspensions, tinctures, etc.) suitable for oral administration of the pharmaceutical compositions provided by the present invention can be prepared according to techniques known in the art, while any commonly used media, e.g., water, ethylene glycol, oils, alcohols, and the like can be used. Solid formulations suitable for oral administration (e.g., tablets, capsules, granules, and pills) can be prepared according to techniques known in the art, while some solid excipients, such as fillers, binders, disintegrants, and the like can be used.

In some embodiments, the pharmaceutical compositions of OAD2 or a pharmaceutically acceptable salt thereof provided by the present invention are solid pharmaceutical compositions.

In some embodiments, the pharmaceutical compositions of OAD2 or a pharmaceutically acceptable salt thereof provided by the present invention are in a dosage form selected from the group consisting of tablets, pills, capsules, mini-tablets or granules, wherein the tablets, pills, and granules may or may not have a coating.

As a specific embodiment, the pharmaceutical compositions of the present invention may further comprise a coating comprising one or more selected from the group consisting of water-soluble polymers, water-insoluble polymers, gastric-soluble polymers, and enteric-soluble polymers as the coating layer.

As a specific embodiment, the pharmaceutical compositions of OAD2 or a pharmaceutically acceptable salt thereof provided by the present invention are tablets.

The present invention further provides a preparation method of the pharmaceutical composition, which mainly comprises the following steps:
a. processing raw auxiliary materials: sieving filler, disintegrant, solubilizer, and optionally other auxiliary materials, then putting aside;
b. granulating and mixing: subjecting formulated amounts of OAD2 or a pharmaceutically acceptable salt thereof, filler, solubilizer, disintegrant, and optionally binder, acidifer, lubricant, wetting agent, acidifer, glidant, absorption enhancer and surfactant to granulating and mixing;
c. mixing the granules with filler, disintegrating agent, lubricant, glidant, acidifer and other auxiliary materials as appropriate, and tableting the mixture, wherein the hardness is controlled to be 8-20 Kg.

The compositions of the present invention comprise OAD2 or a pharmaceutically acceptable salt thereof in association with one or more pharmaceutically acceptable excipients suitable for oral administration.

Compositions of the present invention can be adapted for administration by any suitable oral route by selection of appropriate excipient materials and a dosage of the active ingredient effective for the treatment intended. Accordingly, any carrier materials employed can be solids or liquids, or both, and the compositions contain about 1% to about 95%, or about 10% to about 90%, or about 25% to about 85%, or about 10% to about 40%, by weight of OAD2 or a pharmaceutically acceptable salt thereof. Such pharmaceutical compositions can be prepared by any of the well-known techniques of pharmacy comprising admixing the components.

The compositions of the present invention contain a desired amount of OAD2 or a pharmaceutically acceptable salt thereof per dose unit and can be in the form of, for example, a tablet, a pill, a hard or soft capsule, a lozenge, a cachet, a dispensable powder, granules, a suspension, an elixir, a liquid, or any other form reasonably adapted for oral administration. Such a composition may be made in the form of discrete dose units each containing a predetermined amount of OAD2 or a pharmaceutically acceptable salt thereof, such as tablets, pills, or capsules. In addition, tablets, pills, and the like can be prepared with or without coatings.

Compositions of the present invention can be prepared by any suitable method of pharmacy which includes the step of bringing into association the OAD2 or a pharmaceutically acceptable salt thereof and carrier materials. In general, the compositions are prepared by uniformly and intimately admixing the OAD2 or a pharmaceutically acceptable salt thereof with liquid or finely divided solid carriers, or both, and then, if necessary, encapsulating or shaping the product.

For example, tablets can be prepared by compressing or molding a powder or granules of the compound, together with one or more excipients. Compressed tablets can be prepared by compressing, in a suitable machine, a free-flowing composition, such as a powder or granules, comprising the OAD2 or a pharmaceutically acceptable salt thereof mixed with one or more excipients. Molded tablets can be made by molding, in a suitable machine, a powdered compound moistened with an inert liquid filler.

These pharmaceutical excipients may be appropriately added alone, or as a combination of two or more pharmaceutical excipients in appropriate amounts. With respect to the contents of the pharmaceutical excipients, each excipient may be used in an amount such that the desired effects of the present invention may be achieved.

Compositions for oral administration of the present invention can be produced by known methods comprising the steps of, for example, mixing, granulation, drying, molding (tableting), film coating, and the like. The method of manufacturing compositions for oral administration of the present invention will be explained below.

Prior to mixing, the active ingredient and any solid excipients may be pulverized and/or sieved in an ordinary pharmaceutical manner. Examples of a pulverizer include a hammer mill, a ball mill, a jet mill, a colloid mill, and the like. The conditions for pulverization may be appropriately selected and are not particularly limited.

In the step of mixing components, both the apparatus and the means are not particularly limited, so long as it is a method in which the components can be uniformly mixed in an ordinary pharmaceutical manner.

In the granulation step, both the apparatus and the means are not particularly limited, so long as it is a method in which the active ingredient and appropriate excipients can be granulated in an ordinary pharmaceutical manner.

Examples of a granulation method and a granulation apparatus which are used in a wet granulation using a solvent such as water include a high shear granulation method, a milling (pulverization) granulation method, a fluidized bed granulation method, an extrusion granulation method, a tumbling granulation method, a spray granulation method, and apparatuses and the like. Spray granulation method and spray granulator are preferable, and there is no particular limitation as to the drying method, so long as it can realize drying in an ordinary pharmaceutical manner.

In the drying step, both the apparatus and the means are not particularly limited, so long as it is a method in which the granulated product can be dried in an ordinary pharmaceutical manner. Examples of the apparatus include a forced-air dryer, a dryer under reduced pressure, a vacuum dryer, a fluidized bed granulation dryer, and the like.

After drying, the dried product may be sieved and sized using a sieve, a comil, or the like, if desired.

In the molding step, both the apparatus and the means are not particularly limited, so long as it is a method of molding the pharmaceutical composition for oral administration of the present invention. Examples of the method include a method in which the active ingredient and appropriate excipients are granulated and dried, and compression-molded to prepare the pharmaceutical compositions for oral administration, or a method in which the active ingredient and appropriate excipients are granulated, and further mixed with one or more extragranular excipients such as a binder, filler, glidant, lubricant, and/or acidifer and the mixture is compression-molded to prepare the pharmaceutical compositions for oral administration; and the like.

After tableting, the surfaces of the pharmaceutical compositions for oral administration may be film coated. The method of film coating is not particularly limited, so long as it can realize coating in an ordinary pharmaceutical manner. Examples of the coating include pan coating, dip coating, and the like. A film coating agent may be appropriately added alone, or as a combination of two or more, in appropriate amounts.

The coating rate is not particularly limited, so long as a film can be formed. The coating rate is, for example, with respect to the total weights of the pharmaceutical compositions for oral administration, 0.5 wt% to 10 wt%. In another embodiment, the coating results in a weight gain of between 0.5 wt% and 5 wt%, or between 2 wt% to 4 wt%.

During film coating or after film coating, the coated product may be dried. The drying method is not particularly limited, so long as it may realize drying in an ordinary pharmaceutical manner. The conditions for drying are not particularly limited, so long as they are appropriately selected in view of, for example, the stability of the pharmaceutical compositions for oral administration.

The specific implementation modes of the preparation method of the pharmaceutical compositions provided by the present invention are shown in the examples.

### Pharmaceutical uses and/or methods of treatment of OAD2 pharmaceutical compositions

The present invention further provides the use of pharmaceutical compositions of OAD2 and a pharmaceutically acceptable salt thereof in the medical field.

In some embodiments, the present invention provides the use of pharmaceutical compositions of OAD2 and a pharmaceutically acceptable salt thereof, for the preparation of a medicament for mediating GLP-1 receptor.

In some embodiments, the present invention provides the use of pharmaceutical compositions of OAD2 and a pharmaceutically acceptable salt thereof, for the preparation of a medicament for the treatment or prevention of GLP-1 receptor mediated diseases.

In some embodiments, the present invention provides the use of a pharmaceutical composition of OAD2 and a pharmaceutically acceptable salt thereof, for the preparation of a medicament for the treatment or prevention of metabolic diseases and/or disorders; wherein said metabolic diseases and/or disorders includes, but is not limited to, a disease selected from the group consisting of metabolic syndrome, glucose intolerance, hyperglycemia, dyslipidemia, type I diabetes, type II diabetes, syndrome X, insulin resistance, impaired glucose tolerance (IGT), obesity, diabetic dyslipidemia, hyperlipidemia, arteriosclerosis, atherosclerosis, other cardiovascular diseases that would benefit from activation of GLP-1 receptor, hypertension, metabolic disorders that would benefit from activation of GLP-1 receptor, and the like, and complications arising from or associated with diabetes including, but not limited to, neurological diseases, retinopathy, nephropathy, and impaired wound healing.

As a specific embodiment, the present invention provides the use of a pharmaceutical composition of OAD2 and a pharmaceutically acceptable salt thereof, for the preparation of a medicament for the treatment or prevention of type II diabetes.

The present invention also relates to a method of mediating the GLP-1 receptor comprising administering to a patient in need thereof a therapeutically effective amount of a pharmaceutical composition of OAD2 and a pharmaceutically acceptable salt thereof.

The present invention also relates to a method of treating or preventing GLP-1 receptor agonist mediated diseases comprising administering to a patient in need thereof a therapeutically effective amount of a pharmaceutical composition of OAD2 and pharmaceutically acceptable salts thereof.

The present invention is also directed to a method of treating or preventing metabolic diseases and/or disorders comprising administering to a patient in need thereof a therapeutically effective amount of a pharmaceutical composition of OAD2 and a pharmaceutically acceptable salt thereof; wherein said metabolic diseases and/or disorders include, but not limited to, a disease selected from the group consisting of metabolic syndrome, glucose intolerance, hyperglycemia, dyslipidemia, type I diabetes, type II diabetes, syndrome X, insulin resistance, Impaired glucose tolerance (IGT), obesity, diabetic dyslipidemia, hyperlipidemia, arteriosclerosis, atherosclerosis, other cardiovascular diseases, hypertension, metabolic disorders that would benefit from activation of GLP-1 receptor, and the like, as well as complications arising from or associated with diabetes including, but not limited to, neurological diseases, retinopathy, nephropathy, and impaired wound healing.

The present invention also relates to a method of treating or preventing type II diabetes comprising administering to a patient in need thereof a therapeutically effective amount of a pharmaceutical composition of OAD2 and a pharmaceutically acceptable salt thereof.

The present invention also relates to a pharmaceutical composition of OAD2 and a pharmaceutically acceptable salt thereof, which is useful as GLP-1 receptor agonists.

The present invention also relates to a pharmaceutical compositions of OAD2 and a pharmaceutically acceptable salt thereof, for use in treating or preventing GLP-1 receptor mediated diseases and/or disorders.

The present invention also relates to a pharmaceutical composition of OAD2 and a pharmaceutically acceptable salt thereof, for use in the treatment or prevention of metabolic diseases and/or disorders including, but not limited to, a disease selected from the group consisting of metabolic syndrome, glucose intolerance, hyperglycemia, dyslipidemia, type I diabetes, type II diabetes, syndrome X, insulin resistance, Impaired glucose tolerance (IGT), obesity, diabetic dyslipidemia, hyperlipidemia, arteriosclerosis, atherosclerosis, other cardiovascular diseases, hypertension, metabolic disorders that would benefit from activation of GLP-1 receptor, and the like, as well as complications arising from or associated with diabetes, including, but not limited to, neurological diseases, retinopathy, nephropathy and impaired wound healing.

The present invention also relates to a pharmaceutical composition of OAD2 and a pharmaceutically acceptable salt thereof, for use in the treatment or prevention of type II diabetes.

In another embodiment, the present invention provides a method of treating type I diabetes by administering to a human subject a therapeutically effective amount of OAD2 or a pharmaceutically acceptable salt thereof as part of a pharmaceutical composition described herein.

In another embodiment, the present invention provides a method of treating obesity by administering to a human subject a therapeutically effective amount of OAD2 or a pharmaceutically acceptable salt thereof as part of a pharmaceutical composition described herein.

In another embodiment, the present invention provides a method of slowing gastric emptying by administering to a human subject a therapeutically effective amount of OAD2 or a pharmaceutically acceptable salt thereof as part of a pharmaceutical composition described herein. In another embodiment, the present invention provides a method of lowering an HbA1c level by administering to a human subject a therapeutically effective amount of OAD2 or a pharmaceutically acceptable salt thereof as part of a pharmaceutical composition described herein. In an embodiment, the method may reduce the amount of HbA1c in a subject in need thereof by at least 0.1 %, or 0.2%, or 0.3%, or 0.4%, or 0.5%, or 0.6%, or 0.7%, or 0.8%, or 0.9%, or 1.0 %. In still other embodiments, the method of treatment may reduce the level of HbA1c in a subject in need thereof to less than 7%. In other embodiments, the level of HbA1c may be reduced to a level between 5% and 6.5%.

The dosage of the pharmaceutical compositions of OAD2 or a pharmaceutically acceptable salt thereof used in the treatment method provided by the present invention may vary depending on the progress of the disease, the severity of the disease, the basic condition of the subject, etc., and in general, the appropriate dosage of the pharmaceutical compositions of the present invention can be 0.5-1000 mg.

The OAD2 or a pharmaceutically acceptable salt thereof may be administered at a dosage such that the amount of OAD2 or a pharmaceutically acceptable salt thereof administered is between 1 mg and 1000 mg per day, or 25 mg to 200 mg per day, or 25 to 75 mg per day, or 50 to 100 mg per day, or 75 to 125 mg per day, or 100 mg to 150 mg per day, or 125 to 175 mg per day, or 150 mg to 200 mg per day, or 75 mg to 150 mg per day, or 25 mg ± 5 mg per day, or 30 mg ± 5 mg per day, or 40 ± 5 mg per day, 50 mg ± 5 mg per day, or 60 mg ± 5 mg per day, or 75 mg ± 5 mg per day, or 80 mg ± 5 mg per day, or 90 mg± 5 mg per day, or 100 mg ± 5 mg per day, or 110 mg ± 5 mg per day, or 125 mg ± 5 mg per day, or 150 mg ± 5 mg per day, or 175 mg ± 5 mg per day, or 200 mg ± 5 mg per day, or 225 mg ± 5 mg per day, or 250 mg ± 5 mg per day. In an embodiment, the OAD2 or a pharmaceutically acceptable salt thereof may be administered at a dosage such that the amount of OAD2 or a pharmaceutically acceptable salt thereof administered is 25 mg ± 2.5 mg per day, or 50 mg ± 2.5 mg per day, or 75 mg ± 2.5 mg per day, or 100 mg ± 2.5 mg per day, or 125 mg ± 2.5 mg per day, or 150 mg ± 2.5 mg per day.

The dosage may be individualized by the clinician based on the specific clinical condition of the subject being treated.

It will be understood that the specific dosage level for any particular subject will depend upon a variety of factors including the activity of the specific compound employed, age, body weight, general health condition, sex, diet, time of administration, route of administration, rate of excretion, drug combination and the severity of the particular disease undergoing therapy.

In some embodiments, the dosage of the pharmaceutical compositions of OAD2 and a pharmaceutically acceptable salt thereof of the present invention is 0.5-1000 mg per day, or 1-500 mg per day, 2-300 mg per day, 5-200 mg per day, 10-100 mg per day, 20-50mg per day, or 5-20mg per day, 25-40mg per day, 45-60mg per day, 65-80mg per day, 85-100mg per day, 105-120mg per day, 125-140mg per day, 145-160mg per day, 165-180mg per day, 185-200mg per day, or 10±2.5mg per day, 15±2.5mg per day, 20±2.5mg per day, 25±2.5mg per day, 30±2.5mg per day, 35±2.5mg per day, 40±2.5mg per day, 45±2.5mg per day, 50±2.5mg per day, 55±2.5mg per day, 60±2.5mg per day, 65±2.5mg per day, 70±2.5mg per day, 75±2.5mg per day, 80±2.5mg per day, 85±2.5mg per day, 90±2.5mg per day, 95±2.5mg per day, 100±2.5mg per day, 105±2.5mg per day, 110±2.5mg per day, 115±2.5mg per day, 120±2.5mg per day, 125± 2.5mg per day, 130±2.5mg per day, 135±2.5mg per day, 140±2.5mg per day, 145±2.5mg per day, 150±2.5mg per day, 155±2.5mg per day, 160±2.5mg per day, 165±2.5mg per day, 170±2.5mg per day, 175±2.5mg per day, 180±2.5mg per day, 185±2.5mg per day, 190±2.5mg per day, 195±2.5mg per day, 200±2.5mg per day, 205±2.5mg per day, 210±2.5mg per day, 215±2.5mg per day, 220±2.5mg per day, 225±2.5mg per day, 230±2.5mg per day, 235±2.5mg per day, 240±2.5mg per day, 245±2.5mg per day, 250± 2.5mg per day, 255±2.5mg per day, 260±2.5mg per day, 265±2.5mg per day, 270±2.5mg per day, 275±2.5mg per day, 280±2.5mg per day, 285±2.5mg per day, 290±2.5mg per day, 295±2.5mg per day, 300±2.5mg per day.

The present invention also relates to pharmaceutical compositions of OAD2 and a pharmaceutically acceptable salt thereof, in combination or association with one or more other pharmaceutically active ingredients. In an embodiment, the present invention provides the administration of OAD2 or a pharmaceutically acceptable salt thereof in combination with one or more other pharmaceutically active compounds, for example other anti-diabetic drugs. The combination therapy may include the active ingredient and the other pharmaceutically active compound within a single pharmaceutical composition as well as in two separate pharmaceutical compositions administered to the same subject simultaneously or at a time interval determined by a skilled artisan.

### Pharmaceutical compositions with low impurity content

The present invention provides compositions that may have low or minor levels of impurities. The term "impurity" refers to an undesired substance in a composition. In some embodiments, the amount of impurities may be present in the initial composition and/or may form after a certain shelf-life of the composition. In some embodiments, impurities may form through the degradation of one or more components (e.g., active ingredient) of the composition. Sources of degradation impurities include, but are not limited to, oxidation, racemization, visible light, ultraviolet light, moisture, heat, pH changes, and interaction of components of the composition.

In some embodiments, the total content of impurities in the pharmaceutical compositions provided by the present invention is not greater than 5% by weight from the time the composition is prepared to the shelf life of the composition, or the mass percentage of total impurities of the pharmaceutical compositions provided by the present invention is less than 4.8%, or less than 4.7%, or less than 4.6%, or less than 4.5%, or less than 4.4%, or less than 4.3%, or less than 4.2%, or less than 4.1%, or less than 4.0%, or less than 3.9%, or less than 3.8%, or less than 3.7%, or less than 3.6%, or less than 3.5%, or less than 3.4%, or less than 3.3%, or less than 3.2% , or less than 3.1%, or less than 3.0%, or less than 2.9%, or less than 2.8%, or less than 2.7%, or less than 2.6%, or less than 2.5%, or less than 2.4%, or less than 2.3%, or less than 2.2%, or less than 2.2%, or less than 2.1%, or less than 2.0%, or less than 1.9%, or less than 1.8%, or less than 1.7%, or less than 1.6%, or less than 1.5%, or less than 1.4%, or less than 1.3%, or less than 1.2%, or less than 1.1%, or less than 1.0%, or less than 0.9%, or less than 0.8%, or less than 0.7%, or less than 0.6%, or less than 0.5%, or less than 0.4%, or less than 0.3%, or less than 0.2%, or less than 0.1%, or less than 0.09%, or less than 0.08%, or less than 0.07%, or less than 0.06%, or less than 0.05%, or less than 0.04%, or less than 0.03%, or less than 0.02%.

In some embodiments, the composition may have no more than about 10 wt% total impurities after a certain period of shelf life. In another embodiment, the composition may have no more than about 9.9 wt%, or no more than about 9.8 wt%, or no more than about 9.6 wt%, or no more than about 9.4 wt%, or no more than about 9.2 wt%, or no more than about 9 wt%, or no more than about 8.8 wt%, or no more than about 8.6 wt%, or no more than about 8.4 wt%, or no more than about 8.2 wt%, or no more than about 8 wt%, or no more than about 7.8 wt%, or no more than about 7.6wt%, or no more than about 7.4wt%, or no more than about 7.2wt%, or no more than about 7wt%, or no more than about 6.8wt%, or no more than about 6.6wt%, or no more than about 6.4wt%, or no more than about 6.2wt%, or no more than about 6wt%, or no more than about 5.8wt%, or no more than about 5.6wt%, or no more than about 5.4wt%, or no more than about 5.2wt%, or no more than about 5wt%, or no more than about 4.8wt%, or no more than about 4.6wt%, or no more than about 4.4wt%, or no more than about 4.2wt%, or no more than about 4wt% , or no more than about 3.8 wt%, or no more than about 3.6 wt%, or no more than about 3.4 wt%, or no more than about 3.2 wt%, or no more than about 3 wt%, or no more than about 2.8 wt%, or no more than about 2.6wt%, or no more than about 2.5wt%, or no more than about 2.4wt%, or no more than about 2.3wt%, or no more than about 2.2wt%, or no more than about 2.1wt%, or no more than about 2wt%, or no more than about 1.9wt%, or no more than about 1.8wt%, or no more than about 1.7wt%, or no more than about 1.6wt%, or no more than about 1.5wt%, or no more than about 1.4wt% , or no more than about 1.3 wt%, or no more than about 1.2 wt%, or no more than about 1.1 wt%, or no more than about 1 wt%, or no more than about 0.9 wt%, or no more than about 0.8 wt%, or no more than about 0.7wt%, or no more than about 0.6wt%, or no more than about 0.5wt%, or no more than about 0.4wt%, or no more than about 0.3wt%, or no more than about 0.2wt%, or no more than about 0.1 wt%, or no more than about 0.09 wt%, or no more than about 0.08 wt%, or no more than about 0.07 wt%, or no more than about 0.06 wt%, or no more than about 0.05 wt%, or no more than about 0.04 wt%, or no more than about 0.03 wt%, or no more than about 0.02 wt%, or no more than about 0.01 wt% of total impurities.

The invention provides a technical solution and technical means for reducing the impurity content of the composition, especially for reducing the content of the degradation impurity B, through the research on the compatibility of raw auxiliary materials.

In some embodiments, the present invention predicts the potential incompatibility of the drug in the final dosage form by studying the compatibility of raw auxiliary materials in the pharmaceutical composition, and screens the selection and dosage of each auxiliary material. Among them, the researches on the compatibility of raw auxiliary materials include, but is not limited to: selection of auxiliary materials/excipients, evaluation of composition stability, identification of degradation products and research on interaction mechanisms, etc.

The researchers of the present invention have studied the chemical interactions between raw drug materials and auxiliary materials/excipients in pharmaceutical compositions, such as hydrolysis, dehydration, isomerization, elimination, cyclization, oxidation, photodegradation effects, and special reactions between auxiliary materials/excipients; the main factors affecting the above reactions include but are not limited to temperature, pH, moisture content, relative temperature, light exposure, oxygen, physical form, and particle size of auxiliary materials; the researches focus on the influences of moisture and the pH value of the microenvironment, the reactions of auxiliary materials/excipients and their impurities, the investigation of stabilizers, etc.

In some embodiments, impurities are formed during standing of the composition, e.g., affected by temperature, humidity, oxygen content, pH, light exposure, and the like.

The researchers of the present invention further analyze possible formation reasons of each impurity by determination of the growth conditions of various impurities of the active ingredient under different conditions through a forced degradation experiment of OAD2 and a pharmaceutically acceptable salt thereof. Based on the forced degradation experiment detailed below, researchers reasonably judge that impurity B is an impurity formed by oxidation or degradation of OAD2 and a pharmaceutically acceptable salt thereof, and thus judge that by reducing the content of oxides, peroxides, superoxides and other oxides or active oxygen structural components in the raw auxiliary materials, the formation or growth of degradation impurity B can be facilitated to be reduced.

The present invention further provides pharmaceutical compositions having low levels of impurity B.

In another aspect, the present invention provides a composition containing auxiliary materials/excipients with low reactive oxygen species which can effectively control the content of impurities in the composition by studying the influences of reactive oxygen species in the auxiliary materials/excipients.

In some embodiments, the concentration or amount of impurities present in the compositions may be attributable, at least in part, to degradation of components of the compositions other than the active ingredient after a certain period of shelf life. In some embodiments, the concentration or amount of impurities present in the compositions at the end of shelf life can be attributable, at least in part, to degradation of the active ingredient. In some embodiments, degradation of OAD2 or a pharmaceutically acceptable salt thereof may be the result of physical or chemical stress. Examples of stress include, but are not limited to, exposure to oxygen, ROS, HPO, pH, light, processed surfaces, and soluble trace metals.

In some embodiments, the impurities will be affected by the components of the auxiliary materials in the compositions. By optimizing the components of the auxiliary materials in the compositions, the formation of impurities or the growth rate of impurities can be suppressed to a certain extent.

In some embodiments, the components of the compositions may be present in concentrations or amounts that at least partially inhibit the formation of impurities in the compositions. In other embodiments, the compositions may comprise excipients with low levels of reactive oxygen species (ROS) such as HPO.

In some embodiments, the amount of impurity B in the compositions may be greater than zero and no more than about 5 wt% after a certain period of shelf life. In another embodiment, the amount of impurity B in the compositions may be greater than zero and no more than about 4.9 wt%, or no more than about 4.8 wt%, or no more than about 4.7 wt%, or no more than about 4.6 wt%, or no more than about 4.5wt%, or no more than about 4.4wt%, or no more than about 4.3wt%, or no more than about 4.2wt%, or no more than about 4.1wt%, or no more than about 4wt%, or no more than about 3.9wt%, or no more than about 3.8wt%, or no more than about 3.7wt%, or no more than about 3.6wt%, or no more than about 3.5wt%, or no more than about 3.4wt%, or no more than about 3.3 wt%, or no more than about 3.2wt%, or no more than about 3.1wt%, or no more than about 3wt%, or no more than about 2.9wt%, or no more than about 2.8wt%, or no more than about 2.7wt%, or no more than about 2.6 wt%, or no more than about 2.5 wt%, or no more than about 2.4 wt%, or no more than about 2.3 wt%, or no more than about 2.2 wt%, or no more than about 2.1 wt%, or no more than about 2wt%, or no more than about 1.9wt%, or no more than about 1.8wt%, or no more than about 1.7wt%, or no more than about 1.6wt%, or no more than about 1.5wt%, or no more than about 1.4 wt%, or no more than about 1.3 wt%, or no more than about 1.2 wt%, or no more than about 1.1 wt%, or no more than about 1 wt%, or no more than about 0.9 wt%, or no more than about 0.8 wt%, or no more than about 0.7 wt%, or no more than about 0.6 wt%, or no more than about 0.5 wt%, or no more than about 0.4 wt%, or no more than about 0.3 wt%, or no more than about 0.2 wt%, or no more than about 0.1 wt%, or no more than about 0.09 wt%, or no more than about 0.08 wt%, or no more than about 0.07 wt%, or no more than about 0.06 wt%, or no more than about 0.05 wt%, or no more than about 0.04 wt%, or no more than about 0.03 wt%, or no more than about 0.02 wt%, or no more than about 0.01 wt%.

In some embodiments, the content of degradation impurity B in the pharmaceutical compositions provided by the present invention is not higher than 4% by mass from the time the compositions are prepared to the shelf life, or the mass percentage of total impurities of the pharmaceutical compositions provided by the present invention is less than 4.0%, or less than 3.9%, or less than 3.8%, or less than 3.7%, or less than 3.6%, or less than 3.5%, or less than 3.4%, or less than 3.3%, or less than 3.2%, or less than 3.1%, or less than 3.0%, or less than 2.9%, or less than 2.8%, or less than 2.7%, or less than 2.6%, or less than 2.5%, or less than 2.4 %, or less than 2.3%, or less than 2.2%, or less than 2.2%, or less than 2.1%, or less than 2.0%, or less than 1.9%, or less than 1.8%, or less than 1.7%, or less than 1.6%, or less than 1.5%, or less than 1.4%, or less than 1.3%, or less than 1.2%, or less than 1.1%, or less than 1.0%, or less than 0.9%, or less than 0.8%, or less than 0.7%, or less than 0.6%, or less than 0.5%, or less than 0.4%, or less than 0.3%, or less than 0.2%, or less than 0.1%, or less than 0.09 %, or less than 0.08%, or less than 0.07%, or less than 0.06%, or less than 0.05%, or less than 0.04%, or less than 0.03%, or less than 0.02%, or less than 0.01%.

As a specific embodiment, the mass percentage of the degradation impurity B in the compositions from the time the compositions are prepared to the shelf life is kept at 0 to 1%.

In a preferred embodiment, the amount of impurity B is greater than zero and no more than about 1.0 wt%, based on the total weight of the pharmaceutical dosage form, after a certain shelf-life. In another preferred embodiment, the amount of impurity B is greater than zero and after 12 months at 25°C±2°C/60%RH±5%RH or at 30°C±2°C/65%RH±5%RH, no more than about 1.0 wt%, or after 6 months at 30°C±2°C/65%RH±5%RH, no more than about 1.0wt%, or after 6 months at 40°C±2°C/75%RH±5% RH, no more than about 1.0 wt%.

In some embodiments, the initial amount of impurity B in the composition or the amount after a certain storage period may be greater than zero and no more than about 2.5 wt%, or no more than about 2 wt%, or no more than about 1.5 wt%, or no more than about 1 wt%, or no more than about 0.5 wt%, or no more than about 0.4 wt%, or no more than about 0.3 wt%, or no more than about 0.2 wt%, or no more than about 0.1 wt%, or no more than about 0.09 wt% , or no more than about 0.08 wt%, or no more than about 0.07 wt%, or no more than about 0.06 wt%, or no more than about 0.05 wt%, or no more than about 0.04 wt%, or no more than about 0.03 wt%, or no more than about 0.02 wt%, or no more than about 0.01 wt%.

As a specific embodiment, the formation of impurity B may be affected by an oxygen-dependent mechanism, such that reducing the oxygen accessible to the composition can reduce the rate at which impurity B forms. As a specific embodiment, the content of active oxygen will affect the growth rate of impurity B.

In some embodiments, the concentration of oxidative degradation products present in the compositions is such that the compositions do not undergo physical changes after a certain period of shelf life. Examples of physical changes include, but are not limited to, color changes and formation of insoluble particle.

In some embodiments, the rate of formation and/or concentration of oxidative degradation products in the compositions may be reduced by other components of the compositions after a certain shelf life. In some embodiments, the rate of formation and/or concentration of oxidative degradation products in the compositions can be reduced by the presence of an antioxidant.

As a specific embodiment, impurity B can be used as a reference substance for OAD2 or a pharmaceutically acceptable salt thereof.

The present invention also provides a composition comprising a low level of ROS, such as HPO.

Thus, in an embodiment, any excipient or carrier material formulated with the active ingredient may comprise a low level of ROS, such as one or more HPO. In an embodiment, the HPO value may be solely a measure of the amount of H₂O₂.

In an embodiment, the combined or total HPO value of all materials in the compositions or the compositions as a whole have an HPO value that is less than 200 ppm, or less than 190 ppm, or less than 180 ppm, or less than 170 ppm, or less than 160 ppm, or less than 150 ppm, or less than 140 ppm, or less than 130 ppm, or less than 120 ppm, or less than 110 ppm, or less than 100 ppm, or less than 90 ppm, or less than 80 ppm, or less than 70 ppm, or less than 60 ppm, or less than 50 ppm, or less than 40 ppm, or less than 30 ppm, or less than 20 ppm, or less than 10 ppm, or less than 5 ppm.

In a further embodiment, the combined HPO value of all materials in the compositions or the compositions as a whole have an HPO value that is less than 20,000 nmole/g, or less than 19,000 nmole/g, or less than 18,000 nmole/g, or less than 17,000 nmole/g, or less than 16,000 nmole/g, or less than 15,000 nmole/g, or less than 12,000 nmole/g, or less than 11,000 nmole/g, or less than 10,000 nmole/g, or less than 9000 nmole/g, or less than 8000 nmole/g, or less than 7000 nmole/g, or less than 6000 nmole/g, or less than 5000 nmole/g, or less than 4000 nmole/g, or less than 3000 nmole/g, or less than 2000 nmole/g, or less than 1000 nmole/g, or less than 500 nmole/g.

In another embodiment, the combined HPO value of all materials in the compositions or the compositions as a whole have an HPO value that is less than 10.0 mEq^O2/kg, or less than 9.5 mEq^O2/kg, or less than 9.0 mEq^O2/kg, or less than 8.5 mEq^O2/kg, or less than 8.0 mEq^O2/kg, or less than 7.5 mEq^O2/kg, or less than 7.0 mEq^O2/kg, or less than 6.5 mEq^O2/kg, or less than 6.0 mEq^O2/kg, or less than 5.5 mEq^O2/kg, or less than 5.0 mEq^O2/kg, or less than 4.5 mEq^O2/kg, or less than 4.0 mEq^O2/kg, or less than 3.5 mEq^O2/kg, or less than 3.0 mEq^O2/kg, or less than 2.5 mEq^O2/kg, or less than 2.0 mEq^O2/kg, or less than 1.5 mEq^O2/kg, or less than 1.0 mEq^O2/kg, or less than 0.9 mEq^O2/kg, or less than 0.8 mEq^O2/kg, or less than 0.7 mEq^O2/kg, or less than 0.6 mEq^O2/kg, or less than 0.5 mEq^O2/kg.

In an embodiment, the composition comprises a disintegrant, wherein the disintegrant has an HPO value that is less than 50 ppm, or less than 49 ppm, or less than 48 ppm, or less than 47 ppm, or less than 46 ppm, or less than 45 ppm, or less than 44 ppm, or less than 43 ppm, or less than 42 ppm, or less than 41 ppm, or less than 40 ppm, or less than 39 ppm, or less than 38 ppm, or less than 37 ppm, or less than 36 ppm, or less than 35 ppm, or less than 34 ppm, or less than 33 ppm, or less than 32 ppm, or less than 31 ppm, or less than 30 ppm, or less than 29 ppm, or less than 28 ppm, or less than 27 ppm, or less than 26 ppm, or less than 25 ppm, or less than 24 ppm, or less than 23 ppm, or less than 22 ppm, or less than 21 ppm, or less than 20 ppm, or less than 19 ppm, or less than 18 ppm, or less than 17 ppm, or less than 16 ppm, or less than 15 ppm, or less than 14 ppm, or less than 13 ppm, or less than 12 ppm, or less than 11 ppm, or less than 10 ppm. In a preferred embodiment, the composition comprises a disintegrant, wherein the disintegrant has an HPO value that is less than 50 ppm, or less than 40 ppm, or less than 30 ppm, or less than 25 ppm, or less than 10 ppm. In a further preferred embodiment, the composition comprises a disintegrant, wherein the disintegrant is crospovidone and the disintegrant has an HPO value or an H₂O₂ value that is less than 50 ppm, or less than 40 ppm, or less than 30 ppm, or less than 25 ppm, or less than 10 ppm as measured by European Pharmacopoeia Type A method. In a further embodiment, the European Pharmacopoeia Type A method may be the method in 2012, 2013, 2014, or 2015. In a further preferred embodiment, the composition comprises a disintegrant, wherein the disintegrant is crospovidone and the disintegrant has an HPO value or an H₂O₂ value that is less than 125 ppm, or less than 100 ppm, or less than 75 ppm, or less than 50 ppm, as measured by European Pharmacopoeia Type B method. In a further embodiment, the European Pharmacopoeia Type B method may be the method in 2012, 2013, 2014, or 2015.

In an embodiment, the composition comprises a disintegrant, wherein the disintegrant has an HPO value that is less than 6000 nmole/g, or less than 5000 nmole/g, or less than 4900 nmole/g, or less than 4800 nmole/g, or less than 4700 nmole/g, or less than 4600 nmole/g, or less than 4500 nmole/g, or less than 4400 nmole/g, or less than 4300 nmole/g, or less than 4200 nmole/g, or less than 4100 nmole/g, or less than 4000 nmole/g, or less than 3900 nmole/g, or less than 3800 nmole/g, or less than 3700 nmole/g, or less than 3600 nmole/g, or less than 3500 nmole/g, or less than 3400 nmole/g, or less than 3300 nmole/g, or less than 3200 nmole/g, or less than 3100 nmole/g, or less than 3000 nmole/g, or less than 2900 nmole/g, or less than 2800 nmole/g, or less than 2700 nmole/g, or less than 2600 nmole/g, or less than 2500 nmole/g, or less than 2400 nmole/g, or less than 2300 nmole/g, or less than 2200 nmole/g, or less than 2100 nmole/g, or less than 2000 nmole/g, or less than 1900 nmole/g, or less than 1800 nmole/g, or less than 1700 nmole/g, or less than 1600 nmole/g, or less than 1500 nmole/g, or less than 1400 nmole/g, or less than 1300 nmole/g, or less than 1200 nmole/g, or less than 1100 nmole/g, or less than 1000 nmole/g. In a preferred embodiment, the composition comprises a disintegrant, wherein the disintegrant has an HPO value that is less than 5000 nmole/g or less than 4000 nmole/g. In a further preferred embodiment, the composition comprises a disintegrant, wherein the disintegrant is crospovidone and the disintegrant has an HPO value that is less than 5000 nmole/g or less than 4000 nmole/g.

In another embodiment, the composition comprises a disintegrant, wherein the disintegrant has an HPO value that is less than 2.00 mEq^O2/kg, or less than 1.90 mEq^O2/kg, or less than 1.80 mEq^O2/kg, or less than 1.70 mEq^O2/kg, or less than 1.60 mEq^O2/kg, or less than 1.50 mEq^O2/kg, or less than 1.40 mEq^O2/kg, or less than 1.30 mEq^O2/kg, or less than 1.20 mEq^O2/kg, or less than 1.10 mEq^O2/kg, or less than 1.00 mEq^O2/kg, or less than 0.90 mEq^O2/kg, or less than 0.80 mEq^O2/kg, or less than 0.70 mEq^O2/kg, or less than 0.60 mEq^O2/kg, or less than 0.50 mEq^O2/kg, or less than 0.40 mEq^O2/kg, or less than 0.30 mEq^O2/kg, or less than 0.20 mEq^O2/kg, or less than 0.10 mEq^O2/kg, or less than 0.09 mEq^O2/kg, or less than 0.08 mEq^O2/kg, or less than 0.07 mEq^O2/kg, or less than 0.06 mEq^O2/kg, or less than 0.05 mEq^O2/kg. In a preferred embodiment, the composition comprises a disintegrant, wherein the disintegrant has an HPO value that is less than 1.00 mEq^O2/kg. In a further preferred embodiment, the composition comprises a disintegrant, wherein the disintegrant is crospovidone and the disintegrant has an HPO value that is less than 1.00 mEq^O2/kg.

In any of the previous embodiments, the HPO measured may be a measure of total HPO or H₂O₂. Further, quantitation of HPO levels may be accomplished using methods known to those skilled in the art.

In an embodiment, the HPO level may be determined according to methods measuring reduction of HPO by Fe(II) under acidic conditions, such as the methods described in Gay (Gay et al. 1999. "Hydroperoxide assay with the ferric-xylenol orange complex" Anal. Biochem 273:149-155).

In another embodiment, the HPO level may be determined according to methods measuring formation of triphenylphosphine oxide from triphenylphosphine, such as the methods described in Nakamura (Nakamura et al. 1991. "A simple assay for lipid hydroperoxides based on triphenylphosphine oxidation and high-performance liquid chromatography" Lipids 26:765-768).

Additional methods to measure HPO are described in Wasylaschuk (Wasylaschuk et al. 2007. "Evaluation of hydroperoxides in common pharmaceutical excipients" J. Pharm. Sci. 96:106-116).

Additional methods to measure peroxides (H₂O₂) are described in European Pharmacopoeia monograph: Type A: maximum (no more than) 400 ppm expressed as H₂O₂; Type B: maximum (no more than) 1000 ppm expressed as H₂O₂, as of 2012, 2013, 2014, or 2015.

Each of the above methods of measuring HPO are herein incorporated by reference.

The present invention also provides compositions that may have an extended shelf life relative to compositions with elevated levels of ROS such as HPO.

As used herein, the term "shelf life" refers to the length of time a product can be stored without becoming unsuitable for medical use. Examples of compositions that are unsuitable for medical use include, but are not limited to, compositions that have unacceptably high levels of impurities and/or the presence of physical changes described herein (e.g., color changes and/or the presence of insoluble particles).

In some embodiments, the period of shelf life of the compositions may be 7 days, or 11 days, or 14 days, or 1 month, or 2 months, or 3 months, or 4 months, or 5 months, or 6 months, or 7 months, or 8 months, or 9 months, or 10 months, or 11 months, or 12 months, or 13 months, or 14 months, or 15 months, or 16 months, or 17 months, or 18 months, or 19 months, or 20 months, or 21 months, or 22 months, or 23 months, or 24 months, or 25 months, or 26 months, or 27 months, or 28 months, or 29 months, or 30 months, or 31 months, or 32 months, or 33 months, or 34 months, or 35 months, or 36 months, or 48 months.

In other embodiments, the period of shelf life of the compositions may be extended for 7 days, or 11 days, or 14 days, or 1 month, or 2 months, or 3 months, or 4 months, or 5 months, or 6 months, or 7 months, or 8 months, or 9 months, or 10 months, or 11 months, or 12 months, or 13 months, or 14 months, or 15 months, or 16 months, or 17 months, or 18 months, or 19 months, or 20 months, or 21 months, or 22 months, or 23 months, or 24 months, or 25 months, or 26 months, or 27 months, or 28 months, or 29 months, or 30 months, or 31 months, or 32 months, or 33 months, or 34 months, or 35 months, or 36 months, or 48 months, relative to same or similar compositions having elevated levels of ROS.

In some embodiments, shelf life may be determined by measuring certain characteristics of the compositions that may indicate that the compositions are unfit for medical use. In some embodiments, shelf life may be determined by measuring the concentration of impurities in the compositions after storage at 25°C and 60% relative humidity. In some embodiments, shelf life may be determined by measuring the concentration of impurities in the compositions after storage at 37°C and 65% relative humidity. In some embodiments, shelf life may be determined by measuring the concentration of impurities in the compositions after storage at 40°C and 75% relative humidity. In some embodiments, shelf life may be determined by measuring the concentration of impurities in the compositions after storage at between 50°C and 60°C or at between 55°C and 65°C.

In some embodiments, shelf life may be determined by measuring the concentration of impurities in the compositions after storage at (long term - 12 months) 25 °C ± 2 °C /60% RH ± 5% RH or at 30 °C ± 2 °C / 65% RH ± 5% RH, or (intermediate term - 6 months) at 30 °C ± 2 °C/ 65% RH ± 5% RH, or (accelerated - 6 months) at 40 °C ± 2 °C /75% RH ± 5% RH.

In some embodiments, shelf life may be determined by measuring the concentration of impurities in the compositions using the guidelines as outlined in the ICH Harmonized Tripartite Guideline: Stability Testing of New Drug Substances and Products Q1A(R2), dated Feb. 6, 2003.

For example, shelf life may be determined for long term, accelerated, and, where appropriate, intermediate storage conditions by measuring the concentration of impurities after storage in these conditions, wherein the compositions are packaged in a container closure system that is the same as or simulates the packaging proposed for storage and distribution.

### Method for identifying and controlling impurity B

As is known from the results of the active ingredient forced degradation experiments (see Examples), oxidative destruction resulted in an impurity that appeared in front of the main peak, and the impurity and the main peak had a separation degree of 0.68, which was an impurity with a poor separation degree from the main peak. Meanwhile, the lowest purity of the main peak after oxidative destruction in each sub-experiment was 91.6%, and thus it is known that OAD2 and a pharmaceutically acceptable salt thereof are firstly most sensitive to oxidative destruction and secondly sensitive to high temperature destruction, and thus further control should be performed on the generation of oxidative impurities.

From the change situation of the impurities in the forced degradation experiment, in the oxidative destruction sub-experiment, after oxidative destruction, the growth of the component of impurity B was increased to 2.77, which was the most obvious changed component in each sub-experiment, so it is necessary to optimize the analysis method of impurity B, and reduce the limit of impurity B.

Meanwhile, the technical personnel reasonably judged that impurity B is the impurity formed by oxidizing or degrading OAD2 or a pharmaceutically acceptable salt thereof through the above forced degradation experiment, so the formation or growth of the oxidative degradation impurity B can be reduced by reducing oxides, peroxides, superoxides and other oxides or active oxygen structural components contained in the raw auxiliary materials.

The present invention provides one of related substances of OAD2, i.,e., degradation impurity B, 2-3(-(4-((3,4-dichlorobenzyl)oxy)phenyl)-8-oxo-12-(1-phenylpropyl)-2,3,6,8,9, 10-hexahydro-7H-6,9-epimino[1,4]dioxino[2',3':4,5]benzo[1,2-c]azapin-7-yl)-3-(4-(2,3-dimethylpyridin-4-yl)phenyl)propionic acid, as shown in structural formula B. The molecular formula of the degradation impurity B is C₅₀H₄₅Cl₂N₃O₆, and the molecular weight is 854.82.

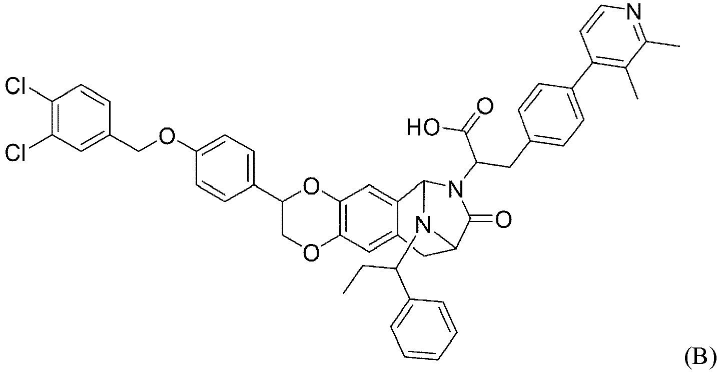

The data on detection, analysis, characterization, etc. of impurity B are described in Examples.

In some embodiments, impurity B is characterized by a relative retention time relative to OAD2 of between 0.45 and 0.66 under certain HPLC or LCMS conditions, such as those described in the sections of Examples 18-24. In one embodiment, the HPLC relative retention time of impurity B is between 0.47 and 0.63 relative to OAD2. In another embodiment, the HPLC relative retention time of impurity B is between 0.55 and 0.64 relative to OAD2.

In another embodiment, impurity B is a product produced from a method comprising the step of oxidizing OAD2 or a pharmaceutically acceptable salt thereof. The step of oxidation may comprise mixing OAD2 or a pharmaceutically acceptable salt thereof with an oxidizing agent. The oxidizing agent may be any reagent capable of removing two electrons and/or H₂ from OAD2 or a pharmaceutically acceptable salt thereof. An oxidizing agent may be molecular oxygen (O₂), hydrogen peroxide (H₂O₂), a superoxide, a hypochlorite, or an organic hydroperoxide (ROOH) where R is a carbon atom such as a C1-C6 alkyl group. In an embodiment, the oxidizing agent is molecular oxygen. In another embodiment, the oxidizing agent is hydrogen peroxide. In another embodiment, the oxidizing agent is an organic hydroperoxide such as 3-chloroperoxybenzoic acid (mCPBA).

### Additional Embodiments

Embodiment A-1: A compound produced by a process comprising the step of: oxidizing (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propionic acid (OAD2) or a pharmaceutically acceptable salt thereof, wherein the compound produced is characterized by having a molecular weight of 854.

Embodiment A-2. The compound of Embodiment A-1, wherein the compound is further characterized by a retention time between 0.45 and 0.66 relative to OAD2 under reverse phase liquid chromatography gradient mobile phase conditions wherein a mobile phase A comprises 0.05% TFA in water (v/v), a mobile phase B comprises 0.05% TFA in acetonitrile : methanol (1:2) (v/v), wherein the sample is run using a step gradient from time zero (50% mobile phase A) to 38 minutes (99% mobile phase B).

Embodiment A-3. A composition comprising: (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propionic acid (OAD2) or a pharmaceutically acceptable salt thereof, the compound according to Embodiment A-1 or a pharmaceutically acceptable salt thereof, and one or more pharmaceutically acceptable excipients, wherein the compound according to Embodiment A-1 or A-2 or a pharmaceutically acceptable salt thereof in the composition is greater than zero and less than 2.5 wt%.

Embodiment A-4. A composition comprising: (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propionic acid (OAD2) or a pharmaceutically acceptable salt thereof, and a disintegrant that has an HPO value of less than 50 ppm.

Embodiment A-5. The composition of Embodiment A-4, comprising: from 10 wt% to 40 wt% of OAD2 dihydrocholoride salt, and from 0.1 wt% to 20 wt% of the disintegrant.

Embodiment A-6. The composition of Embodiment A-5, wherein the disintegrant is crospovidone and is present ranging from 0.2 wt% to 10 wt%.

Embodiment A-7. The composition of Embodiment A-4, wherein the composition comprises the compound according to Embodiment A-1 or A-2 or a pharmaceutically acceptable salt thereof, wherein the amount of the compound according to Embodiment A-1 or A-2 or a pharmaceutically acceptable salt thereof present is greater than zero and less than 1.0 wt%.

Embodiment A-8. The composition of Embodiment A-7, wherein the composition comprises 0.4 wt% or less of the compound according to Embodiment A-1 or A-2 or a pharmaceutically acceptable salt thereof after 24 months of storage at 25 °C ± 2 °C /60% RH ± 5% RH.

Embodiment A-9. The composition of Embodiment A-7, wherein the composition comprises 1 wt% or less of the compound according to Embodiment A-1 or A-2 or a pharmaceutically acceptable salt thereof after 14 days of storage at 55 °C to 65 °C.

Embodiment A-10. The composition of Embodiment A-4, comprising from 10 wt% to 40 wt% of OAD2 dihydrocholoride salt, from 1 wt% to 5 wt% of the disintegrant, wherein the disintegrant comprises crospovidone, from 0.1 wt % to 20 wt% of a binder, from 10 wt% to 85 wt% of a filler, from 0.25 wt% to 15 wt% of a surfactant, from 0.1 wt% to 10 wt% of a lubricant, from 0.1 wt% to 10 wt% of a glidant, from 1 wt% to about 50 wt% of an acidifer; wherein the composition comprises the compound according to Embodiment A-1 or A-2 or a pharmaceutically acceptable salt thereof, wherein the amount of the compound according to Embodiment A-1 or A-2 or a pharmaceutically acceptable salt thereof present is greater than zero and less than or equal to 0.4 wt% after 24 months of storage at 25 °C ± 2 °C /60% RH ± 5% RH.

Embodiment A-11. The composition of any one of Embodiment A-3 to Embodiment A-10, in the form of a tablet or capsule.

Embodiment A-12. The composition of any one of Embodiment A-3 to Embodiment A-10, comprising between 1 to 500 mg of OAD2 or a pharmaceutically acceptable salt thereof.

Embodiment A-13. A method of preparing the composition of any one of Embodiment A-3 to Embodiment A-10, wherein the method comprises admixing the OAD2 or a pharmaceutically acceptable salt thereof with one or more pharmaceutically acceptable excipients.

Embodiment A-14. The method of Embodiment A-13, wherein the method is a spray granulation process.

Embodiment A-15. A method of treating a condition comprising administering to a human in need thereof the composition of any one of Embodiment A-3 to Embodiment A-10, wherein the condition is selected from the group consisting of metabolic syndrome, glucose intolerance, hyperglycemia, dyslipidemia, type I diabetes, type II diabetes, hypertriglyceridemia, syndrome X, insulin resistance, impaired glucose tolerance (IGT), obesity, diabetic dyslipidemia, hyperlipidemia, arteriosclerosis, atherosclerosis, other cardiovascular diseases, hypertension, and complications resulting from or associated with diabetes including, but not limited to, neuropathy, retinopathy, nephropathy, and impaired wound healing.

Embodiment A-16. The method of Embodiment A-15, wherein the condition is type II diabetes.

Embodiment A-17. The method of Embodiment A-16, wherein the amount of OAD2 or a pharmaceutically acceptable salt thereof administered is between 25 mg to 200 mg per day.

### Definition of terms

The term "active ingredient" refers to OAD2 and a pharmaceutically acceptable salt thereof.

The term "pharmaceutically acceptable salt" refers to salts of compounds prepared by reacting a free base with a suitable organic or inorganic acid or by reacting an acid with a suitable organic or inorganic base. Representative salts include the following salts: acetate, benzenesulfonate, benzoate, bicarbonate, bisulfate, bitartrate, borate, bromide, calcium edetate, camphorsulfonate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, laurate, malate, maleate, mandelate, mesylate, methylbromide, methylnitrate, methyl sulfate, monopotassium maleate, mucate, napsylate, nitrate, N-methylglucamine, oxalate, pamoate (embonate), palmitate, pantothenate, phosphate/biphosphate, polygalacturonate, potassium salt, salicylate, sodium salt, stearate, subacetate, succinate, tannate, tartrate, teoclate, p-tosylate, triethiodide, trimethylammonium salt, and valerate. When an acidic substituent, such as -COOH is present, ammonium salt, morpholinium salt, sodium salt, potassium salt, barium salt, calcium salt, and the like can be formed for use in the dosage form. When a basic group, such as amino or a basic heteroaryl radical, such as pyridyl is present, an acidic salt, such as hydrochloride, hydrobromide, phosphate, sulfate, trifluoroacetate, trichloroacetate, acetate, oxalate, maleate, pyruvate, malonate, succinate, citrate, tartrate, fumarate, mandelate, benzoate, cinnamate, methanesulfonate, ethanesulfonate, picrate, and the like can be formed. In certain embodiments, the GLP-1R agonist is a hydrochloride or a dihydrocholoride.

The term "active oxygen" refers to a class of one-electron reduction products of oxygen in the body, generated by electrons leaking out of the respiratory chain and consuming about 2% of the oxygen before they are not transferred to the terminal oxidase, including the one-electron reduction product of oxygen: superoxide anion (Oz -), the two-electron reduction product hydrogen peroxide (H₂O₂), the three-electron reduction product hydroxyl radical (· OH), and nitric oxide, among others.

The term "hydroperoxides" or HPO can be an organic hydroperoxide (ROOH), where R is carbon atom, or hydrogen peroxide (H₂O₂).

The term "reactive oxygen species" or ROS includes peroxides, hydroperoxides, HPO, superoxides, hypochlorites and/or formic acid.

The term "HPO value" means the amount of HPO. Units of an HPO value may be ppm, mEq^O2/kg, nmole/g, or units of absorbance, depending on the assay used to measure HPO.

The term "shelf life" refers to the length of time that a pharmaceutical composition can be stored without being used, provided that it remains effective. Examples of compositions which are unfit for medical use include, but are not limited to, compositions with unacceptably high impurity levels and/or the presence of physical changes described herein, such as color change and/or the presence of insoluble particles.

The term "storage" refers to the conditions under which the compositions are stored, such as temperature, light exposure, relative humidity, and other conditions under which the pharmaceutical compositions are stored. In some embodiments, unless otherwise stated, storage refers to storing the pharmaceutical compositions at a temperature of 25±5° C and a relative humidity of 50±10%.

The term "mass of the composition" or " mass of the preparation" refers to the used mass of active ingredient or other pharmaceutically acceptable auxiliary materials calculated from the weight of the tablet core without coating.

The term "excipient" includes any substance used as a vehicle for delivery of the active ingredient to a subject, and any substance added to the active ingredient, for example to improve its handling properties or to permit the resulting compositions to be formed into an orally deliverable unit dose having the desired shape and consistency. Excipients can include, by way of illustration and not by limitation, diluents, disintegrants, binders, adhesives, wetting agents, lubricants, glidants, substances added to mask or counteract a bad taste or odor, flavors, dyes, substances added to improve appearance of a dosage form, and any other substances other than the active ingredient conventionally used in the preparation of oral dosage forms. In an embodiment, excipient may be an excipient having peroxide content of less than 50 ppm, or less than 40 ppm, or less than 30 ppm, or less than 20 ppm. In a further embodiment, the peroxide content may be a measure of H₂O₂.

The term "subject" refers to any mammal such as, but not limited to, humans, horses, cows, sheep, pigs, mice, rats, dogs, cats, and primates such as chimpanzees, gorillas, and rhesus monkeys. In some embodiments, the "subject" is a human. In some such embodiments, the "subject" is a human who exhibits one or more symptoms characteristic of a disease, disorder, or condition. The term "subject" does not require one to have any particular status with respect to a hospital, clinic, or research facility (e.g., as an admitted patient, a study participant, or the like). In certain embodiments, a subject is human having an HbA1c level of greater than 6.0%, 6.5%, 7.0%, 7.5%, or 8.0%. In other embodiments, a subject is human subject in need of the administration of the GLP1R agonist. The nature of the need depends on the therapeutic goals. In some embodiments, the subject exhibits elevated levels of glycated hemoglobin in its blood, for example, elevated levels of HbA1c in its blood. In some such embodiments, administering the GLP1R agonist is carried out to reduce the subject's HbA1c levels. In some other embodiments of any of the foregoing embodiments, the subject exhibits one or more symptoms consistent with type II diabetes. In some such embodiments, administering the GLP1R agonist is carried out to treat the type II diabetes or type I diabetes (including treating one or more of the symptoms associated therewith). In some other embodiments of any of the foregoing embodiments, the subject has elevated body mass, or in some cases, obesity. In some such embodiments, administering the GLP1R agonist is carried out to reduce body mass, treat obesity (including treating one or more of the symptoms associated therewith), or delay gastric emptying. In some other embodiments, the subject exhibits one or more symptoms consistent with poor glycemic control. In such embodiments, administering the GLP1R agonist is carried out to improve glycemic control (including treating one or more of the symptoms associated therewith).

The term "administer" or "administering" means to introduce, such as to introduce to a subject a compound or composition. The term is not limited to any specific mode of delivery, but preferably refers to oral delivery. Furthermore, the administering can be carried out by various individuals, including, for example, a health-care professional (e.g., physician, nurse, etc.), a pharmacist, or the subject (i.e., self-administration).

The term "treat" or "treating" or "treatment" can refer to one or more of: delaying the progress of a disease, disorder, or condition; controlling a disease, disorder, or condition; delaying the onset of a disease, disorder, or condition; ameliorating one or more symptoms characteristic of a disease, disorder, or condition; or delaying the recurrence of a disease, disorder, or condition, or characteristic symptoms thereof, depending on the nature of the disease, disorder, or condition and its characteristic symptoms.

### Description of drawings

Figure 1 is a Mass spectrum (MS) diagram of impurity B
Figure 2 is a hydrogen nuclear magnetic resonance spectrum (¹H-NMR) diagram of impurity B
Figure 3 is a carbon nuclear magnetic resonance spectrum (¹³C-NMR) diagram of impurity B
Figure 4 is a nuclear magnetic resonance DEPT 135° spectrum diagram of impurity B
Figure 5 is a nuclear magnetic resonance carbon-hydrogen correlation spectrum (HSQC) diagram of impurity B
Figure 6 is a nuclear magnetic resonance carbon-hydrogen remote correlation spectrum (HMBC) diagram of impurity B
Figure 7 is a nuclear magnetic resonance hydrogen-hydrogen correlation spectrum (¹H-¹H COSY) diagram of impurity B

### Examples

The present invention will be described in further detail with reference to specific examples. The following examples are provided for understanding the method and core idea of the present invention, and any possible changes or substitutions are within the protection scope of the present invention without departing from the conception of the present invention. The experimental methods without specifying the specific conditions in the examples of the present invention are usually conventional conditions, or according to the conditions suggested by the raw material or commodity manufacturer; the reagents without specifying the source are usually commercially available conventional reagents.

The dihydrochloride salt of OAD2 may be obtained according to the methods described in patent CN102378574A or related international publication WO 2010/114824, which is hereby incorporated by reference in its entirety.

Free state OAD2 can be obtained according to the method described in patent CN102378574A or related international publication WO 2010/114824, which is hereby incorporated by reference in its entirety.

### Example 1

Vitamin E TPGS 1000 was melted at 50 °C, and the melted vitamin E TPGS 1000, tween, poloxamer 188 and polyvinylpyrrolidone were respectively added into purified water according to their formulation amounts. The API was added to the above solution and stirred such that it is dissolved completely. Formulated amounts of microcrystalline cellulose, pregelatinized starch and crospovidone were transferred into a fluidized bed, spray granulation was performed. The prepared granules were sieved with a 40-mesh sieve for size stabilization, mixed with formulated amounts of microcrystalline cellulose, crospovidone, colloidal silicon dioxide, magnesium stearate and anhydrous citric acid, and tableted, wherein the hardness of plain tablets was controlled to be 10-14 kg.

**Table 1- Unit dose formulation composition of the composition of Example 1**

| Compositon | | Dosage (mg/tablet) |
|---|---|---|
| Granulating | OAD2 dihydrochloride (calculated as C₅₀H₄₇Cl₂N₃O₆) | 75.00 |
| | Polyvinylpyrrolidone | 6.20 |
| | Vitamin E TPGS 1000 | 9.92 |
| | Tween 80 | 18.60 |
| | Poloxamer 188 | 6.20 |
| | Microcrystalline cellulose | 148.80 |
| | Pregelatinized starch | 124.00 |
| | Copovidone | 62.00 |
| Mixing of | Microcrystalline cellulose | 43.40 |
| | Copovidone | 44.68 |
| | Colloidal silica | 3.10 |
| | Magnesium stearate | 3.10 |
| | Anhydrous citric acid | 75.00 |

### Example 2

Vitamin E TPGS 1000 was melted at 50 °C, and the melted vitamin E TPGS 1000, tween, poloxamer 188 and polyvinylpyrrolidone were respectively added into purified water according to their formulation amounts. The API was added to the above solution and stirred such that it is dissolved completely. Formulated amounts of microcrystalline cellulose, lactose and the low-substituted hydroxypropyl cellulose were transferred into a fluidized bed, spray granulation was performed. The prepared granules were sieved with a 40-mesh sieve for size stabilization, mixed with formulated amounts ofmicrocrystalline cellulose, low-substituted hydroxypropyl cellulose, colloidal silicon dioxide, magnesium stearate and anhydrous citric acid, and tableted, wherein the hardness of the tablets was controlled to be 10-14 kg.

**Table 2- Unit dose formulation composition of the composition of Example 2**

| Composition | | Dosage (mg/tablet) |
|---|---|---|
| Granulating | OAD2 dihydrochloride (calculated as C₅₀H₄₇Cl₂N₃O₆) | 75.00 |
| | Polyvinylpyrrolidone | 6.20 |
| | Vitamin E TPGS 1000 | 9.92 |
| | Tween 80 | 18.60 |
| | Poloxamer 188 | 6.20 |
| | Microcrystalline cellulose | 148.80 |
| | Lactose | 124.00 |
| | Low-substituted hydroxypropyl cellulose | 62.00 |
| Mixing of | Microcrystalline cellulose | 43.40 |
| | Low-substituted hydroxypropyl cellulose | 38.00 |
| | Colloidal silica | 3.10 |
| | Magnesium stearate | 3.10 |
| | Anhydrous citric acid | 75.00 |

### Example 3

Vitamin E TPGS 1000 was melted at 50 °C, and the molten vitamin E TPGS 1000, HS15, poloxamer 188 and polyvinylpyrrolidone were respectively added into purified water according to their formulation amounts. The API was added to the above solution and stirred such that it is dissolved completely. Formulated amounts of microcrystalline cellulose, lactose and low-substituted hydroxypropyl cellulose were transferred into a fluidized bed, spray granulation was performed. The prepared granules were sieved with a 40-mesh sieve for size stabilization, mixed with formulated amounts of microcrystalline cellulose, low-substituted hydroxypropyl cellulose, colloidal silicon dioxide, magnesium stearate and anhydrous citric acid, and tableted, wherein the hardness of the tablets was controlled to be 10-14 kg.

**Table 3- Unit dose formulation composition of the composition of Example 3**

| Composition | | Dosage (mg/tablet) |
|---|---|---|
| Granulating | OAD2 dihydrochloride (calculated as C₅₀H₄₇Cl₂N₃O₆) | 75.00 |
| | Polyvinylpyrrolidone | 6.20 |
| | Vitamin E TPGS 1000 | 9.92 |
| | HS15 | 18.60 |
| | Poloxamer 188 | 6.20 |
| | Microcrystalline cellulose | 148.80 |
| | Lactose | 124.00 |
| | Low-substituted hydroxypropyl cellulose | 62.00 |
| Mixing of | Microcrystalline cellulose | 43.40 |
| | Low-substituted hydroxypropyl cellulose | 38.00 |
| | Colloidal silica | 3.10 |
| | Magnesium stearate | 3.10 |
| | Anhydrous citric acid | 75.00 |

### Example 4

Formulated amounts of SoluPlus and polyvinylpyrrolidone were added to purified water separately. The API was added to the above solution and stirred such that it is dissolved completely. Formulated amounts of microcrystalline cellulose, lactose and low-substituted hydroxypropyl cellulose were transferred into a fluidized bed, spray granulation was performed. The prepared granules were sieved with a 40-mesh sieve for size stabilization, mixed with formulated amounts of microcrystalline cellulose, low-substituted hydroxypropyl cellulose, colloidal silicon dioxide, magnesium stearate and anhydrous citric acid, and tableted, wherein the hardness of the tablets was controlled to be 10-14 kg.

**Table 4- Unit dose formulation composition of the composition of Example 4**

| Composition | | Dosage (mg/tablet) |
|---|---|---|
| Granulating | OAD2 dihydrochloride (calculated as C₅₀H₄₇Cl₂N₃O₆) | 75.00 |
| | Polyvinylpyrrolidone | 6.20 |
| | SoluPlus | 6.20 |
| | Microcrystalline cellulose | 148.80 |
| | Lactose | 124.00 |
| | Low-substituted hydroxypropyl cellulose | 62.00 |
| Mixing of | Microcrystalline cellulose | 43.40 |
| | Low-substituted hydroxypropyl cellulose | 38.00 |
| | Colloidal silica | 3.10 |
| | Magnesium stearate | 3.10 |
| | Anhydrous citric acid | 75.00 |

### Example 5

Formulated amounts of hydroxypropyl β cyclodextrin, poloxamer 188 and polyvinylpyrrolidone were added to purified water separately. The API was added to the above solution and stirred such that it is dissolved completely. Formulated amounts of microcrystalline cellulose, lactose and low-substituted hydroxypropyl cellulose were transferred into a fluidized bed, spray granulation was performed. The prepared granules were sieved with a 40-mesh sieve for size stabilization, mixed with formulated amounts of microcrystalline cellulose, low-substituted hydroxypropyl cellulose, colloidal silicon dioxide, magnesium stearate and anhydrous citric acid, and tableted, wherein the hardness of the tablets was controlled to be 10-14 kg.

**Table 5- Unit dose formulation composition of the composition of Example 5**

| Compolsition | | Dosage (mg/tablet) |
|---|---|---|
| Granulating | OAD2 dihydrochloride (calculated as C₅₀H₄₇Cl₂N₃O₆) | 75.00 |
| | Polyvinylpyrrolidone | 6.20 |
| | Hydroxypropyl β cyclodextrin | 18.60 |
| | Poloxamer 188 | 6.20 |
| | Microcrystalline cellulose | 148.80 |
| | Lactose | 124.00 |
| | Low-substituted hydroxypropyl cellulose | 62.00 |
| Mixing of | Microcrystalline cellulose | 43.40 |
| | Low-substituted hydroxypropyl cellulose | 38.00 |
| | Colloidal silica | 3.10 |
| | Magnesium stearate | 3.10 |
| | Anhydrous citric acid | 75.00 |

### Example 6

Vitamin E TPGS 1000 was melted at 50 °C, and the molten vitamin E TPGS 1000, HS15, poloxamer 188 and polyvinylpyrrolidone were respectively added into purified water according to their formulation amounts. The API was added to the above solution and stirred such that it is dissolved completely. Formulated amounts of microcrystalline cellulose, pregelatinized starch and crospovidone were transferred into a fluidized bed, spray granulation was performed. The prepared granules were sieved with a 40-mesh sieve for size stabilization, mixed with formulated amounts of microcrystalline cellulose, crospovidone, colloidal silicon dioxide, magnesium stearate and anhydrous citric acid, and tableted, wherein the hardness of plain tablets was controlled to be 10-14 kg.

**Table 6- Unit dose formulation composition of the composition of Example 6**

| Composition | | Dosage (mg/tablet) |
|---|---|---|
| Granulating | OAD2 dihydrochloride (calculated as C₅₀H₄₇Cl₂N₃O₆) | 75.00 |
| | Polyvinylpyrrolidone | 6.20 |
| | Vitamin E TPGS 1000 | 9.92 |
| | HS15 | 18.60 |
| | Poloxamer 188 | 6.20 |
| | Microcrystalline cellulose | 148.80 |
| | Pregelatinized starch | 124.00 |
| | Copovidone | 62.00 |
| Mixing of | Microcrystalline cellulose | 43.40 |
| | Copovidone | 44.68 |
| | Colloidal silica | 3.10 |
| | Magnesium stearate | 3.10 |
| | Anhydrous citric acid | 75.00 |

### Example 7

Vitamin E TPGS 1000 was melted at 50 °C, and the molten vitamin E TPGS 1000, RH40, poloxamer 188 and polyvinylpyrrolidone were respectively added into purified water according to their formulation amounts. The API was added to the above solution and stirred such that it is dissolved completely. Formulated amounts of microcrystalline cellulose, pregelatinized starch and crospovidone were transferred into a fluidized bed, spray granulation was performed. The prepared granules were sieved with a 40-mesh sieve for size stabilization, mixed with formulated amounts of microcrystalline cellulose, crospovidone, colloidal silicon dioxide, magnesium stearate and anhydrous citric acid, and tableted, wherein the hardness of plain tablets was controlled to be 10-14 kg.

**Table 7- Unit dose formulation composition of the composition of Example 7**

| Composition | | Dosage (mg/tablet) |
|---|---|---|
| Granulating | OAD2 dihydrochloride (calculated as C₅₀H₄₇Cl₂N₃O₆) | 75.00 |
| | Polyvinylpyrrolidone | 6.20 |
| | Vitamin E TPGS 1000 | 9.92 |
| | RH40 | 18.60 |
| | Poloxamer 188 | 6.20 |
| | Microcrystalline cellulose | 148.80 |
| | Pregelatinized starch | 124.00 |
| | Copovidone | 62.00 |
| Mixing of | Microcrystalline cellulose | 43.40 |
| | Copovidone | 44.68 |
| | Colloidal silica | 3.10 |
| | Magnesium stearate | 3.10 |
| | Anhydrous citric acid | 75.00 |

### Example 8

Formulated amounts of poloxamer 188 and polyvinylpyrrolidone were added to purified water separately. The API was added to the above solution and stirred such that it is dissolved completely. Formulated amounts of microcrystalline cellulose, lactose and low-substituted hydroxypropyl cellulose were transferred into a fluidized bed, spray granulation was performed. The prepared granules were sieved with a 40-mesh sieve for size stabilization, mixed with formulated amounts of microcrystalline cellulose, low-substituted hydroxypropyl cellulose, colloidal silicon dioxide, magnesium stearate and anhydrous citric acid, and tableted, wherein the hardness of the tablets was controlled to be 10-14 kg.

**Table 8- Unit dose formulation composition of the composition of Example 8**

| Composition | | Dosage (mg/tablet) |
|---|---|---|
| Granulating | OAD2 dihydrochloride (calculated as C₅₀H₄₇Cl₂N₃O₆) | 75.00 |
| | Polyvinylpyrrolidone | 6.20 |
| | Poloxamer 188 | 49.62 |
| | Microcrystalline cellulose | 148.80 |
| | Lactose | 124.00 |
| | Low-substituted hydroxypropyl cellulose | 62.00 |
| Mixing of | Microcrystalline cellulose | 43.40 |
| | Low-substituted hydroxypropyl cellulose | 38.00 |
| | Colloidal silica | 3.10 |
| | Magnesium stearate | 3.10 |
| | Anhydrous citric acid | 75.00 |

### Example 9

Formulated amounts of SoluPlus, poloxamer 188, and polyvinylpyrrolidone were added to purified water, respectively. The API was added to the above solution and stirred such that it is dissolved completely. Formulated amounts of microcrystalline cellulose, lactose and low-substituted hydroxypropyl cellulose were transferred into a fluidized bed, spray granulation was performed. The prepared granules were sieved with a 40-mesh sieve for size stabilization, mixed with formulated amounts of microcrystalline cellulose, low-substituted hydroxypropyl cellulose, colloidal silicon dioxide, magnesium stearate and anhydrous citric acid, and tableted, wherein the hardness of the tablets was controlled to be 10-14 kg.

**Table 9- Unit dose formulation composition of the composition of Example 9**

| Composition | | Dosage (mg/tablet) |
|---|---|---|
| Granulating | OAD2 dihydrochloride (calculated as C₅₀H₄₇Cl₂N₃O₆) | 75.00 |
| | Polyvinylpyrrolidone | 6.20 |
| | SoluPlus | 6.20 |
| | Poloxamer 188 | 6.20 |
| | Microcrystalline cellulose | 148.80 |
| | Lactose | 124.00 |
| | Low-substituted hydroxypropyl cellulose | 62.00 |
| Mixing of | Microcrystalline cellulose | 43.40 |
| | Low-substituted hydroxypropyl cellulose | 38.00 |
| | Colloidal silica | 3.10 |
| | Magnesium stearate | 3.10 |
| | Anhydrous citric acid | 75.00 |

### Example 10

Vitamin E TPGS 1000 was melted at 50 °C, and the melted vitamin E TPGS 1000, tween, poloxamer 188 and polyvinylpyrrolidone were respectively added into purified water according to their formulation amounts. The API was added to the above solution and stirred such that it is dissolved completely. Formulated amounts of microcrystalline cellulose, lactose and crospovidone were transferred into a fluidized bed, spray granulation was performed. The prepared granules were sieved with a 40-mesh sieve for size stabilization, mixed with formulated amounts of microcrystalline cellulose, crospovidone, colloidal silicon dioxide, magnesium stearate and anhydrous citric acid, and tableted, wherein the hardness of plain tablets was controlled to be 10-14 kg.

**Table 10- Unit dose formulation composition of the composition of Example 10**

| Composition | | Dosage (mg/tablet) |
|---|---|---|
| Granulating | OAD2 dihydrochloride (calculated as C₅₀H₄₇Cl₂N₃O₆) | 75.00 |
| | Polyvinylpyrrolidone | 6.20 |
| | Vitamin E TPGS 1000 | 9.92 |
| | Tween 80 | 18.60 |
| | Poloxamer 188 | 6.20 |
| | Microcrystalline cellulose | 148.80 |
| | Lactose | 124.00 |
| | Copovidone | 62.00 |
| Mixing of | Microcrystalline cellulose | 43.40 |
| | Copovidone | 44.68 |
| | Colloidal silica | 3.10 |
| | Magnesium stearate | 3.10 |
| | Anhydrous citric acid | 75.00 |

### Example 11

Vitamin E TPGS 1000 was melted at 50 °C, and the melted vitamin E TPGS 1000, tween, poloxamer 188 and polyvinylpyrrolidone were respectively added into purified water according to their formulation amounts. The API was added to the above solution and stirred such that it is dissolved completely. Formulated amounts of microcrystalline cellulose, lactose and sodium carboxymethylcellulose were transferred into a fluidized bed, spray granulation was performed. The prepared granules were sieved with a 40-mesh sieve for size stabilization, mixed with formulated amounts of microcrystalline cellulose, sodium carboxymethylcellulose, colloidal silicon dioxide, magnesium stearate and anhydrous citric acid, and tableted, wherein the hardness of the tablets was controlled to be 10-14 kg.

**Table 11- Unit dose formulation composition of the composition of Example 11**

| Composition | | Dosage (mg/tablet) |
|---|---|---|
| Granulating | OAD2 dihydrochloride (calculated as C₅₀H₄₇Cl₂N₃O₆) | 75.00 |
| | Polyvinylpyrrolidone | 6.20 |
| | Vitamin E TPGS 1000 | 9.92 |
| | Tween 80 | 18.60 |
| | Poloxamer 188 | 6.20 |
| | Microcrystalline cellulose | 148.80 |
| | Lactose | 124.00 |
| | Sodium carboxymethyl cellulose | 62.00 |
| Mixing of | Microcrystalline cellulose | 43.40 |
| | Sodium carboxymethyl cellulose | 44.68 |
| | Colloidal silica | 3.10 |
| | Magnesium stearate | 3.10 |
| | Anhydrous citric acid | 75.00 |

### Example 12

### Investigation of stability of compositions of OAD2 or a pharmaceutically acceptable salt thereof (preliminary screening)

The compositions were prepared according to the formulations and methods described in Examples 1 to 11, and the prepared compositions were placed under the conditions of a temperature of 25 °C and a conventional humidity, and the total mass change of each tablet at the beginning, 1 month, 2 months and 3 months was recorded, respectively, and the result records are shown in Table 12.1.

**Table 12.1- Three-month stability experiment of pharmaceutical compositions of OAD2**

| Situations of amounts of total impurities at 25°C (%) | | | | |
|---|---|---|---|---|
| | 0 month | 1 month | 2 months | 3 months |
| Example 1 | 1.83 | 2.23 | 2.33 | 2.87 |
| Example 2 | 1.84 | 1.83 | 1.99 | 1.97 |
| Example 3 | 1.49 | 1.57 | 1.79 | / |
| Example 4 | 1.82 | 1.52 | 1.73 | 1.79 |
| Example 5 | 1.53 | 1.64 | 1.69 | 1.68 |
| Example 6 | 1.87 | 2.16 | 2.25 | 2.65 |
| Example 7 | 1.83 | 2.08 | 2.18 | / |
| Example 8 | 1.47 | 1.55 | 1.59 | 1.72 |
| Example 9 | 1.52 | 1.47 | 1.67 | 1.8 |
| Example 10 | 1.82 | 1.90 | 2.08 | / |
| Example 11 | 1.80 | 2.01 | 2.11 | / |

The researchers found that impurity B was the only shelf life impurity among all related substances, namely, the content of impurity B was gradually increased along with the prolonging of the storage time. Therefore, the researchers independently analyzed the increase situation of impurity B. Similarly, compositions were prepared according to the formulations and methods described in Examples 1 to 11, the prepared compositions were placed under the conditions of a temperature of 25 °C and a conventional humidity, and the content of impurity B in the pharmaceutical compositions at the beginning, 1 month, 2 months and 3 months was recorded, respectively, and the result records are shown in Table 12.2.

**Table 12.2-Experiment on the growth situations of impurity B in the pharmaceutical compositions of OAD2**

| Situations of amounts of impurity B at 25°C (%) | | | | |
|---|---|---|---|---|
| | 0 month | 1 month | 2 months | 3 months |
| Example 1 | 0.13 | 0.42 | 0.58 | 0.78 |
| Example 2 | 0.10 | 0.17 | 0.14 | 0.13 |
| Example 3 | 0.09 | 0.13 | 0.18 | / |
| Example 4 | 0.10 | 0.11 | 0.13 | 0.15 |
| Example 5 | 0.08 | 0.11 | 0.11 | 0.14 |
| Example 6 | 0.17 | 0.4 | 0.46 | 0.61 |
| Example 7 | 0.15 | 0.37 | 0.43 | / |
| Example 8 | 0.08 | 0.09 | 0.08 | 0.13 |
| Example 9 | 0.09 | 0.11 | 0.13 | 0.15 |
| Example 10 | 0.13 | 0.24 | 0.37 | / |
| Example 11 | 0.12 | 0.25 | 0.37 | / |

### Example 13

### Forced degradation experiment of active ingredient

Preparation of solutions:
(1) undestructive test article solution: 25mg of OAD2 or a pharmaceutically acceptable salt thereof was placed into a 50ml measuring flask. Diluent was added to dissolve it and the mixture was diluted to the scale mark, shaken well and used as a test article solution.
(2) test article solution destructed by acid: 25.35mg of OAD2 or a pharmaceutically acceptable salt thereof was placed into a 50ml measuring flask. 2ml of 1mol/L hydrochloric acid was added, and the mixture was mixed, stood at room temperature for 24h, adjusted pH to neutral with 1mol/L sodium hydroxide solution. Diluent was added to dilute the mixture to the scale mark. The mixture was shaken well, and used as the test article solution destructed by acid.
(3) test article solution destructed by alkali: 26.64mg of OAD2 or a pharmaceutically acceptable salt thereof was placed into a 50ml measuring flask. 2ml of 1mol/L sodium hydroxide solution was added, and the mixture was mixed, stood at room temperature for 24h, adjusted pH to neutral with 1mol/L hydrochloric acid solution. Diluent was added to dilute the mixture to scale mark. The mixture was shaken well, and used as test article solution destructed by alkali.
(4) acid-base blank: 2ml of 1mol/L sodium hydroxide solution was placed into a 50ml measuring flask. The solution was neutralized with 1mol/L hydrochloric acid solution, then diluted to the scale mark with a diluent, and shaken well to serve as the acid-base destruction blank solution.
(5) test article solution destructed by oxidation: 28.10mg of OAD2 or a pharmaceutically acceptable salt thereof was placed into a 50ml measuring flask. 2ml of 15% hydrogen peroxide was added, stood at room temperature for 9h. The mixture was diluted to the scale mark with a diluent. The mixture was shaken well, and used as the test article solution destructed by oxidation.
(6) oxidative destruction blank: 2ml of 15% hydrogen peroxide was placed into a 50ml measuring flask, diluted to the scale mark with a diluent, shaken well, and used as the oxidative destruction blank.
(7) test article solution destructed by high temperature: 26.07mg of OAD2 or a pharmaceutically acceptable salt thereof was accurately weighted, placed into a 50ml measuring flask, stood at a high temperature of 105 °C for 72h. It was dissolved and diluted to the scale mark with a diluent, shaken well, and used as the test article solution destructed by high temperature.
(8) test article solution destructed by light exposure: 25.12mg of OAD2 or pharmaceutically acceptable salt thereof was accurately weighted, placed into a 50ml measuring flask, stood under irradiation of light 4500Lx + -500 Lx for 6 days. It was dissolved and diluted to the scale mark with a diluent, shaken well, and used as the test article solution destructed by light exposure.
(9) test article solution destructed by high-humidity destruction: 25.71mg of OAD2 or a pharmaceutically acceptable salt thereof was placed into a 50ml measuring flask, then the measuring flask was placed into a drier with KNO₃ saturated solution at the bottom (humidity was 92.5%), stood for 5 days. It was dissolved and diluted to the scale mark with a diluent, shaken well, and used as the test article solution destructed by high-humidity destruction.
(10) blank solution that is not destructed, not destructed by high-temperature, not destructed by high-humidity and not destructed by light exposure: a diluent.

The measuring method: 2 µl of each of the above solutions (1)-(10) was injected into a liquid chromatograph, and the chromatograms were recorded. Among them, the results of the forced degradation experiment are shown in Table 13.1, and the results of the changes of various impurity components after the forced degradation are shown in Table 13.2.

**Table 13.1-The results of the forced degradation experiment**

| Destructive condition | Number of impurites (≥0.05%) | Purity of main peak (%) | Number of theoretic al plates | Degree of separation of major peak from former impurity | Degree of separation of major peak from latter impurity | Peak purity |
|---|---|---|---|---|---|---|
| No destruction | 3 | 99.4 | 61969 | 6.73 | 2.85 | 999.967 |
| Acid destruction | 4 | 99.4 | 62591 | 7.20 | 2.84 | 999.966 |
| Alkali destruction | 4 | 99.3 | 60654 | 6.69 | 2.82 | 999.965 |
| Oxidative destruction | 18 | 91.8 | 64374 | 0.68 | 2.89 | 999.964 |
| High temperature destruction | 25 | 92.2 | 67767 | 1.50 | 2.93 | 999.988 |
| Light exposure destruction | 24 | 93.1 | 58084 | 1.73 | 2.21 | 999.907 |
| High humidity destruction | 3 | 99.3 | 52165 | 5.49 | 3.11 | 999.987 |

**Table 13.2-Impurity change situation of forced degradation**

| Destructive condition | No destruction | Acid destruction | Alkali destruction | Oxidative destruction | High temperature destruction | Light expostre destruction | High humidity destruction |
|---|---|---|---|---|---|---|---|
| Impurit y A | 0.12 | 0.13 | 0.13 | 0.36 | 1.76 | 1.13 | 0.12 |
| Impurit y B | Undetect ed | Undetect ed | 0.02 | 2.77 | 0.97 | 0.26 | Undetect ed |
| Impuriti es C+D | 0.05 | 0.03 | 0.05 | 0.18 | 0.08 | 0.01 | 0.04 |
| Impurit y E | Undetect ed | Undetect ed | Undetect ed | Undetect ed | 0.08 | 0.12 | 0.01 |
| Impurit y F | 0.31 | 0.34 | 0.36 | 0.26 | 0.26 | 0.22 | 0.31 |
| Impurit y G | 0.03 | 0.05 | 0.07 | 0.20 | 0.09 | 0.03 | 0.04 |
| Impurit y H | Undetect ed | Undetect ed | Undetect ed | Undetect ed | 0.02 | 0.38 | Undetect ed |
| Impurit y I | Undetect ed | Undetect ed | Undetect ed | 0.02 | Undetect ed | Undetect ed | Undetect ed |
| Largest unknow n impurit y | 0.07 | 0.07 | 0.07 | 2.56 | 2.36 | 0.78 | 0.08 |
| | RRT=0.8 4 | RRT=0.8 3 | RRT=0.8 3 | RRT=0.9 6 | RRT=0.5 4 | RRT=0.8 5 | RRT=0.8 1 |
| Total Impuriti es | 0.60 | 0.65 | 0.73 | 8.2 | 7.8 | 6.9 | 0.68 |

From the results of the forced degradation experiments, it was found that oxidative destruction resulted in an impurity that appeared in front of the main peak, and the impurity and the main peak had a separation degree of 0.68, which was an impurity with a poor separation degree from the main peak. Meanwhile, the lowest purity of the main peak after oxidative destruction in each sub-experiment was 91.6%, and thus it is known that OAD2 or a pharmaceutically acceptable salt thereof are firstly most sensitive to oxidative destruction and secondly sensitive to high temperature destruction, and thus further control should be performed on impurities generated from oxidation and destruction.

From the change situation of the impurities in the forced degradation experiment, in the oxidative destruction sub-experiment, after oxidative destruction, the growth of the component of impurity B was increased to 2.77, which was the most obvious changed component in each sub-experiment, so it is necessary to optimize the analysis method of impurity B, and reduce the limit of impurity B.

Meanwhile, the technical personnel reasonably judged that impurity B is the impurity formed by oxidizing or degrading OAD2 or a pharmaceutically acceptable salt thereof through the forced degradation experiment, so the formation or growth of the oxidative degradation impurity B can be reduced by reducing oxides, peroxides, superoxides and other oxides or active oxygen structural components contained in the raw auxiliary materials. As an improvement to the pharmaceutical formulation of OAD2 or a pharmaceutically acceptable salt thereof, the researchers found that solubilizers and disintegrants with low content of peroxides could reduce the formation of oxidative degradation impurity B.

### Example 14

### Characterization of the oxidative degradation impurity B

1. MS:
   instrument model: Agilent Q-TOF-6545A;
   testing conditions: ESI source;
   molecular formula: C₅₀H₄₅Cl₂N₃O₆
   theoretical molecular weight: 854.2758 ([M + H]⁺)
   found by high resolution mass spectrometry: M/Z =854.2771
2. NMR:
   instrument model: Bruker AVANCE III HD 500 MHz;
   measuring method: a certain amount of oxidative degradation impurity B (Batch No.: RD 181112) was weighed, dissolved in DMSO-d6, and ¹H-NMR, ¹³C-NMR, DEPT135 °, HSQC, HMBC, ¹H-¹H COSY were measured.

Table 14.1 recites the ¹H-NMR and ¹H-¹H COSY spectra data of impurity B, and Table 14.2 recites the ¹³C-NMR, DEPT135 °, HSQC and HMBC spectra data of impurity B.

**Table 14.1 ¹H-NMR and ¹H-¹H COSY spectra data and attribution of impurity B**

| Chemical shift *δ*H (ppm) | Multiplicity *J* (Hz) | Number of protons | Attribution (H No.) | Related H shown by ¹H-¹H COSY | |
|---|---|---|---|---|---|
| | | | | *δ*H (ppm) | Attribution H |
| 8.250 | d (5.0) | 1 | H-3 8 | 6.957 | H-39 |
| 7.707 | d (1.4) | 1 | H-6 | 7.445-7.422 | H-4 |
| 7.638 | d (8.3) | 1 | H-3 | 7.445-7.422 | H-4 |
| 7.445-7.422 | m | 1 | H-4 | 7.707, 7.638 | H-6, H-3 |
| 7.410 | d (9.0) | 2 | H-10/12 | 7.042 | H-9/13 |
| 7.310 | t (7.3) | 2 | H-47/49 | 7.253, 7.173 | H-48, H-46/50 |
| 7.253 | t (7.2) | 1 | H-48 | 7.310 | H-47/49 |
| 7.173 | d (7.2) | 2 | H-46/50 | 7.310 | H-47/49 |
| 7.129-7.063 | overlap | 2 | H-30/34 | \ | \ |
| | | 2 | H-31/33 | \ | \ |
| 7.042 | d (8.7) | 2 | H-9/13 | 7.410 | H-10/12 |
| 6.957 | d (5.0) | 1 | H-39 | 8.250 | H-38 |
| 6.933 | s | 1 | H-18 | \ | \ |
| 6.508 | s | 1 | H-21 | \ | \ |
| 5.467 | s | 1 | H-24 | \ | \ |
| 5.121 | s | 2 | H-7 | \ | \ |
| 5.112-5.083 | m | 1 | H-14 | 3.971-3.897 | H-15b |
| 4.842 | dd (10.3, 5.4) | 1 | H-26 | 3.303, 3.076-2.993 | H-27 |
| 4.325 | d (9.8) | 1 | H-15a | 3.971-3.897 | H-15b |
| 3.971-3.897 | m | 1 | H-15b | 5.112-5.083, 4.325 | H-14, H-15a |
| 3.417 | d (7.0) | 1 | H-42 | 1.629-1.532 | H-43b |
| 3.303 | dd (14.7, 4.6) | 1 | H-27a | 4.842, 3.076-2.993 | H-26, H-27b |
| 3.111 | d (5.1) | 1 | H-23 | 2.740 | H-22a |
| 3.076-2.993 | m | 1 | H-27b | 4.842, 3.303 | H-26, H-27a |
| 2.740 | dd (17.1, 5.9) | 1 | H-22a | 3.111 | H-23 |
| 2.475 | s | 3 | H-40 | \ | \ |
| 2.424 | d (17.1) | 1 | H-22b | 2.740 | H-22a |
| 2.110 | s | 3 | H-41 | \ | \ |
| 1.953-1.864 | m | 1 | H-43a | 1.629-1.532, 0.531 | H-43b, H-44 |
| 1.629-1.532 | m | 1 | H-43b | 3.417, 1.953-1.864, 0.531 | H-4H-42, H-43a, H-44 |
| 0.531 | t (7.2) | 3 | H-44 | 1.953-1.864, 1.629-1.532 | H-43 |

**Table 14.2- ¹³C-NMR, DEPT135 °, HSQC, HMBC spectra data and attribution of impurity B**

| Chemical shift δC (ppm) | DEPT 135° | Number of C | Attribution (C No. in structural formula) | HSQC Attribution H | Key HMBC (HC) | |
|---|---|---|---|---|---|---|
| | | | | Set No. of H Peaks | δC (ppm) | Attribution C |
| 173.53 | s | 1 | C-25 | \ | \ | \ |
| 171.91 | s | 1 | C-28 | \ | \ | \ |
| 158.10 | s | 1 | C-8 | \ | \ | \ |
| 157.33 | s | 1 | C-37 | \ | \ | \ |
| 148.47 | s | 1 | C-35 | \ | \ | \ |
| 145.48 | d | 1 | C-38 | 8.250 | 157.33, 148.47, 122.08 | C-37, C-35, C-39 |
| 142.15 | s | 1 | C-16 | \ | \ | \ |
| 141.42 | s | 1 | C-45 | \ | \ | \ |
| 140.71 | s | 1 | C-17 | \ | \ | \ |
| 138.22 | s | 1 | C-5 | \ | \ | \ |
| 137.14 | s | 1 | C-29 | \ | \ | \ |
| 136.75 | s | 1 | C-32 | \ | \ | \ |
| 131.16 | s | 1 | C-1 | \ | \ | \ |
| 130.68 | d | 1 | C-3 | 7.638 | 138.22, 131.16 | C-5, C-1 |
| 130.51 | s | 1 | C-19 | \ | \ | \ |
| 130.40 | s | 1 | C-2 | \ | \ | \ |
| 129.39 | d | 1 | C-6 | 7.707 | 130.40, 127.72 | C-2, C-4 |
| 129.10 | s | 1 | C-11 | \ | \ | \ |
| 128.39 | d | 2 | C-30/34 | 7.129-7.063 | 136.75, 35.30 | C-32, C-27 |
| 128.37 | s | 1 | C-36 | \ | \ | \ |
| 128.18 | d | 2 | C-47/49 | 7.310 | 141.42 | C-45 |
| 128.11 | d | 2 | C-10/12 | 7.410 | 158.10, 114.87, 73.81 | C-8, C-9/13, C-14 |
| 128.03 | d | 2 | C-46/50 | 7.173 | 127.27,63.91 | C-48, C-42 |
| 127.72 | d | 1 | C-4 | 7.445-7.422 | 129.39, 67.66 | C-6, C-7 |
| | | 2 | C-31/33 | 7.129-7.063 | 148.47, 137.14 | C-35, C-29 |
| 127.27 | d | 1 | C-48 | 7.253 | 128.03 | C-46/50 |
| 125.83 | s | 1 | C-20 | \ | \ | \ |
| 122.08 | d | 1 | C-39 | 6.957 | 145.48, 136.75, 128.37 | C-38, C-32, C-36 |
| 117.22 | d | 1 | C-21 | 6.508 | 142.15, 140.71 | C-16, C-17 |
| 114.87 | d | 2 | C-9/13 | 7.042 | 158.10, 129.10 | C-8, C-11 |
| 114.57 | d | 1 | C-18 | 6.933 | 142.15, 140.71, 125.83 | C-16, C-17, C-20 |
| 74.19 | d | 1 | C-24 | 5.467 | 173.53, 125.83, 114.57,60.81 | C-25, C-20, C-18, C-23 |
| 73.81 | d | 1 | C-14 | 5.112-5.083 | 68.28 | C-15 |
| 68.28 | t | 1 | C-15 | 4.325, 3.971-3.897 | 142.15,73.81 | C-16, C-14 |
| 67.66 | t | 1 | C-7 | 5.121 | 158.10, 138.22, 129.39, 127.72 | C-8, C-5, C-6, C-4 |
| 63.91 | d | 1 | C-42 | 3.417 | 141.42, 128.03, 74.19, 60.81, 27.04 | C-45, C-46/50, C-24, C-23, C-43 |
| 60.81 | d | 1 | C-23 | 3.111 | 173.53, 125.83, 74.19,63.91, 27.34 | C-25, C-20, C-24, C-42, C-22 |
| 54.59 | d | 1 | C-26 | 4.842 | 173.53, 171.91, 137.14, 74.19, 35.30 | C-25, C-28, C-29, C-24, C-27 |
| 35.30 | t | 1 | C-27 | 3.303, 3.076-2.993 | 137.14, 128.39, 54.59 | C-29, C-30/34, C-26 |
| 27.34 | t | 1 | C-22 | 2.740,2.424 | 173.53, 130.51,125.83, 117.22, 60.81 | C-25, C-19, C-20, C-21, C-23 |
| 27.04 | t | 1 | C-43 | 1.953-1.864, 1.629-1.532 | 141.42 | C-45 |
| 23.15 | q | 1 | C-40 | 2.475 | 157.33, 128.37 | C-37, C-36 |
| 15.81 | q | 1 | C-41 | 2.110 | 157.33, 148.47, 128.37 | C-37, C-35, C-36 |
| 9.23 | q | 1 | C-44 | 0.531 | 63.91,27.04 | C-42, C-43 |

Mass spectrum of impurity B adopted an ESI source positive ion detection mode to obtain a peak of sample [M + H]⁺ 854.2771, consistent with the molecular weight corresponding to C₅₀H₄₅Cl₂N₃O₆. The molecular structure of the sample was further confirmed by NMR. Hydrogen spectrum (¹H-NMR) and carbon spectrum (¹³C-NMR, DEPT135°) show that the sample molecule has 44 hydrogen atoms (without active hydrogen) and 50 carbon atoms, and it can be determined that 2 carbonyl groups, 2 1,4-disubstituted benzene ring fragments, 1 monosubstituted benzene ring fragment, 1 1,3,4-trisubstituted benzene ring fragment, 1 1,2,4,5-tetrasubstituted benzene ring fragment and 1 2,3,4-trisubstituted pyridine ring fragment exist in the molecule in view of two-dimensional correlation spectra (¹H-¹H COSY, HSQC, HMBC); the connection modes of various fragments can be determined in view of the structure of the precursor of OAD2 of the sample and a two-dimensional correlation spectra: the signals related to H-24 (delta H5.467, s, 1H) and C-25 (delta C173.53, s) and the signals related to H-26 (delta H4.842, dd, J = 10.3, 5.4 Hz, 1H) and C-24 (delta C74.19, d) can be observed in HMBC, and it is known that the methylene at C-24 'in OAD2 or a pharmaceutically acceptable salt thereof is oxidized and is connected with C25' -NH- to form a 4-imidazolidinone ring structure fragment, so that the oxidative degradation impurity B, 2-3(-(4-((3,4-dichlorobenzyl)oxy)phenyl)-8-oxo-12-(1-phenylpropyl)-2,3,6,8,9,10-hexahydro-7H-6,9- epimino[1,4]dioxono[2',3':4,5]benzo[1,2-c]azepin-7-yl)-3-(4-(2,3-dimethylpyridin-4-yl)phenyl)propanoic acid is obtained.

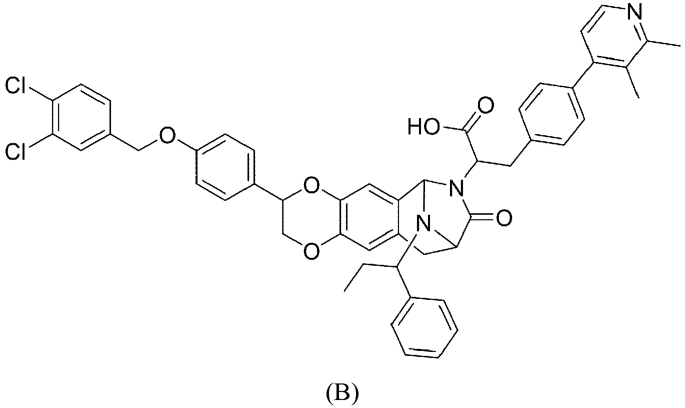

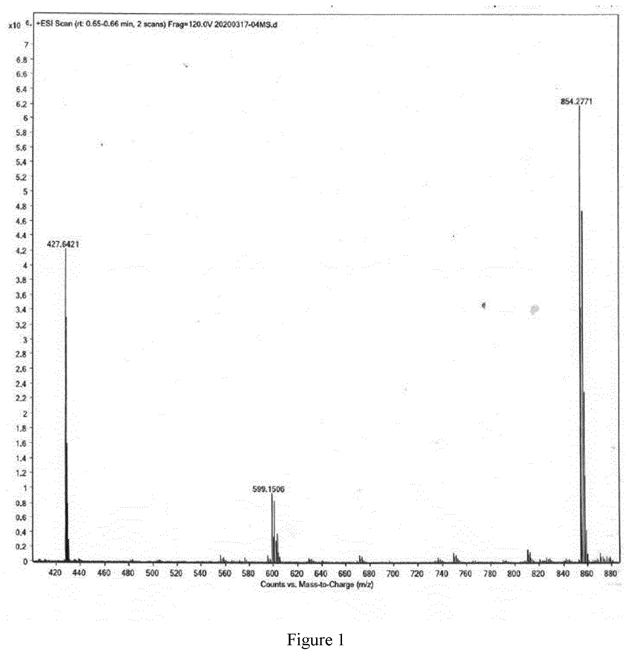

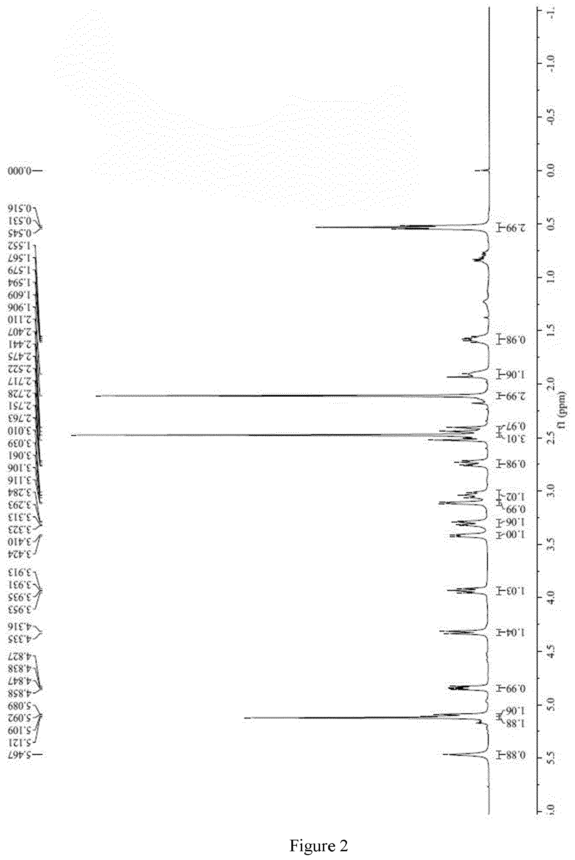

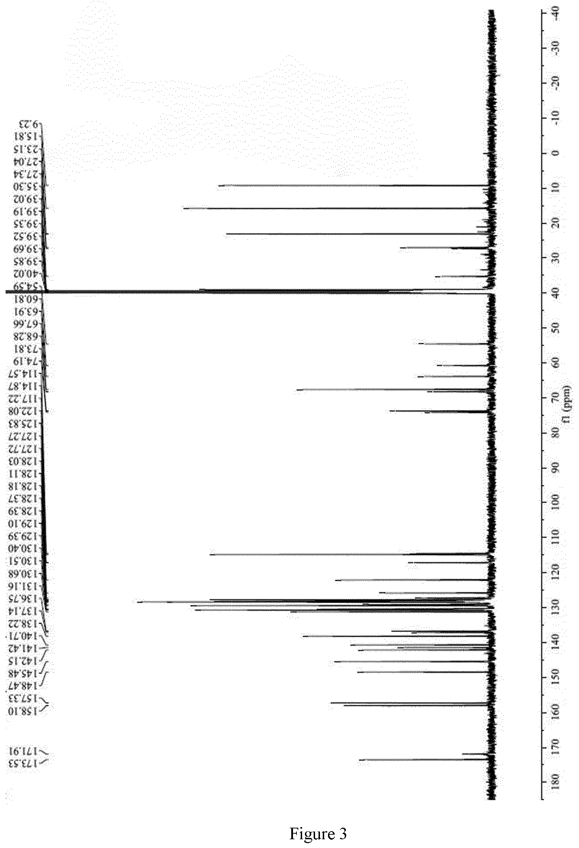

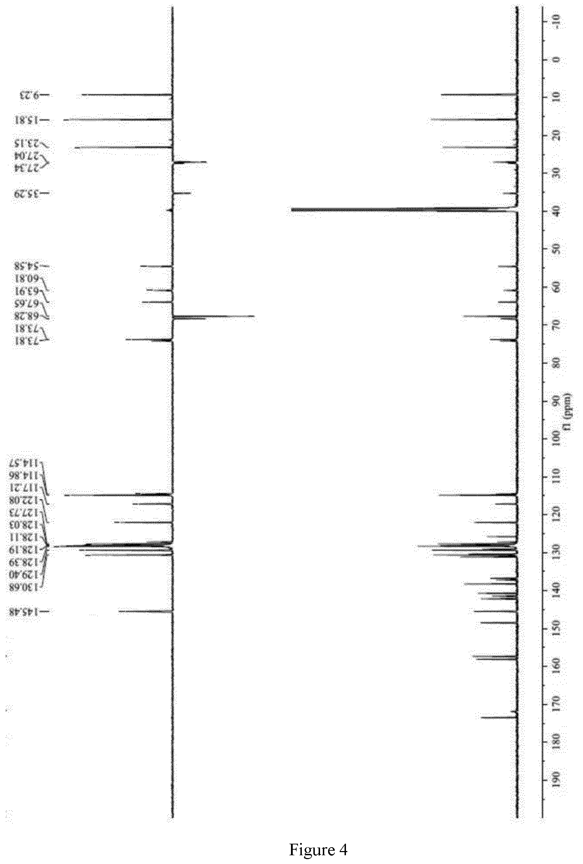

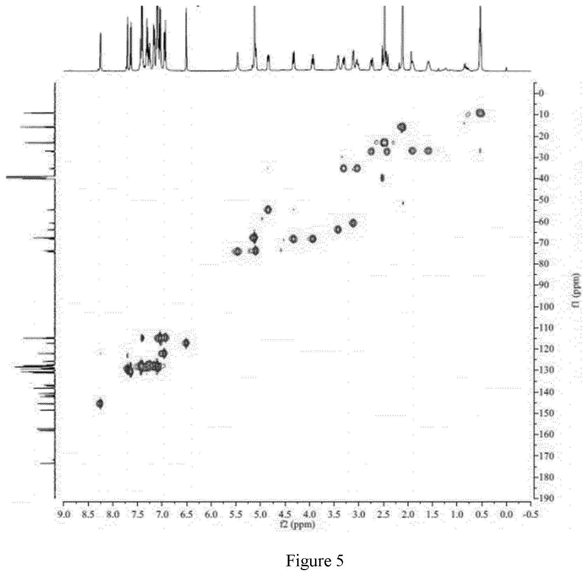

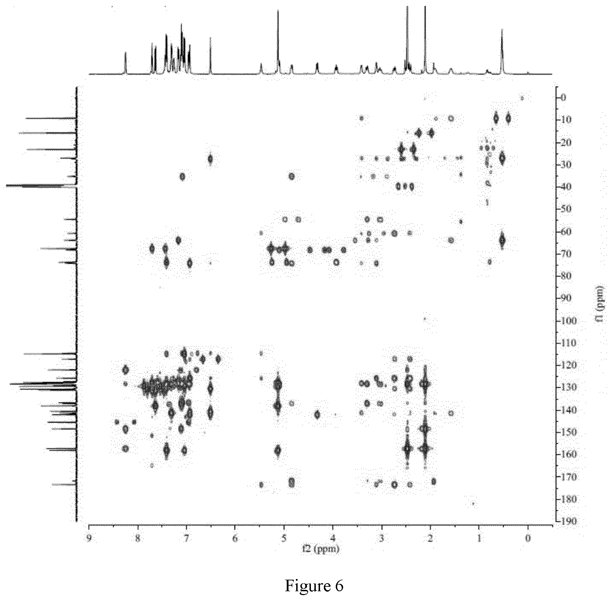

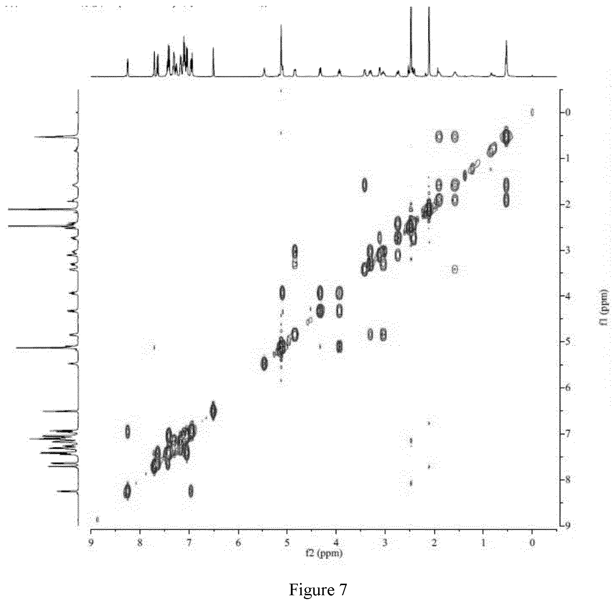

Figure 1-7 recites the analytical spectra corresponding to above-mentioned mass analysis.

### Example 15

### Methods for analyzing OAD2 or a pharmaceutically acceptable salt thereof or related substances

### high performance liquid chromatography (HPLC) (for Examples 1-17):

chromatographic condition and system adaptability test: amido hexadecyl silica gel as filler (Supelco Ascentis Express RP-Amide, 3.0mm×150mm, 2.7 µm); mobile phase A was 0.05% trifluoroacetic acid in water (V/V) and mobile phase B was 0.05% trifluoroacetic acid in acetonitrile-0.05% trifluoroacetic acid in methanol (1: 2, V/V); the flow rate was 0.5 ml/min; the column temperature was 50 °C; the detection wavelength was 280 nm; the temperature of the sample injection tray was 5 °C, and the number of theoretical plates was not less than 5000 calculated according to the peak of OAD2 or pharmaceutically acceptable salt thereof. The mobile phase gradient is shown in Table 15.

**Table 15- Mobile phase gradient conditions of HPLC detection system**

| Time (min) | Phase A (%) | Phase B (%) |
|---|---|---|
| 0 | 50 | 50 |
| 10 | 45 | 55 |
| 12 | 45 | 55 |
| 35 | 15 | 85 |
| 36 | 0 | 100 |
| 38 | 0 | 100 |
| 38.1 | 50 | 50 |
| 45 | 50 | 50 |

The determination method: an appropriate amount of OAD2 or a pharmaceutically acceptable salt thereof or related substances thereof was placed into a 250 ml measuring flask. An appropriate amount of diluent (0.05% trifluoroacetic acid acetonitrile solution: 0.05% trifluoroacetic acid aqueous solution =60:40 (V/V)) was added, and the mixture was subjected to ultrasonic treatment for 30 minutes to dissolve OAD2 or a pharmaceutically acceptable salt thereof or related substances thereof. The solution was cooled, diluted to the scale mark with a diluent, shaken well, filtered. 2ml of subsequent filtrate was precisely measured, placed into a 20ml measuring flask, and diluent was added to dilute the filtrate to the scale mark, shaken well to serve as the test article solution. 5 µl of the test article solution was precisely measured, injected into a liquid chromatograph, and the chromatogram was recorded. Separately, an appropriate amount of the reference substance of OAD2 or a pharmaceutically acceptable salt was precisely weighed, and a diluent was added for dissolving and diluting the same to a constant volume to prepare a solution whose amount of OAD2 or a pharmaceutically acceptable salt was equivalent to that of the test article solution (calculated by active ingredient C₅₀H₄₇Cl₂N₃O₆). The two were measured by the same method, and peak areas were calculated according to an external standard method.

### Example 16

### Stability experiment (preferred validation) of compositions of OAD2 or a pharmaceutically acceptable salt thereof

Through screening of the pharmaceutical compositions of the respective component formule in Examples 1 to 11, the stability experiment described in Example 12, and screening of the total impurity content and impurity B content, it is further determined that the preferred compositions are Examples 4 and 5. The researchers further measured the situations of related substances in the formulations of the compositions of Example 4 and Example 5.

The respective pharmaceutical formulation was prepared according to the formulation proportion and preparation method respectively corresponding to Example 4 and Example 5. The formulations were placed under different storage conditions, and the content of impurity B and the total content of impurities in the formulations were detected according to a plan. The specific stability experimental results of Example 4 are shown in Tables 16.1 and 16.2. The specific stability experimental results of Example 5 are shown in Tables 16.3 and 16.4.

**Table 16.1 Analysis of impurity content of the composition of Example 4 (Batch No. S200103) at 2-8 °C and 25-60 °C, respectively**

| **Example** | **Stability experiment (2 to 8°C)** | | | | | **Stability experiment (25 to 60°C)** | | | | | | |
|---|---|---|---|---|---|---|---|---|---|---|---|---|
| **4 (Batch No. S200103 )** | 0 day | 3 mont hs | 6 mont hs | 9 mont hs | 12 mont hs | 0 day | 1 mont h | 2 mont hs | 3 mont hs | 6 mont hs | 9 mont hs | 12 mont hs |
| API content | 99.8 | 100. 8 | 99.1 | 99.3 | 100. 3 | 99.8 | 99.8 | 99.4 | 99.6 | 97.5 | 99.6 | 99.7 |
| Impurity A | 0.06 | 0.08 | 0.07 | 0.06 | 0.06 | 0.06 | 0.08 | 0.1 | 0.11 | 0.09 | 0.08 | 0.08 |
| **Impurit yB** | **0.08** | **0.10** | **0.10** | **0.12** | **0.10** | **0.08** | **0.15** | **0.12** | **0.13** | **0.13** | **0.16** | **0.19** |
| Impuritie sC/D | 0.04 | 0.03 | 0.05 | 0.06 | 0.04 | 0.04 | 0.04 | 0.05 | 0.04 | 0.05 | 0.04 | 0.04 |
| Impurity E | / | / | / | / | / | / | / | / | / | / | / | / |
| Impurity F | 0.34 | 0.35 | 0.35 | 0.35 | 0.36 | 0.34 | 0.36 | 0.37 | 0.37 | 0.36 | 0.35 | 0.36 |
| Impurity G | 0.03 | 0.03 | 0.04 | 0.03 | 0.02 | 0.03 | 0.03 | 0.03 | 0.03 | 0.03 | 0.02 | 0.02 |
| Impurity H | / | / | / | / | / | / | / | / | / | / | / | / |
| Impurity I | / | / | / | / | / | / | / | / | / | / | / | / |
| Unknow n single impurity | 0.02 | 0.04 | / | / | 0.03 | 0.02 | 0.02 | 0.03 | 0.03 | / | 0.03 | 0.04 |
| **Total Impuriti es** | **0.61** | **0.63** | **0.59** | **0.61** | **0.66** | **0.61** | **0.71** | **0.69** | **0.73** | **0.66** | **0.72** | **0.81** |
| Water | 2.7 | 2.8 | 2.8 | 3.1 | 2.9 | 2.7 | 2.8 | 2.6 | 2.7 | 2.8 | 2.9 | 2.8 |

**Table 16.2 Analysis of impurity content of the composition of Example 4 (Batch No. S200103) at 30-65 °C and 40-75 °C, respectively**

| **Example** | **Stability experiment (30 to 65°C)** | | | | | **Stability experiment (40 to 75°C)** | | | | |
|---|---|---|---|---|---|---|---|---|---|---|
| **4 (Batch No. S200103 )** | 0 day | 3 mont hs | 6 mont hs | 9 mont hs | 12 mont hs | 0 day | 1 mont h | 2 mont hs | 3 mont hs | 6 mont hs |
| API content | 99.8 | 99.7 | 99.5 | 98.0 | 98.8 | 99.8 | 98 | 99.1 | 99.1 | 96.0 |
| Impurity A | 0.06 | 0.1 | 0.09 | 0.08 | 0.10 | 0.06 | 0.09 | 0.12 | 0.11 | 0.19 |
| **Impurit yB** | **0.08** | **0.14** | **0.19** | **0.19** | **0.24** | **0.08** | **0.17** | **0.22** | **0.20** | **0.47** |
| Impuritie sC/D | 0.04 | 0.03 | 0.04 | 0.05 | 0.04 | 0.04 | 0.05 | 0.04 | 0.03 | 0.06 |
| Impurity E | / | / | / | / | / | / | / | / | / | / |
| Impurity F | 0.34 | 0.37 | 0.36 | 0.36 | 0.37 | 0.34 | 0.36 | 0.37 | 0.38 | 0.34 |
| Impurity G | 0.03 | 0.03 | 0.03 | 0.03 | 0.03 | 0.03 | 0.03 | 0.03 | 0.03 | 0.02 |
| Impurity H | / | / | / | / | / | / | / | / | / | / |
| Impurity I | / | / | / | / | / | / | / | / | / | / |
| Unknow n single Impurity | 0.02 | 0.03 | 0.03 | 0.03 | 0.04 | 0.02 | 0.02 | 0.03 | 0.04 | 0.06 |
| **Total Impuriti es** | **0.61** | **0.72** | **0.74** | **0.76** | **0.95** | **0.61** | **0.78** | **0.88** | **0.86** | **1.30** |
| Water | 2.7 | 2.8 | 2.7 | 2.8 | 2.8 | 2.7 | 3.1 | 3.10 | 3.1 | 3.2 |

**Table 16.3 Analysis of impurity content of the composition of Example 5 (Batch No. S200108) at 2-8 °C and 25-60 °C, respectively**

| **Example** | **Stability experiment (2 to 8°C)** | | | | | **Stability experiment (25 to 60°C)** | | | | | | |
|---|---|---|---|---|---|---|---|---|---|---|---|---|
| **5 (Batch No. S200108 )** | 0 day | 3 mont hs | 6 mont hs | 9 mont hs | 12 mont hs | 0 day | 1 mont h | 2 mont hs | 3 mont hs | 6 mont hs | 9 mont hs | 12 mont hs |
| API content | 99.6 | 100. 9 | 97.6 | 100. 0 | 100. 5 | 99.6 | 99.1 | 99.8 | 99.7 | 96.9 | 99.9 | 99.6 |
| Impurity A | 0.06 | 0.07 | 0.08 | 0.06 | 0.06 | 0.06 | 0.07 | 0.1 | 0.09 | 0.07 | 0.08 | 0.08 |
| **Impurit yB** | **0.08** | **0.09** | **0.10** | **0.09** | **0.09** | **0.08** | **0.12** | **0.11** | **0.11** | **0.13** | **0.15** | **0.17** |
| Impuritie sC/D | 0.04 | 0.04 | 0.05 | 0.03 | 0.04 | 0.04 | 0.03 | 0.05 | 0.03 | 0.04 | 0.04 | 0.04 |
| Impurity E | / | / | / | / | / | / | / | / | / | / | / | / |
| Impurity F | 0.35 | 0.36 | 0.35 | 0.36 | 0.37 | 0.35 | 0.36 | 0.37 | 0.35 | 0.34 | 0.35 | 0.36 |
| Impurity G | 0.02 | 0.03 | 0.04 | 0.02 | 0.03 | 0.02 | 0.03 | 0.02 | 0.02 | 0.03 | 0.03 | 0.03 |
| Impurity H | / | / | / | / | / | / | / | / | / | / | / | / |
| Impurity I | / | / | / | / | / | / | / | / | / | / | / | / |
| Unknow n single Impurity | 0.02 | 0.04 | / | 0.02 | 0.03 | 0.02 | 0.03 | 0.03 | 0.03 | 0.02 | 0.02 | 0.04 |
| **Total Impuriti es** | **0.63** | **0.66** | **0.61** | **0.60** | **0.64** | **0.63** | **0.68** | **0.74** | **0.67** | **0.66** | **0.67** | **0.79** |
| Water | 2.7 | 2.9 | 2.9 | 3.0 | 2.9 | 2.7 | 2.8 | 2.7 | 2.8 | 2.9 | 2.8 | 2.9 |

**Table 16.4 Analysis of impurity content of the composition of Example 5 (Batch No. S200108) at 30-65 °C and 40-75 °C, respectively**

| **Example** | **Stability experiment (30 to 65°C)** | | | | | **Stability experiment (40 to 75°C)** | | | | |
|---|---|---|---|---|---|---|---|---|---|---|
| **5 (Batch No. S200108 )** | 0 day | 3 mont hs | 6 mont hs | 9 mont hs | 12 mont hs | 0 day | 1 mont hs | 2 mont hs | 3 mont hs | 6 mont hs |
| API content | 99.6 | 100. 2 | 99.3 | 99.0 | 98.4 | 99.6 | 98.2 | 99.0 | 97.3 | 98.0 |
| Impurity A | 0.06 | 0.10 | 0.09 | 0.09 | 0.10 | 0.06 | 0.1 | 0.12 | 0.13 | 0.14 |
| **Impurit yB** | **0.08** | **0.16** | **0.17** | **0.23** | **0.23** | **0.08** | **0.19** | **0.18** | **0.19** | **0.34** |
| Impuritie sC/D | 0.04 | 0.04 | 0.04 | 0.05 | 0.04 | 0.04 | 0.04 | 0.04 | 0.03 | 0.05 |
| Impurity E | / | / | / | / | / | / | / | / | / | / |
| Impurity F | 0.35 | 0.37 | 0.35 | 0.38 | 0.36 | 0.35 | 0.35 | 0.36 | 0.35 | 0.36 |
| Impurity G | 0.02 | 0.03 | 0.03 | 0.03 | 0.02 | 0.02 | 0.03 | 0.03 | 0.02 | 0.03 |
| Impurity H | / | / | / | / | / | / | / | / | / | / |
| Impurity I | / | / | / | / | / | / | / | / | / | / |
| Unknow n single Impurity | 0.02 | 0.04 | 0.03 | 0.03 | 0.04 | 0.02 | 0.02 | 0.04 | 0.06 | 0.05 |
| **Total Impuriti es** | **0.63** | **0.79** | **0.71** | **0.89** | **0.92** | **0.63** | **0.76** | **0.84** | **0.87** | **1.05** |
| Water | 2.7 | 2.8 | 2.9 | 2.7 | 2.7 | 2.7 | 2.8 | 2.7 | 2.7 | 2.8 |

As can be seen from Tables 16.1 to 16.4, the compositions corresponding to the preferred Example 4 and Example 5 have good formulation stability and can be stored stably at room temperature. Impurity B is the only impurity whose content increases with time. After the optimization of solubilizer and disintegrant in the formulation, its growth rate has been well controlled, so that the total impurity content has also been well controlled.

### Example 17

### Preparation method of impurity B

22 g of free OAD2 and 350 ml of dichloromethane was charged to a 500 ml reaction bottle and the mixture was stirred to dissolve. 100 ml of water was added and the system became cloudy. 7 g of TEMPO was added. After stirring for 15 minutes, 20 g of iodobenzenediacetic acid was added in batches, and the reactants were allowed to react at 20°C for 2 hours till completion. The reaction solution was washed successively with 15% aqueous sodium thiosulfate solution, saturated aqueous sodium bicarbonate solution and saturated aqueous sodium chloride solution. The reaction solution was dried over anhydrous sodium sulfate for 15 minutes, filtered to remove the desiccant, and concentrated under reduced pressure to obtain the crude impurity B.

The crude product was purified by column chromatography, slurried with ethyl acetate/petroleum ether mixed system for 2 hours, filtered, and oven-dried to obtain 8g offwhite solid impurity B.

### Example 18

### Forced Degradation Studies No.2

### Sample preparation for test groups 1-6

A stock solution was prepared by dissolving 12.90 mg of OAD2 2HCl in acetonitrile:water (70:30) to a final volume of 25 mL (0.442 mg/mL). For test groups 1 and 2, the test solution was prepared by mixing 2 mL of the stock solution and 2 mL of water. For test groups 3 and 4, the test solution was prepared by mixing 2 mL of the stock solution and 2 mL of 0.1 N HCl solution. For test groups 5 and 6, the test solution was prepared by mixing 2 mL of the stock solution and 2 mL of 0.1 N NaOH solution. Test groups 1, 3, and 5 (at room temperature) were shielded from light by wrapping with aluminum foil.

### Sample preparation for test groups 7-8

A stock solution was prepared by dissolving 5.37 mg of OAD2 2HCl in acetonitrile:water (40:60) to a final volume of 10 mL (0.46 mg /mL). For test group 7, the test solution was prepared by mixing 2 mL of the stock solution and 2 mL of 3% aqueous H₂O₂. For test group 8, the test solution was prepared by dissolving 5.58 mg to 5.64 mg of OAD2 2HCl in acetonitrile:water (40:60) to a final volume of 25 mL.

The samples in each test group were stored either at room temperature or at 60 °C and observed after the periods indicated in the Table below.

| Test Group | Conditions | Temperature | Period |
|---|---|---|---|
| 1 | Water | RT | 1 and 2 days |
| 2 | Water | 60 °C | 1 and 2 days |
| 3 | 0.05 N HCl solution | RT | 1 and 2 days |
| 4 | 0.05 N HCl solution | 60 °C | 1 and 2 days |
| 5 | 0.05 N NaOH solution | RT | 1 and 2 days |
| 6 | 0.05 N NaOH solution | 60 °C | 1 and 2 days |
| 7 | 1.5% solution of H₂O₂ | RT | 90 min and 24 hours |
| 8 | UV light (365 nm) | RT | 24 hours |

With the exception of test group 7, no substantial growth in any impurities or loss of active ingredient was seen by HPLC analysis in any of these forced degradation studies. In test group 7, a significant increase in impurities was seen after 90 minutes with an associated 10% loss of active ingredient. Further, through HPLC analysis, the largest impurity had a relative retention time consistent with impurity B.

### HPLC conditions (for Examples 18-24)

### Assay (on anhydrous basis)

A validated, reverse phase liquid chromatography gradient method was applied to determine assay (content of the active ingredient). The column used was Supelco Ascentis Express RP-Amide, 150 × 3.0 mm, 2.7µ or equivalent column. Mobile phase A comprises 0.05% TFA in water (v/v). Mobile phase B comprises 0.05% TFA in acetonitrile: methanol (1:2) (v/v). The sample was run using a step gradient from time zero (50% mobile phase A) to 38 minutes (99% mobile phase B). The diluent used to dissolve the standard and sample was a 60:40 mixture of acetonitrile and water. Chromatographic peaks were detected using UV detector at 280 nm. Assay value was obtained by comparing peak responses, expressed as the peak area of a sample formulation of known concentration and the peak area obtained from a standard formulation of known concentration.

The impurities were quantitated using the assay procedure described above. The method utilized area normalization approach to determine percent level of each impurity.

### Example 19

### Preparation of comparative formulation 1 ("Formulation CF1")

The following process was used to prepare tablets with 75 mg of active ingredient. The amounts of individual ingredients are provided in Table 19.2 below.

Step 1. Polysorbate 80, vitamin E TPGS, poloxamer 188 and copovidone were dissolved in an amount of water with an overhead stirrer until dissolution. The amount of water may be equal to between 50% and 100% (by weight of the combined weights of polysorbate 80/vitamin E TPGS/ poloxamer 188/copovidone). Water was warmed to 50°C to speed up the dissolution.

Step 2. OAD2 dihydrochloride was added to the above polysorbate 80/vitamin E TPGS/ poloxamer 188/copovidone solution and the mixture was mixed until dissolution.

Step 3. Microcrystalline cellulose, pregelatinized starch and crospovidone were passed through sieve # 20 and charged into a fluid bed dryer and mixed.

Step 4. The solution from Step 2 was sprayed onto the mixture from Step 3 to form sprayed granules.

Step 5. The sprayed granules were milled and passed through #40 screen.

Step 6. The potency of OAD2 dihydrochloride in the sprayed granules material was determined and all of the extragranular materials were adjusted to achieve the required potency of 75 mg per tablet.

Step 7. Colloidal silicon dioxide, microcrystalline cellulose, crospovidone, and citric acid were passed through #30 screen in separate polyethylene bags.

Step 8. The screened granules and the screened excipients from Step 6 were charged into a V shell blender and mixed for 15-20 minutes.

Step 9. Equal amount of mixtures were charged to the screened magnesium stearate in polyethylene bags and mixed for a few minutes.

Step 10. The screened magnesium stearate was charged into the V shell blender and mixed for several minutes.

Step 11. The powder was taken out and compressed into tablets having 75 mg of OAD2 dihydrochloride and a total tablet weight of about 620-640 mg.

Step 12. After compression, the tablets were coated using Opadry II white 85F18422 to a weight gain of 3%.

**Table 19.1 Source and grade of ingredients for Formulation CF1**

| Ingredient | Brand/Grade |
|---|---|
| Polysorbate 80 | Tween 80 HP-LQ- (MH), USP-NF/EP/JP |
| Vitamin E TPGS | NF |
| Copovidone | Kollidon VA 64, USP/NF/JP |
| Poloxamer 188 | Kolliphor P188, USP/NF/EP/JP |
| Microcrystalline Cellulose, | Avicel PH 133, NF, Ph. Eur., JP |
| Pregelatinized Corn Starch | Starch 1500, USP/NF |
| Crospovidone, | Kollidon CL, USP/NF |
| Citric Acid | Anhydrous powder, USP |
| Colloidal Silicon Dioxide | Cab-O-Sil M5P, NF |
| Magnesium Stearate | non-bovine #5712, NF/EP |
| Coating | Opadry II White 85F18422 |
| Purified Water | USP/EP |

The manufacturer's certificate of analysis for the lot of crospovidone used in Formulation CF1 showed that the peroxide level, expressed as H₂O₂, was 58 ppm as measured by processes described in "Crospovidone" Type A of Ph. Eur. 8th edition, USP37/NF32, Japanese Pharmacopoeia 16th ed. Supplement 1. Independent analysis was performed using the methods in NF 33 monograph on this batch of crospovidone and prior to use in the CF1 formulation, the assay for H₂O₂ showed an absorbance against the appropriate compensation liquid of 0.13 where an absorbance of 0.35 corresponds to 400 ppm of H₂O₂. Absorbance of 0.13 converts to about 148 ppm of H₂O₂ in Type A of Ph. Eur.

**Table 19.2. Ingredients of Formulation CF1**

| | Ingredients | %/Tablet | Weight/Tablet (mg) |
|---|---|---|---|
| Intragranular | OAD2 dihydrochloride salt * | 12.10 | 75.00 |
| | Copovidone, | 1.00 | 6.20 |
| | Vitamin E TPGS | 1.60 | 9.92 |
| | Polysorbate 80 | 3.00 | 18.60 |
| | Poloxamer 188 | 1.00 | 6.20 |
| | Microcrystalline Cellulose* | 24.00 | 148.80 |
| | Pregelatinized Corn Starch | 20.00 | 124.00 |
| | Crospovidone | 10.00 | 62.00 |
| | Purified Water** | QS | QS |
| | Intragranular Total | 72.70 | 450.72 |
| Extragranular | Microcrystalline Cellulose | 7.00 | 43.40 |
| | Crospovidone | 7.21 | 44.68 |
| | Colloidal silicon dioxide | 0.50 | 3.10 |
| | Magnesium Stearate | 0.50 | 3.10 |
| | Citric Acid | 12.10 | 75.00 |
| | Opadry II White | | 18.60 |
| | Extragranular Total | 27.31 | 187.88 |
| Total | | 100.0 | 638.6 |

* Adjusted based on Use-at-Value (UAV). Corresponding adjustment was made in microcrystalline cellulose based on the UAV of the API to keep the weight of the tablet constant.
** Removed while drying

### Example 20

### Stability study of Formulation CF1

Tablets from three separate batches (Batch 1, 2, and 3) of the Formulation CF1 were packaged in PVC/aluminum foil blisters as per ICH conditions and the tablets of formulation CF1 were placed on a stability program at 5 °C, at 25 °C/60% relative humidity (RH) and at 40 °C/75% RH.

The growth of impurity B in each of Batches 1, 2, and 3 over time under various storage conditions is provided in Tables 20.1, 20.2, and 20.3.

Tables 20.1-A, 20.2-A, 20.3-A provide complete lists of impurities in each batch after 3 months under various storage conditions wherein the retention times are relative to the retention time of OAD2 dihydrochloride, and impurity B is highlighted in bold. The relative retention times of impurity B in Tables 20.1-A, 20.2-A, and 20.3-A at time zero (initial) are 0.56-0.60. The assay used high performance liquid chromatography methods described herein.

The symbol "-" in the Tables indicates time points where no data was collected.

**Table 20.1 - Growth of impurity B in Batch 1 of Formulation CF1**

| | Storage Conditions | | |
|---|---|---|---|
| | 5 °C | 25 °C/60% RH | 40 °C/75% RH |
| Period | Area % of impurity B | Area % of impurity B | Area % of impurity B |
| Initial | 0.57 | 0.57 | 0.57 |
| 1 month | 0.53 | 0.53 | 0.49 |
| 2 months | 0.60 | 0.61 | 0.54 |
| 3 months | 0.89 | 1.02 | 0.66 |

**Table 20.2 - Growth of impurity B in Batch 2 of Formulation CF1**

| | Storage Conditions | | |
|---|---|---|---|
| | 5 °C | 25 °C/60% RH | 40 °C/75% RH |
| Period | Area % of impurity B | Area % of impurity B | Area % of impurity B |
| Initial | 0.38 | 0.38 | 0.38 |
| 1 month | 0.44 | 0.49 | 0.45 |
| 2 months | 0.53 | 0.63 | 0.55 |
| 3 months | 0.47 | 0.66 | 0.54 |
| 6 months | 0.58 | 0.85 | - |
| 9 months | 0.75 | 1.02 | - |

**Table 20.3 - Growth of impurity B in Batch 3 of Formulation CF1**

| | Storage Conditions | | |
|---|---|---|---|
| | 5 °C | 25 °C/60% RH | 40 °C/75% RH |
| Period | Area % of impurity B | Area % of impurity B | Area % of impurity B |
| Initial | 0.67 | 0.67 | 0.67 |
| 1 month | 0.68 | 0.77 | 0.78 |
| 2 months | 0.68 | 0.92 | 0.77 |
| 3 months | 0.69 | 0.89 | 0.78 |
| 6 months | 0.74 | 1.11 | - |
| 9 months | 0.50 | 1.00 | - |

**Table 20.1-A - Stability data of Batch 1 after 3 months under various storage conditions**

| Test | Acceptanc e Criteria | Initial | | 3 months | | | | | |
|---|---|---|---|---|---|---|---|---|---|
| | | | | 5 °C | | 25 °C/60% RH | | 40 °C/75% RH | |
| Assay | 90.0-110% | 101.44% | | 101.8% | | 102.2% | | 100.04% | |
| Impuritie s | Reported impurities ≥0.05% by area | RRT | Area % | RRT | Area % | RRT | Area % | RRT | Area % |
| | | 0.16 | 0.07 | 0.14 | 0.08 | 0.14 | 0.08 | 0.14 | 0.08 |
| | | 0.45 | 0.16 | 0.45 | <0.05 | 0.45 | <0.0 5 | 0.45 | <0.05 |
| | | **0.56** | **0.57** | **0.63** | **0.89** | **0.63** | **1.02** | **0.63** | **0.66** |
| | | 0.63 | 0.12 | 0.71 | 0.11 | 0.71 | 0.10 | 0.71 | 0.10 |
| | | 0.74 | 0.13 | - | - | - | - | - | - |
| | | 0.84 | 0.07 | 0.86 | 0.10 | 0.86 | 0.10 | 0.86 | 0.10 |
| | | 0.89 | <0.0 5 | 0.89 | 0.06 | 0.89 | 0.06 | 0.89 | 0.05 |
| | | | | 0.98 | 0.04 | 0.98 | 0.04 | 0.98 | 0.03 |
| | | 1.13 | 0.94 | 1.06 | 0.84 | 1.06 | 0.84 | 1.06 | 0.80 |
| | | 1.19 | 0.13 | 1.10 | 0.10 | 1.10 | 0.15 | 1.10 | 0.11 |
| | | 1.25 | 0.11 | 1.13 | 0.12 | 1.13 | 0.12 | 1.13 | 0.13 |
| | | | | | | | | 2.49 | 0.13 |
| | | | | | | | | 2.50 | 0.05 |
| Total Impuritie s | Reported results by area % | 2.27% | | 2.39% | | 2.61% | | 2.64% | |

**Table 20.2-A - Stability data of Batch 2 after 3 months under various storage conditions**

| Test | Acceptanc e Criteria | Initial | | 3 months | | | | | |
|---|---|---|---|---|---|---|---|---|---|
| | | | | 5 °C | | 25 °C/60% RH | | 40 °C/75% RH | |
| Assay | 90.0-110% | 103.2% | | 105.1% | | 102.4% | | 100.4% | |
| Impuritie s | Reported impurities ≥0.05% by area | RRT | Area % | RRT | Area % | RRT | Area % | RRT | Area % |
| | | 0.14 | ND | 0.14 | ND | 0.14 | ND | 0.14 | ND |
| | | 0.48 | ND | 0.48 | ND | 0.48 | ND | 0.48 | ND |
| | | **0.58** | **0.38** | **0.58** | **0.47** | **0.56** | **0.66** | **0.56** | **0.54** |
| | | 0.76 | ND | 0.76 | ND | 0.76 | ND | 0.76 | ND |
| | | 1.08 | 0.91 | 1.10 | 0.92 | 1.10 | 0.92 | 1.10 | 0.88 |
| | | 1.11 | 0.07 | 1.11 | ND | 1.11 | ND | 1.11 | ND |
| | | 1.15 | 0.12 | 1.13 | ND | 1.14 | 0.08 | 1.14 | 0.06 |
| | | 1.15 | ND | 1.15 | ND | 1.15 | ND | 2.11 | ND |
| | | 2.11 | 0.06 | 2.11 | ND | 2.11 | ND | 2.15 | ND |
| | | 2.15 | 0.05 | 2.17 | ND | 2.17 | ND | 2.18 | ND |
| | | 2.16 | 0.06 | 2.23 | 0.10 | 2.30 | 2.30 | 2.22 | ND |
| | | 2.31 | ND | 2.31 | 0.08 | 2.33 | 0.05 | 2.36 | 0.19 |
| | | 2.34 | ND | 2.34 | 0.07 | 2.44 | 0.07 | 2.43 | 0.08 |
| | | 2.45 | ND | 2.45 | 0.06 | 2.53 | 0.06 | 2.47 | 0.05 |
| | | 2.50 | ND | 2.50 | 0.06 | 2.54 | 0.09 | 2.54 | 0.39 |
| Total Impuritie s | Reported results by area % | 1.4% | | 1.5% | | 1.8% | | 1.9% | |

**Table 20.3-A - Stability data of Batch 3 after 3 months under various storage conditions**

| Test | Acceptanc e Criteria | Initial | | 3 months | | | | | |
|---|---|---|---|---|---|---|---|---|---|
| | | | | 5 °C | | 25 °C/60% RH | | 40 °C/75% RH | |
| Assay | 90.0-110% | 103.3% | | 104.4% | | 103.3% | | 100.1% | |
| Impuritie s | Reported impurities ≥0.05% by area | RR T | Area % | RR T | Area % | RRT | Area % | RRT | Area % |
| | | 0.51 | ND | 0.51 | ND | 0.51 | ND | 0.51 | 0.06 |
| | | **0.59** | **0.28*** | **0.56** | **0.25*** | **0.56** | **0.46*** | **0.56** | **0.43*** |
| | | **0.60** | **0.39*** | **0.59** | **0.44*** | **0.59** | **0.43*** | **0.59** | **0.35*** |
| | | 0.68 | 0.12 | 0.68 | 0.10 | 0.68 | 0.10 | 0.69 | 0.11 |
| | | 0.82 | 0.10 | 0.82 | 0.09 | 0.82 | 0.09 | 0.80 | 0.09 |
| | | 0.87 | 0.06 | 0.87 | 0.06 | 0.85 | 0.06 | 0.85 | 0.06 |
| | | 1.08 | 0.84 | 1.11 | 0.85 | 1.10 | 0.85 | 1.09 | 0.81 |
| | | 1.11 | 0.16 | 1.14 | 0.16 | 1.14 | 0.15 | 1.11 | ND |
| | | 1.15 | 0.12 | 1.18 | 0.12 | 1.18 | 0.11 | 1.14 | 0.14 |
| | | 1.17 | ND | 1.17 | ND | 1.17 | ND | 1.17 | 0.12 |
| | | 2.09 | 0.06 | 2.09 | ND | 2.09 | ND | 2.09 | ND |
| | | 2.10 | 0.07 | 2.10 | ND | 2.10 | ND | 2.10 | ND |
| | | 2.29 | ND | 2.29 | ND | 2.29 | 0.09 | 2.28 | 0.08 |
| | | 2.43 | ND | 2.43 | ND | 2.43 | 0.06 | 2.43 | ND |
| | | 2.54 | ND | 2.54 | ND | 2.54 | 0.07 | 2.54 | ND |
| Total Impuritie s | Reported results by area % | 2.1% | | 2.1% | | 2.3% | | 2.5% | |

| | | | | | | | | | |
|---|---|---|---|---|---|---|---|---|---|
| * indicates a split peak | | | | | | | | | |

### Example 21

### Preparation of comparative formulation 2 ("Formulation CF2")

The following process was used to prepare tablets with 100 mg of active ingredient. Process for the preparation of tablets with 100 mg of active substance. The amounts of individual ingredients are provided in Table 21.2 below.

Step 1. Polysorbate 80, vitamin E TPGS and copovidone were dissolved in an amount of water with an overhead stirrer until dissolution. Water was warmed to 50°C to speed up the dissolution.

Step 2. OAD2 dihydrochloride was added to the above polysorbate 80/vitamin E TPGS/copovidone solution and the mixture was mixed until dissolution.

Step 3. Microcrystalline cellulose, pregelatinized starch and crospovidone were passed through sieve #30 and charged into a fluid bed dryer and mixed.

Step 4. The solution from Step 2 was sprayed onto the mixture from Step 3 to form sprayed granules.

Step 5. The sprayed granules were milled and passed through #40 screen.

Step 6. The potency of OAD2 dihydrochloride in the sprayed granules material was determined and all of the extragranular materials were adjusted to achieve the required potency of 100 mg per tablet.

Step 7. Colloidal silicon dioxide, microcrystalline cellulose, crospovidone, poloxamer 188, and citric acid were passed through #30 screen in separate polyethylene bags.

Step 8. The screened granules and the screened excipients from Step 6 were charged into a V shell blender and mixed for 15-20 minutes.

Step 9. Equal amount of mixtures were charged to the screened magnesium stearate in polyethylene bags and mixed for a few minutes.

Step 10. The screened magnesium stearate was charged into the V shell blender and mixed for several minutes.

Step 11. The powder was taken out and compressed into tablets having 100 mg of OAD2 dihydrochloride and a total tablet weight of about 820-840 mg.

**Table 21.1 Source and grade of ingredients for Formulation CF2**

| Ingredient | Brand/Grade |
|---|---|
| Polysorbate 80 | Tween 80, NF |
| Vitamin E TPGS | NF |
| Copovidone | Kollidon VA 64, USP |
| Poloxamer 188 | Pluronic F68, NF |
| Microcrystalline Cellulose, | Avicel PH 133, NF/BP |
| Pregelatinized Corn Starch | Starch 1500, NF |
| Crospovidone, | Kollidon CL, Ph.Eur. USP/NF, Type A |
| Citric Acid | Anhydrous powder, USP |
| Colloidal Silicon Dioxide | Cab-O-Sil M5P, NF |
| Magnesium Stearate | Hyqual, vegetable grade, non-bovine, NF |
| Purified Water | USP |

The manufacturer's certificate of analysis for the lot of crospovidone used in Formulation CF2 showed that the peroxide level, expressed as H₂O₂, was 50 mg/kg (or ppm) as measured by processes described in "Crospovidone" of Ph. Eur. 6^{th} edition, Supplement 6.3 (Type A), USP32/NF37, JPE 2004. Prior to use in Formulation CF2, independent analysis was performed using the methods in European Pharmacopeia as of 2013 (Type A) on the same batch of crospovidone and found that the amount of H₂O₂ was not more than 400 ppm.

**Table 21.2 - Ingredients of Formulation CF2**

| | Ingredients | %/Tablet | Weight/Tablet (mg) |
|---|---|---|---|
| Intragranular | OAD2 dihydrochloride salt * | 12.00 | 100.00 |
| | Copovidone | 1.00 | 8.33 |
| | Vitamin E TPGS | 1.60 | 13.34 |
| | Polysorbate 80 | 3.00 | 25.00 |
| | Microcrystalline Cellulose* | 24.00 | 200.00 |
| | Pregelatinized Corn Starch | 20.00 | 166.67 |
| | Crospovidone | 10.00 | 83.34 |
| | Purified Water** | QS | QS |
| | Intragranular Total | 71.60 | 596.68 |
| Extragranular | Microcrystalline Cellulose | 7.00 | 58.33 |
| | Crospovidone | 7.40 | 61.67 |
| | Colloidal Silicon Dioxide | 0.50 | 4.17 |
| | Magnesium Stearate | 0.50 | 4.17 |
| | Citric Acid | 12.10 | 100.00 |
| | Poloxamer 188 | 1.00 | 8.33 |
| | Extragranular Total | 28.40 | 236.67 |
| Total | | 100.0 | 833.35 |

| | | | |
|---|---|---|---|
| * Adjusted based on Use-at-Value (UAV). Corresponding adjustment was made in microcrystalline cellulose based on the UAV of the API to keep the weight of the tablet constant. ** Removed while drying | | | |

### Example 22

### Stability study of Formulation CF2

Tablets from a batch of Formulation CF2 were placed on a stability program under the conditions and time points listed in Table 22.1. The tablets were packaged in 75 cc high density polyethylene bottles with a rayon coil and desiccant and capped.

Tables 22.1-A and 22.1-B provide complete lists of impurities in the batch of Formulation CF2 at 3, 12, and 24 months under various storage conditions wherein the retention times are relative to the retention time of OAD2 dihydrochloride, and impurity B is highlighted in bold. The relative retention time of impurity B in Tables 22.1-A and 22.2-B at time zero (initial) is 0.56. The assay used high performance liquid chromatography methods described herein.

The symbol "-" in the Tables indicates time points where no data (ND) was collected.

**Table 22.1 - Growth of impurity B in Formulation CF2**

| | Storage Conditions | |
|---|---|---|
| | 2-8 °C | 25 °C/60% RH |
| Period | Area % of impurity B | Area % of impurity B |
| Initial | <0.05 | <0.05 |
| 1 month | - | <0.05 |
| 2 months | - | <0.05 |
| 3 months | 0.13 | 0.12 |
| 6 months | 0.07 | 0.15 |
| 9 months | - | 0.75 |
| 12 months | <0.05 | 0.43 |
| 18 months | - | 0.68 |
| 24 months | 0.26 | 0.76 |

**Table 22.1-A - Stability data of Formulation CF2 under storage conditions: 5 °C +/- 3 °C**

| Test | Acceptance Criteria | Initial | | 3 months | | 12 months | | 24 months | |
|---|---|---|---|---|---|---|---|---|---|
| Assay | 90.0-110% | 102.2% | | 102.1% | | 98.2% | | 100.4% | |
| Impurities | Reported impurities ≥0.05% by area | RRT | Area % | RRT | Area % | RRT | Area % | RRT | Area % |
| | | | | | | 0.10 | 0.07 | | |
| | | 0.14 | 0.07 | 0.14 | 0.07 | 0.14 | 0.07 | 0.13 | 0.09 |
| | | **0.56** | **<0.05** | **0.56** | **0.13** | **0.54** | **<0.05** | **0.59** | **0.26** |
| | | 0.63 | 0.55* | 0.63 | 0.53 | 0.66 | 0.44 | 0.62 | 0.52 |
| | | 0.69 | 0.20* | 0.65 | 0.16 | 0.68 | 0.24 | 0.66 | 0.22 |
| | | 0.85 | 0.20 | 0.83 | 0.19 | 0.72 | 0.21 | 0.82 | 0.20 |
| | | 0.89 | 0.05 | 0.89 | 0.07 | 0.90 | 0.22 | 0.87 | 0.05 |
| | | 1.06 | 1.23 | 1.06 | 1.11 | 1.05 | 1.02 | 1.06 | 1.19 |
| | | 1.08 | 0.24 | 1.09 | 0.17 | 1.07 | 0.17 | 1.08 | 0.22 |
| | | 1.11 | 0.16 | 1.12 | 0.21 | 1.10 | 0.14 | 1.12 | 0.16 |
| | | | | | | 1.18 | 0.07 | | |
| Total Impurities | Reported results by area % | 2.7% | | 2.6% | | 2.7% | | 2.9% | |

| | | | | | | | | | |
|---|---|---|---|---|---|---|---|---|---|
| * indicates a split peak | | | | | | | | | |

**Table 22.1-B - Stability data of Formulation CF2 under storage conditions: 25 °C/60% RH**

| Test | Acceptance Criteria | Initial | | 3 months | | 12 months | | 24 months | |
|---|---|---|---|---|---|---|---|---|---|
| Assay | 90.0-110% | 102.2% | | 99.7% | | 98.7% | | 98.2% | |
| Impurities | Reported impurities ≥0.05% by area | RRT | %Area | RRT | %Area | RRT | %Area | RRT | %Area |
| | | | | | | 0.10 | 0.08 | 0.07 | 0.14 |
| | | | | | | | | 0.11 | 0.10 |
| | | 0.14 | 0.07 | 0.14 | 0.08 | 0.14 | 0.10 | 0.13 | 0.13 |
| | | **0.56** | **<0.05** | **0.56** | **0.12** | **0.56** | **0.43** | **0.59** | **0.76** |
| | | 0.63 | 0.55 | 0.64 | 0.50 | 0.68 | 0.51 | 0.62 | 0.59 |
| | | 0.69 | 0.20 | 0.65 | 0.19 | 0.72 | 0.22 | 0.66 | 0.23 |
| | | 0.85 | 0.20 | 0.83 | 0.17 | 0.90 | 0.20 | 0.82 | 0.19 |
| | | 0.89 | 0.05 | 0.89 | 0.08 | 0.91 | 0.06 | 0.89 | <0.05 |
| | | 1.06 | 1.23 | 1.06 | 1.11 | 1.05 | 1.02 | 1.06 | 1.17 |
| | | 1.08 | 0.24 | 1.09 | 0.18 | 1.07 | 0.18 | 1.08 | 0.24 |
| | | 1.11 | 0.16 | 1.12 | 0.2 | 1.10 | 0.14 | 1.12 | 0.22 |
| | | 1.20 | <0.05 | | | 1.18 | 0.07 | 1.16 | 0.07 |
| | | | | | | | | 2.14 | 0.09 |
| Total Impurities | Reported results by area % | 2.7% | | 2.6% | | 3.0% | | 3.9% | |

### Example 23

### Preparation of a low peroxide formulation ("Formulation LPO")

The following process was used to prepare tablets with either 75 mg or 150 mg of active ingredient. The amounts of individual ingredients are provided in Table 23.1 below. The most noteworthy difference between Formulation LPO and Formulations CF1 and CF2 is the grade of crospovidone used. Formulation LPO used a grade of crospovidone (Polyplasdone TM Ultra from Ashland Chemical) having a maximum of 30 ppm of peroxides (H₂O₂) according to its product specification and Formulation LPO may have an H₂O₂ level as low as 9.2 ppm (see Pharmaceutical Technology Report (PTR-097), Ashland Specialty Ingredients "Utility of Polyplasdone TM crospovidone as a Superdisintegrant" pp. 1-5 (2014)). As described in Example 19 above, the crospovidone used in Formulation CF1 has an H₂O₂ level of 148 ppm. As described in Example 21 above, the crospovidone used in Formulation CF2 has an H₂O₂ level of at least 50 ppm and no more than 400 ppm.

Step 1. Polysorbate 80, vitamin E TPGS, poloxamer 188 and copovidone were dissolved in an amount of water with an overhead stirrer until dissolution, potentially warming to 50 °C to speed up dissolution.

Step 2. OAD2 dihydrochloride was added to the above polysorbate 80/vitamin E TPGS/poloxamer 188/copovidone solution and the mixture was mixed until dissolution.

Step 3. Microcrystalline cellulose and pregelatinized starch were passed through sieve # 30 and charged into a fluid bed dryer and mixed.

Step 4. The solution from Step 2 was sprayed onto the mixture from Step 3 to form sprayed granules.

Step 5. The sprayed granules were screened and passed through #40 screen.

Step 6. The potency of OAD2 dihydrochloride in the sprayed granules material was determined and all of the extragranular materials were adjusted to achieve the required potency for each tablet.

Step 7. Colloidal silicon dioxide, microcrystalline cellulose, Polyplasdone Ultra, and citric acid were passed through #30 screen in separate polyethylene bags.

Step 8. The screened granules and the screened excipients from Step 6 were charged into a V shell blender and mixed for 15-20 minutes.

Step 9. Equal amount of mixtures were charged to the screened magnesium stearate in polyethylene bags and mixed for a few minutes.

Step 10. The screened magnesium stearate was charged into the V shell blender and mixed for several minutes.

Step 11. The powder was taken out and compressed into tablets having following specifications: total weight: 500 mg with 150 mg of OAD2 dihydrochloride; total weight: 250 mg with 75 mg of OAD2 dihydrochloride.

Step 12. After compression, the tablets were coated using Opadry II.

**Table 23.1 - Ingredients of Formulation LPO**

| | | | | |
|---|---|---|---|---|
| | Ingredients | %/Tablet | Weight/500 mg Tablet (mg) | Weight/250 mg Tablet (mg) |
| Intragranular | OAD2 dihydrochloride * | 30.00 | 150.00 | 75.00 |
| | Microcrystalline Cellulose NF/BP (Avicel PH113) | 10.20 | 51.00 | 25.50 |
| | Pregelatinized Corn Starch NF (Starch 1500) | 4.00 | 20.00 | 10.00 |
| | Polysorbate 80 NF (Tween 80) | 3.00 | 15.00 | 7.50 |
| | Pluronic F68 NF (Poloxamer 188) | 1.00 | 5.00 | 2.50 |
| | Vitamin E TPGS NF | 1.60 | 8.00 | 4.00 |
| | Copovidone, USP/NF (Kollidon VA 64) | 2.00 | 10.00 | 5.00 |
| | Water** | Q.S. | Q.S | Q.S. |
| | Intragranular Total | 51.79 | 259.00 | 129.50 |
| Extragranular | Microcrystalline Cellulose NF/BP (Avicel PH113) | 13.96 | 69.80 | 34.90 |
| | Crospovidone (Polyplasdone Ultra) | 3.00 | 15.00 | 7.50 |
| | Colloidal Silicon Dioxide NF (Cab-O-Sil M5P) | 0.50 | 2.50 | 1.25 |
| | Citric Acid anhydrous, USP | 30.00 | 150.00 | 75.00 |
| | Magnesium Stearate, NF (Non-Bovine Hyqual) | 0.75 | 3.76 | 1.88 |
| Total | | 100.00 | 500.06 | 250.03 |

| | | | | |
|---|---|---|---|---|
| *Adjusted based on Use-at-Value (UAV). Corresponding adjustment was made in microcrystalline cellulose based on the UAV of the API to keep the weight of the tablet constant. ** Removed while drying | | | | |

### Example 24

### Stability studies of Formulations CF2 and LPO

Tablets from a batch of Formulation CF2 and a batch of Formulation LPO were placed under forced degradation conditions at 55-60 °C in open dishs and the growth of impurities including impurity B was studied.

Tables 24.1 and 24.2 summarize the growth of Impurity B under these conditions and at the time points indicated. Tables 24.1-A and 24.2-A provide complete lists of impurities in each formulation at the indicated time points under these conditions, wherein the retention times are relative to the retention time of OAD2 dihydrochloride, and impurity B is highlighted in bold. The relative retention time of impurity B in Tables 24.1-A and 24.2-A at time zero (initial) is either 0.47 or 0.59. The assay used high performance liquid chromatography methods described herein.

The symbol "-" in the Tables indicates time points where no data (ND) was collected.

**Table 24.1 - Growth of impurity B in Formulation CF2 at 55-60 °C**

| | Formulation CF2 |
|---|---|
| | Tablet with 100 mg of API |
| Period | Area % of impurity B |
| Initial | 0.18 |
| 7 days | 0.80 |
| 14 days | 1.30 |

**Table 24.2 - Growth of impurity B in Formulation LPO at 60 °C**

| | Formulation LPO Tablet with 150 mg of API | Formulation LPO Tablet with 75 mg of API |
|---|---|---|
| Period | Area % of impurity B | Area % of impurity B |
| Initial | 0.38 | 0.39 |
| 11 days | 0.41 | 0.41 |

**Table 24.1-A - Stability data of Formulation CF2 at 55-60 °C in an open dish**

| Material: Formulation CF2 - Tablets (100 mg API) | | | | | | | |
|---|---|---|---|---|---|---|---|
| Test | Acceptance Criteria | Initial | | 7 days | | 14 days | |
| Impurities | Reported impurities ≥0.05% by area | RRT | %Area | RRT | %Area | RRT | %Area |
| | | 0.15 | 0.17 | 0.15 | 0.29 | 0.15 | 0.40 |
| | | 0.32 | - | 0.32 | 0.10 | 0.31 | 0.12 |
| | | **0.47** | **0.18** | **0.47** | **0.80** | **0.47** | **1.30** |
| | | 0.58 | 0.39 | 0.58 | 0.36 | 0.58 | 0.27 |
| | | 0.61 | 0.16 | 0.61 | 0.18 | 0.61 | 0.17 |
| | | 0.75 | 0.16 | 0.75 | 0.20 | 0.75 | 0.17 |
| | | 0.84 | 0.04 | 0.84 | 0.06 | 0.84 | 0.05 |
| | | 1.00 | 97.05 | 1.00 | 96.33 | 1.00 | 95.87 |
| | | 1.12 | 1.04 | 1.12 | 1.07 | 1.12 | 1.00 |
| | | 1.16 | 0.20 | 1.16 | 0.19 | 1.16 | 0.17 |
| | | 1.20 | 0.15 | 1.20 | 0.16 | 1.20 | 0.12 |
| | | 1.66 | 0.03 | 1.66 | - | 1.66 | - |
| | | 2.56 | - | 2.56 | - | 2.56 | 0.05 |
| | | 2.59 | - | 2.59 | - | 2.59 | 0.10 |
| | | 2.62 | 0.43 | 2.62 | 0.29 | 2.60 | 0.21 |
| Total Impurities | Reported results by area % | 2.92% | | 3.7% | | 4.13% | |

**Table 24.2-A - Stability data of Formulation LPO at 60 °C in an open dish**

| Material: Tablets (75 and 150 mg API) | | | | | | | |
|---|---|---|---|---|---|---|---|
| Test | Acceptance Criteria | Initial | | | 11 days | | |
| | | | 150 mg | 75 mg | | 150 mg | 75 mg |
| Impurities | Reported impurities ≥0.05% by area | RRT | %Area | %Area | RRT | %Area | %Area |
| | | 0.13 | 0.10 | 0.13 | 0.14 | 0.14 | 0.15 |
| | | 0.40 | - | - | 0.40 | 0.08 | 0.07 |
| | | 0.50 | - | - | 0.50 | 0.07 | 0.09 |
| | | **0.59** | **0.38** | **0.39** | **0.58** | **0.41** | **0.41** |
| | | 0.64 | 0.07 | 0.06 | - | - | - |
| | | 0.76 | 0.13 | 0.13 | 0.73 | 0.20 | 0.19 |
| | | 0.85 | 0.14 | 0.16 | 0.81 | 0.17 | 0.16 |
| | | 0.87 | 0.04 | 0.03 | 0.86 | 0.04 | 0.04 |
| | | 0.95 | 0.04 | 0.05 | - | - | - |
| | | 1.00 | 97.82 | 97.59 | 1.00 | 97.23 | 97.13 |
| | | 1.10 | 0.87 | 0.89 | 1.12 | 1.01 | 0.97 |
| | | 1.15 | 0.09 | 0.20 | 1.17 | 0.15 | 0.14 |
| | | 1.18 | 0.08 | 0.12 | 1.20 | 0.11 | 0.11 |
| | | 1.22 | 0.03 | 0.03 | 1.31 | 0.03 | 0.05 |
| | | 1.69 | 0.01 | 0.02 | - | - | - |
| | | 2.68 | 0.19 | 0.19 | 2.73 | 0.17 | 0.17 |
| | | - | - | - | 2.74 | 0.07 | 0.10 |
| | | - | - | - | 2.77 | 0.02 | 0.02 |
| | | - | - | - | 2.78 | 0.02 | 0.02 |
| | | - | - | - | 2.82 | 0.09 | 0.07 |
| Total Impurities | Reported results by area % | | 2.17% | 2.40% | | 2.78% | 2.79% |

The above-mentioned Examples are only used for understanding the method and core idea of the present invention, and do not limit the scope of the present invention. For those skilled in the art, any possible changes or substitutions without departing from the conception of the present invention fall within the protection scope of the present invention.

## Claims

1. A compound represented by formula B, 2-3(4-((3,4-dichlorobenzyl)oxy)phenyl)-8-oxo-12-(1-phenylpropyl)-2,3,6, 8,9,10-hexahydro-7H-6,9-epimino[1,4]dioxino[2',3':4,5]benzo[1,2-c]azepin-7-yl)-3-(4-(2,3-dimethylpyridin-4-yl)phenyl)propionic acid, and a pharmaceutically acceptable salt thereof:

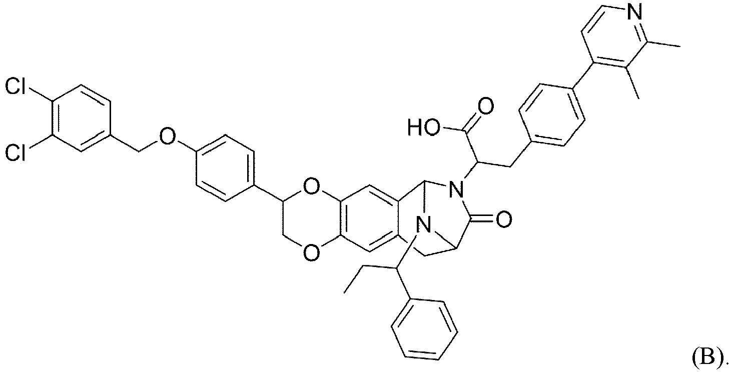

2. A pharmaceutical composition comprising:
(S)-2-(3 S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propanoic acid (OAD2) or a pharmaceutically acceptable salt thereof, and
the compound of formula B according to claim 1 or a pharmaceutically acceptable salt thereof,
wherein the mass percentage content of the compound of formula B is less than 2% and greater than 0%

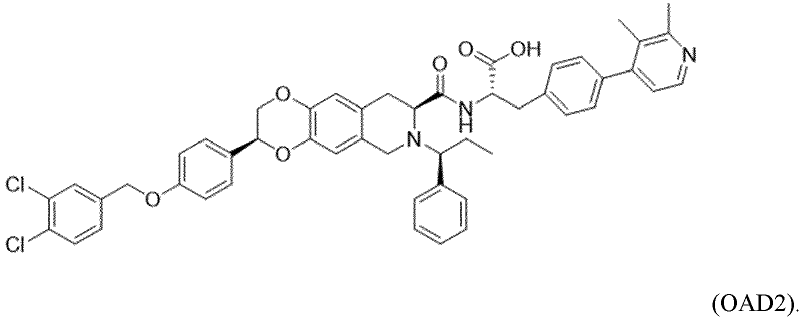

3. A pharmaceutical composition comprising:
(S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propanoic acid (OAD2) or a pharmaceutically acceptable salt thereof, and
at least one solubilizer,
wherein the solubilizer is selected from HS15, RH40, hydroxypropyl βcyclodextrin or Soluplus.

4. The pharmaceutical composition according to claim 3, wherein the mass percentage content of the solubilizer is 0.1% to 10%, preferably 0.2% to 5%.

5. The pharmaceutical composition according to claim 3 comprising one or more disintegrants, wherein the disintegrant is low substituted hydroxypropyl cellulose; and/or the mass percentage content of the disintegrating agent is 5 to 40%, preferably 10 to 30%.

6. The pharmaceutical composition according to claim 3, wherein OAD2 and a pharmaceutically acceptable salt thereof are the only active ingredient in the composition; and/or the pharmaceutically acceptable salt is selected from hydrochloride, dihydrochloride, p-toluenesulfonate, sulfate, hydrobromide, tartrate, citrate, glycolate or methanesulfonate; preferably hydrochloride or dihydrochloride; and/or the mass percentage content of the active ingredient is 1 to 60%; preferably 5% to 40%.

7. The pharmaceutical composition according to claim 3, wherein the composition comprises 5 to 40% of OAD2 or a pharmaceutically acceptable salt thereof, 0.2% to 5% of solubilizer, 20 to 60% of filler, 10 to 30% of disintegrant, 10 to 30% of binder, and less than 2% and more than 0 of the compound of formula B by mass percentage content.

8. The pharmaceutical composition according to claim 7, wherein the composition further comprises 0.2 to 5% of lubricant, 0.2 to 5% of wetting agent, 0.2 to 5% of glidant, 0.2 to 5% of absorption enhancer and 5 to 30% of acidifer by mass percentage content.

9. The pharmaceutical composition according to claim 2 or 7, wherein the mass percentage content of the compound of formula B is more than 0 and less than 1.0%, preferably more than 0 and less than 0.6%, more preferably more than 0 and less than 0.3%.

10. The pharmaceutical composition according to any one of claims 2 to 8, wherein: the pharmaceutical composition is an oral pharmaceutical composition; and/or the pharmaceutical composition is a solid composition selected from tablets, pills, capsules or granules.

11. The pharmaceutical composition according to any one of claims 2 to 8, wherein the pharmaceutical composition comprises from 1 to 300mg of OAD2 and a pharmaceutically acceptable salt thereof.

12. A process for preparing the pharmaceutical composition according to any one of claims 2 to 8, comprising the steps of:
a. processing raw auxiliary materials: sieving filler, disintegrant, solubilizer, and optionally other auxiliary materials, then putting aside;
b. granulating and mixing: subjecting formulated amounts of OAD2 or a pharmaceutically acceptable salt thereof, filler, solubilizer, disintegrant, and optionally binder, acidifer, lubricant, wetting agent, acidifer, glidant, absorption enhancer and surfactant to granulating and mixing;
c. mixing the granules with filler, disintegrating agent, lubricant, glidant, acidifer and other auxiliary materials as appropriate, and tableting the mixture, wherein the hardness was controlled to be 8-20 Kg.

13. Use of the pharmaceutical composition according to any one of claims 2-8 in the preparation of a medicament for treating or preventing a GLP-1 receptor mediated disease.

14. A method of treating or preventing a GLP-1 receptor mediated disease comprising administering to a patient in need thereof a therapeutically effective amount of a pharmaceutical composition according to any one of claims 2 to 8.

15. The method of treating or preventing a GLP-1 receptor mediated disease according to claim 14, wherein the GLP-1 receptor mediated disease is selected from metabolic syndrome, glucose intolerance, hyperglycemia, dyslipidemia, type I diabetes, type II diabetes, syndrome X, insulin resistance, impaired glucose tolerance (IGT), obesity, diabetic dyslipidemia, hyperlipidemia, arteriosclerosis, atherosclerosis, other cardiovascular diseases that would benefit from activation of GLP-1 receptor, hypertension, metabolic disorders that would benefit from activation of GLP-1 receptor, and the like, and complications arising from or associated with diabetes including neurological diseases, retinopathy, nephropathy and impaired wound healing; preferably type II diabetes, type I diabetes and obesity.

16. The method of treating or preventing a GLP-1 receptor mediated disease according to claim 14 comprising administering 25-225 mg OAD2 or a pharmaceutically acceptable salt thereof to a patient in need thereof.

17. Use of the compound of formula B according to claim 1 as impurity control for a raw material of OAD2 and a pharmaceutically acceptable salt thereof and/or a solid composition thereof.

18. A compound produced by the process comprising the step of: oxidizing (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propionic acid (OAD2) or a pharmaceutically acceptable salt thereof, wherein the resulting compound is **characterized by** having a molecular weight of 854.

19. The compound according to claim 18, wherein the compound is **characterized by** a retention time of between 0.45 and 0.66 relative to OAD2 under reverse phase liquid chromatography gradient mobile phase conditions wherein a mobile phase A comprises 0.05% TFA in water (v/v), a mobile phase B comprises 0.05% TFA in acetonitrile methanol (1:2) (v/v), wherein the sample is run using a step gradient from time zero (50% mobile phase A) to 38 minutes (99% mobile phase B).

20. A composition comprising:
(S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propionic acid (OAD2) or a pharmaceutically acceptable salt thereof,
the compound according to claim 18 or a pharmaceutically acceptable salt thereof, and
one or more pharmaceutically acceptable excipients,
wherein the amount of the compound according to claim 18 or 19 or a pharmaceutically acceptable salt thereof present in the composition is greater than 0 and less than 2.5 wt%.

21. A composition comprising:
(S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propionic acid (OAD2) or a pharmaceutically acceptable salt thereof, and
a disintegrant that has an HPO value of less than 50 ppm.

22. The composition according to claim 21, comprising:
from 10 wt% to 40 wt% of OAD2 dihydrocholoride, and
from 0.1 wt% to 20 wt% of the disintegrant.

23. The composition according to claim 22, wherein the disintegrant is crospovidone and is present ranging from 0.2 wt% to 10 wt%.

24. The composition according to claim 21, wherein the composition comprises the compound according to claim 18 or a pharmaceutically acceptable salt thereof, wherein the amount of the compound according to claim 18 or a pharmaceutically acceptable salt thereof present is greater than 0 and less than 1.0 wt%.

25. The composition according to claim 24, wherein the composition comprises 0.4 wt% or less of the compound according to claim 18 or a pharmaceutically acceptable salt thereof after 24 months of storage at 25 °C ± 2 °C /60% RH ± 5% RH.

26. The composition according to claim 24, wherein the composition comprises 1 wt% or less of the compound according to claim 18 or a pharmaceutically acceptable salt thereof after 14 days of storage at 55 °C to 65 °C.

27. The composition according to claim 21, comprising:
from 10 wt% to 40 wt% of OAD2 dihydrocholoride salt,
from 1 wt% to 5 wt% of a disintegrant, wherein the disintegrant comprises crospovidone,
from 0.1 wt % to 20 wt% of a binder,
from 10 wt% to 85 wt% of a filler,
from 0.25 wt% to 15 wt% of a surfactant,
from 0.1 wt% to 10 wt% of a lubricant,
from 0.1 wt% to 10 wt% of a glidant,
from 1 wt% to about 50 wt% of an acidifer,
wherein the composition comprises the compound according to claim 18 or a pharmaceutically acceptable salt thereof,
wherein the amount of the compound according to claim 18 or a pharmaceutically acceptable salt thereof present is greater than 0 and less than or equal to 0.4 wt% after 24 months of storage at 25 °C ± 2 °C /60% RH ± 5% RH.

28. The composition according to any one of claims 20 to 27, in the form of a tablet or capsule.

29. The composition according to any one of claims 20 to 27, comprising between 1 to 500 mg of OAD2 or a pharmaceutically acceptable salt thereof.

30. A method of preparing the composition according to any one of claims 20 to 27, wherein the method comprises admixing OAD2 or a pharmaceutically acceptable salt thereof with the one or more pharmaceutically acceptable excipients.

31. The method according to claim 30, wherein the method is a spray granulation process.

32. A method of treating a condition comprising administering to a human in need thereof the composition according to any one of claims 20 to 31, wherein the condition is selected from the group consisting of metabolic syndrome, glucose intolerance, hyperglycemia, dyslipidemia, type I diabetes, type II diabetes, hypertriglyceridemia, syndrome X, insulin resistance, impaired glucose tolerance (IGT), obesity, diabetic dyslipidemia, hyperlipidemia, arteriosclerosis, atherosclerosis, other cardiovascular diseases, hypertension, and complications resulting from or associated with diabetes including, but not limited to, neuropathy, retinopathy, nephropathy, and impaired wound healing.

33. The method according to claim 32, wherein the condition is type II diabetes.

34. The method according to claim 33, wherein the amount of OAD2 or a pharmaceutically acceptable salt thereof administered is between 25 mg to 200 mg per day.

35. A pharmaceutical composition, comprising (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propanoic acid (OAD2) or a pharmaceutically acceptable salt thereof, and one or more pharmaceutically acceptable carrier auxiliary materials or excipients.
